(19) Europäisches Patentamt
European Patent Office
Office européen des brevets

(11) EP 3 898 645 B1

(12) **EUROPEAN PATENT SPECIFICATION**

(45) Date of publication and mention of the grant of the patent:
**25.06.2025 Bulletin 2025/26**

(21) Application number: **19831850.3**

(22) Date of filing: **20.12.2019**

(51) International Patent Classification (IPC):
***C07J 9/00*** *(2006.01)* ***C07J 41/00*** *(2006.01)*
***C07J 43/00*** *(2006.01)* ***C07J 31/00*** *(2006.01)*
***A61K 31/575*** *(2006.01)* ***A61K 31/58*** *(2006.01)*
***A61P 25/28*** *(2006.01)* ***C07J 51/00*** *(2006.01)*
***C07J 71/00*** *(2006.01)* ***C07J 11/00*** *(2006.01)*

(52) Cooperative Patent Classification (CPC):
**C07J 9/005; C07J 31/006; C07J 41/0061; C07J 43/003;** C07J 11/00; C07J 51/00; C07J 71/001

(86) International application number:
**PCT/GB2019/053665**

(87) International publication number:
**WO 2020/128514 (25.06.2020 Gazette 2020/26)**

(54) **2-FLUORINATED BILE ACIDS FOR THE TREATMENT OF NEURODEGENERATIVE DISEASES**

2-FLUORIERTE GALLENSÄUREN ZUR BEHANDLUNG VON NEURODEGENERATIVEN ERKRANKUNGEN

ACIDES BILIAIRES 2-FLUORÉS POUR LE TRAITEMENT DE MALADIES NEURODÉGÉNÉRATIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2018 GB 201820887**

(43) Date of publication of application:
**27.10.2021 Bulletin 2021/43**

(73) Proprietors:
- **NZP UK Limited**
  **Bristol, BS2 0ZX (GB)**
- **THE UNIVERSITY OF SOUTHAMPTON**
  **Southampton SO17 1BJ (GB)**

(72) Inventors:
- **WEYMOUTH-WILSON, Alexander, Charles**
  **Berkshire RG2 9LH (GB)**
- **LINCLAU, Bruno Jan Pol**
  **Eastleigh Hampshire SO50 7PS (GB)**
- **PACKER, Gemma**
  **Berkshire RG2 9LH (GB)**
- **WATTS, Joseph**
  **Southampton Hampshire SO17 1BJ (GB)**
- **MORTIBOYS, Heather**
  **Sheffield S10 2HQ (GB)**
- **BANDMANN, Oliver**
  **Sheffield S10 2HQ (GB)**
- **HASTINGS, Christopher**
  **Sheffield S10 2HQ (GB)**

(74) Representative: **Sagittarius IP**
**Marlow International**
**Parkway**
**Marlow SL7 1YL (GB)**

(56) References cited:
**WO-A1-2016/145216 WO-A2-2010/012904**
**WO-A2-2014/036379**

- **SIMON M. BELL ET AL: "Ursodeoxycholic Acid Improves Mitochondrial Function and Redistributes Drp1 in Fibroblasts from Patients with Either Sporadic or Familial Alzheimer's Disease", JOURNAL OF MOLECULAR BIOLOGY, vol. 430, no. 21, 1 October 2018 (2018-10-01), United Kingdom, pages 3942 - 3953, XP055671138, ISSN: 0022-2836, DOI: 10.1016/j.jmb.2018.08.019**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HEATHER MORTIBOYS ET AL: "UDCA exerts beneficial effect on mitochondrial dysfunction in LRRK2-G2019S carriers and in vivo", NEUROLOGY, vol. 85, no. 10, 8 September 2015 (2015-09-08), pages 846 - 852, XP055671134, DOI: doi.org/10.1212/WNL.0000000000001905
- HEATHER MORTIBOYS ET AL: "Ursocholanic acid rescues mitochondrial function in common forms of familial Parkinson’s disease", BRAIN, vol. 136, no. 10, 1 October 2013 (2013-10-01), pages 3038 - 3050, XP055671128, ISSN: 0006-8950, DOI: 10.1093/brain/awt224

## Description

The present invention relates to novel compounds which are of use in the treatment of neurodegenerative disorders. In' particular, the invention relates to bile acid derivatives, to pharmaceutical compositions containing them, process for preparing them and to the use of the compounds in the treatment or prevention of neurodegenerative disorders.

Neurodegenerative diseases are a group of disorders of the central nervous system and include Parkinson's disease, mild cognitive impairment, dementia (including Alzheimer's disease, vascular dementia and dementia with Lewy bodies), Huntington's disease and amyotrophic lateral sclerosis (motor neurone disease). The incidence of neurodegenerative disease increases with age and therefore such conditions are a growing problem in societies where the average age of the population is increasing. There is currently no cure for any of these diseases although there are some medications available which alleviate the symptoms of Parkinson's disease and some types of cognitive impairment and dementia.

The symptoms of Parkinson's disease are resting tremor, bradykinesia and rigidity and these symptoms are caused by neurodegeneration and loss of dopaminergic neurons. There is a large body of evidence which suggests that there is a strong association between mitochondrial dysfunction and Parkinson's disease. A mild deficiency of mitochondrial electron transport chain NADH dehydrogenase (complex I) activity has been found in the tissues of Parkinson's disease patients and a number of the proteins that are linked to the familial form of Parkinson's disease are either mitochondrial proteins or are associated with mitochondria.

Alzheimer's disease leads to progressive cognitive impairment and is characterised by the presence of extracellular neuritic plaques and intracellular neurofibrillary tangles. It is thought that mitochondrial dysfunction leads to the deposition of the β-amyloid proteins which are the major component of the neuritic plaques and to the formation of the neurofibrillary tangles.

Huntington's disease is an inherited progressive neurodegenerative disease and is characterised by motor impairment, personality changes and cognitive decline. The pathology of Huntington's disease provides evidence for a link with mitochondrial dysfunction.

Amyotrophic lateral sclerosis is also thought to be linked to mitochondrial dysfunction. This disease targets motor neurons in the central nervous system resulting in muscle weakness, atrophy and, death within 2-3 years of diagnosis.

Attempts have been made to find compounds which are capable of treating neurodegenerative disorders and several compounds have been developed which target mitochondria. For example, it is known that bile acids such as UDCA (ursodeoxycholic acid) exert a beneficial effect on mitochondrial dysfunction in tissue from certain patients suffering from Parkinson's disease, in particular in tissue from *parkin* mutant Parkinson's disease patients (Mortiboys et al, "Ursocholanic acid rescues mitochondrial function in common forms of familial Parkinson's disease", Brain, 136(10), 3038-3050 (2013)) and LRRK2$^{G2019S}$ mutant Parkinson's disease patients (Mortiboys et al, Neurology, 85, 846-852 (2015)). Furthermore it is known bile acids such as UDCA exert a beneficial effect on fibroblasts from patients suffering from both sporadic Alzheimer's Disease and familial Alzheimer's Disease due to PSEN1 mutations (Bell et al, "Ursodeoxycholic Acid Improves Mitochondrial Function and Redistributes Drp1 in Fibroblasts from Patients with either Sporadic or Familial Alzheimer's Disease." Journal of Molecular Biology, pii: S0022-2836(18)30987-2. 2018).

WO 2014/036379, WO 2015/061421 and WO 2016/145216 teach that bile acids may be of use in the treatment of neurodegenerative disorders such as Parkinson's disease, Alzheimer's disease, Huntington's disease and amyotrophic lateral sclerosis. WO 2015/061421 relates to deuterated bile acids and WO 2016/145216 to fluorinated bile acids particularly bile acids fluorinated at the 3- and/or 7-positions.

The present inventors have now found that certain fluorinated bile acids have superior mitochondrial rescue properties and are particularly effective in the treatment of neurodegenerative disorders.

In the present invention there is provided a compound of general formula (I):

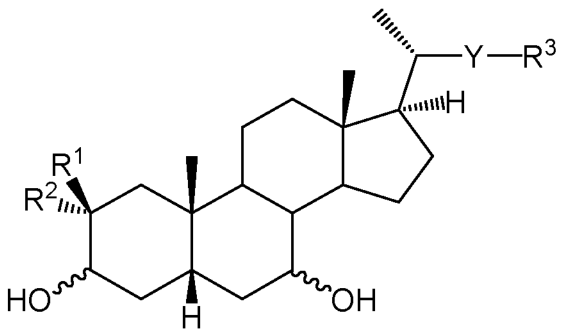

(I)

wherein

one of $R^1$ and $R^2$ is F and the other of $R^1$ and $R^2$ is H or F;

Y is a bond, or a $C_{1-20}$ alkylene, $C_{2-20}$ alkenylene or $C_{2-20}$ alkynylene linker group;

$R^3$ is $C(O)OR^{12}$, $C(O)NR^{12}R^{13}$, $S(O)_2R^{12}$, $OS(O)_2R^{12}$, $S(O)_2OR^{12}$, $OS(O)_2OR^{12}$, $S(O)_2NR^{12}R^{13}$, $C(O)NR^{12}S(O)_2R^{13}$, $NHC(O)NR^{12}S(O)_2R^{13}$, $OP(O)(OR^{12})_2$, $C(O)NR^{12}[CH(R^{15})]_nR^{16}$ or $C(O)NR^{12}C(O)CH_2NR^{12}[CH(R^{15})]_nR^{16}$;

each $R^{12}$ is independently H or $C_{1-6}$ alkyl optionally substituted by one or more substituents selected from halo, $OR^{10}$, $NR^{10}R^{11}$, $R^{16}$ and aryl;
each $R^{10}$ and $R^{11}$ is independently H or $C_{1-6}$ alkyl;
$R^{13}$ is H, $C_{1-6}$ alkyl optionally substituted by one or more substituents selected from halo and aryl; or a 3- to 8-membered carbocyclic ring or heterocyclic ring, wherein said carbocyclic or heterocyclic ring is optionally substituted with one or more substituents selected from =O and $R^{16}$; or a phenyl or 5- or 6-membered heteroaryl ring, wherein said phenyl or heteroaryl ring is optionally substituted with a substituent $R^{16}$; or
when $R^3$ is $C(O)NR^{12}R^{13}$ or $S(O)_2NR^{12}R^{13}$, $R^{12}$ and $R^{13}$ together with the nitrogen atom to which they are attached form a 3- to 8-membered heterocyclic ring which optionally contains one or more further hetero atoms selected from N, O and S; and is optionally substituted with one or more substituents selected from $CH_2C(O)OH$, $C(O)OH$, $C_{1-6}$ alkyl, $C(O)OC_{1-6}$ alkyl, $S(O)_2OH$, =O and =N-OH; and is optionally fused to a phenyl group which is unsubstituted or substituted with one or more substituents selected from halo and nitro;
n is 1, 2 or 3;
each $R^{15}$ is independently H or $C_{1-6}$ alkyl optionally substituted by one or more substituents selected from halo, phenyl and 5- or 6-membered heteroaryl; a 3- to 8-membered cycloalkyl group; or a group $R^{14}$, where $R^{14}$ is a side chain of an amino acid; or
when n is 2 or 3, two $R^{15}$ groups together with the carbon atoms to which they are attached, and optionally an intervening carbon atom where present, can combine to form $-(CH_2)_p-$ such that the group $[CH(R^{15})]_n$ is a 3- to 8 membered carbocyclic ring;
p is 1, 2, 3, 4, 5 or 6;
$R^{16}$ is selected from $C(O)OH$, $S(O)_2OH$, $S(O)_2(C_{1-6}$ alkyl), $OS(O)_2OH$ and $P(O)(OH)_2$;
or a pharmaceutically acceptable salt or isotopic variant thereof.

Compounds of general formula (I) are of use for the treatment of neurodegenerative disorders of the central nervous system, including Parkinson's disease, dementia and amyotrophic lateral sclerosis.

## Detailed description of the invention

In the present specification, except where the context requires otherwise due to express language or necessary implication, the word "comprises", or variations such as "comprises" or "comprising" is used in an inclusive sense i.e. to specify the presence of the stated features but not to preclude the presence or addition of further features in various embodiments of the invention.

The references to methods of treatment in the summary and detailed description of the invention are to be interpreted as references to the compounds, pharmaceutical compositions and medicaments of the present invention for use in a method of treatment of the human (or animal) body by therapy (or for diagnosis)

In the present application, the term "$C_{1-20}$" alkyl refers to a straight or branched fully saturated hydrocarbon group having from 1 to 20 carbon atoms. The term encompasses methyl, ethyl, *n*-propyl, isopropyl, *n*-butyl, isobutyl, *s*-butyl and *t*-butyl. Other alkyl groups, for example $C_{1-12}$ alkyl, $C_{1-10}$ alkyl, $C_{1-8}$ alkyl, $C_{1-6}$ alkyl, $C_{1-5}$ alkyl, $C_{1-4}$ alkyl, $C_{1-3}$ alkyl, or $C_{1-2}$ alkyl are as defined above but contain different numbers of carbon atoms.

The term "$C_{2-20}$ alkenyl" refers to a straight or branched hydrocarbon group having from 2 to 20 carbon atoms and at least one carbon-carbon double bond. Examples of alkenyl groups include $-CH=CH_2$, $-CH=CH(CH_3)$, $-CH_2CH=CH_2$, $-CH=CHCH_3$, $-CH_2CH_2CH=CH_2$, $-CH_2CH=CH(CH_3)$- and $-CH_2CH=CH(CH_2CH_3)$. Other alkenyl groups, for example $C_{2-12}$ alkenyl, $C_{2-10}$ alkenyl, $C_{2-8}$ alkenyl, $C_{2-6}$ alkenyl, $C_{2-5}$ alkenyl, $C_{2-4}$ alkenyl or $C_{2-3}$ alkenyl are as defined above but contain different numbers of carbon atoms.

The term "$C_{2-20}$ alkynyl" refers to a straight or branched hydrocarbon group having from 2 to 20 carbon atoms and

at least one carbon-carbon triple bond. Examples of alkynyl groups include $-C \equiv CH$, $-CH_2C \equiv CH$, $-C \equiv C-CH_3$, $-CH_2CH_2C \equiv CH$, $-CH_2C \equiv CCH_3$ and $-CH_2C \equiv C-CH_2CH_3$. Other alkynyl groups, for example $C_{2-12}$ alkynyl, $C_{2-10}$ alkynyl, $C_{2-8}$ alkynyl, $C_{2-6}$ alkynyl, $C_{2-5}$ alkynyl, $C_{2-4}$ alkynyl or $C_{2-3}$ alkynyl are as defined above but contain different numbers of carbon atoms.

The term "alkylene" refers to a straight or branched fully saturated hydrocarbon chain. Suitably alkylene is $C_{1-20}$ alkylene, $C_{1-12}$ alkylene, $C_{1-10}$ alkylene, $C_{1-8}$ alkylene, $C_{1-6}$ alkylene, $C_{1-5}$ alkylene, $C_{1-4}$ alkylene, $C_{1-3}$ alkylene, or $C_{1-2}$ alkylene. Examples of alkylene groups include $-CH_2-$, $-CH_2CH_2-$, $-CH(CH_3)-CH_2-$, $-CH_2CH(CH_3)-$, $-CH_2CH_2CH_2-$, $-CH_2CH(CH_2CH_3)-$ and $-CH_2CH(CH_2CH_3)CH_2-$.

The term "alkenylene" refers to a straight or branched hydrocarbon chain containing at least one carbon-carbon double bond. Suitably alkenylene is $C_{2-20}$ alkenylene, $C_{2-12}$ alkenylene, $C_{2-10}$ alkenylene, $C_{2-8}$ alkenylene, $C_{2-6}$ alkenylene, $C_{2-5}$ alkenylene, $C_{2-4}$ alkenylene, or $C_{2-3}$ alkenylene. Examples of alkenylene groups include $-CH=CH-$, $-CH=C(CH_3)-$, $-CH_2CH=CH-$, $-CH=CHCH_2-$, $-CH_2CH_2CH=CH-$, $-CH_2CH=C(CH_3)-$ and $-CH_2CH=C(CH_2CH_3)-$.

The term "alkynylene" refers to a straight or branched hydrocarbon chain containing at least one carbon-carbon triple bond. Suitably alkynylene is $C_{2-20}$ alkynylene, $C_{2-12}$ alkynylene, $C_{2-10}$ alkynylene, $C_{2-8}$ alkynylene, $C_{2-6}$ alkynylene, $C_{2-5}$ alkynylene, $C_{2-4}$ alkynylene, or $C_{2-3}$ alkynylene. Examples of alkynylene groups include $-C \equiv C-$, $-CH_2C \equiv C-$, $-C \equiv C-CH_2-$, $-CH_2CH_2C \equiv C-$, $-CH_2C \equiv CCH_2-$ and $-CH_2C \equiv C-CH_2CH_2-$.

The terms "aryl" and "aromatic" refer to a cyclic group with aromatic character having from 6 to 14 ring carbon atoms (unless otherwise specified, for example 6 to 10 ring carbon atoms) and containing up to three rings. Where an aryl group contains more than one ring, not all rings must be aromatic in character. Examples include phenyl, naphthyl and anthracenyl as well as partially saturated systems such as tetrahydronaphthyl (e.g. 1,2,3,4-tetrahydronaphthyl), indanyl and indenyl.

The terms "heteoaryl" and "heteroaromatic" refer to a cyclic group with aromatic character having from 5 to 14 ring atoms (unless otherwise specified, for example 5 to 10 ring atoms), containing at least one heteroatom selected from N, O and S and comprising up to three rings. Where a heteoroaryl group contains more than one ring, not all rings must be aromatic in character. Examples include pyridine, pyrimidine, pyrrole, thiophene, furan, thiazole, oxazole, fused systems such as indole, benzimidazole and benzothiophene; and partially saturated systems such as indoline and dihydrobenzofuran.

The terms "carbocyclic" and "carbocyclyl" refer to a non-aromatic hydrocarbon ring system having from 3 to 10 ring carbon atoms (unless otherwise specified), which may be a fused or bridged ring system and which optionally comprises one or more carbon-carbon double bonds. Examples include cycloalkyl groups such as cyclopropyl, cyclobutyl, cyclopentyl, cyclohexyl, cycloheptyl and cyclooctyl; and cycloalkenyl groups such as cyclohexenyl, and cycloheptenyl; and bridged groups such as adamantyl. More suitably, the carbocyclyl group is a monocylic fully saturated (cycloalkyl) ring.

The terms "heterocyclic" and "heterocyclyl" refer to a non-aromatic ring system having from 3 to 10 ring carbon atoms (unless otherwise specified), and at least one heteroatom selected from N, O and S and which may be a fused or bridged ring system and which may be fully saturated or may comprise one or more carbon-carbon or carbon-nitrogen double bonds. Examples include piperidinyl, morpholinyl, thiomorpholinyl, thiozolidinyl, tetrahydrothiophenyl and tetrahydrothiopyranyl. More suitably, the heterocyclyl group is a monocylic fully saturated ring.

The term "halogen" refers to fluorine, chlorine, bromine or iodine and the term "halo" to fluoro, chloro, bromo or iodo groups.

The term "$C_{1-6}$ haloalkyl" refers to a straight or branched alkyl group as defined above having from 1 to 6 carbon atoms and substituted with one or more halo atoms, up to perhalo substitution. Examples include trifluoromethyl, chloroethyl and 1,1-difluoroethyl. Other haloalkyl groups, for example $C_{1-5}$ haloalkyl, $C_{1-4}$ haloalkyl, $C_{1-3}$ haloalkyl or $C_{1-2}$ haloalkyl are as defined above but contain different numbers of carbon atoms.

The term "side chain of an amino acid" refers to the side chain of a naturally occurring amino acid, which may be a D-amino acid or an L-amino acid but is more suitably a D-amino acid. Examples of naturally occurring amino acids include glycine, proline, cysteine, arginine, histidine, lysine, aspartic acid, glutamic acid, serine, threonine, asparagine, glutamine, alanine, valine, isoleucine, leucine, methionine, phenylalanine, tyrosine and tryptophan.

The term "side chain" refers to the $-Y-R^3$ moiety. In UDCA, $-YR^3$ is $-CH_2CH_2-C(O)OH$ and references to a variant side chain refer to $-YR^3$ moieties other than this.

References to a substituent "=O" refer to an oxygen atom linked by a double bond to an adjacent atom which is suitably a carbon or sulfur atom and which may be a ring atom. Examples of moieties including an "=O" substituent include $-C(O)-$, $-S(O)-$ and $-S(O)_2-$.

Appropriate pharmaceutically acceptable salts of the compounds of general formula (I) include basic addition salts such as sodium, potassium, calcium, aluminium, zinc, magnesium and other metal salts as well as choline, diethanolamine, ethanolamine, ethyl diamine, meglumine and other well-known basic addition salts as summarised in Paulekuhn et al., J. Med. Chem. 2007, 50, 6665-6672 and/or known to those skilled in the art.

The term "isotopic variant" refers to isotopically-labelled compounds which are identical to those recited in formula (I) but for the fact that one or more atoms are replaced by an atom having an atomic mass or mass number different

from the atomic mass or mass number most commonly found in nature, or in which the proportion of an atom having an atomic mass or mass number found less commonly in nature has been increased (the latter concept being referred to as "isotopic enrichment"). Examples of isotopes that can be incorporated into compounds of the invention include isotopes of hydrogen, carbon, nitrogen, oxygen, fluorine, iodine and chlorine such as $^{2}H$ (deuterium), $^{3}H$, $^{11}C$, $^{13}C$, $^{14}C$, $^{18}F$, $^{123}I$ or $^{125}I$ (e.g. $^{3}H$, $^{11}C$, $^{14}C$, $^{18}F$, $^{123}I$ or $^{125}I$), which may be naturally occurring or non-naturally occurring isotopes.

In some cases, the compound of general formula (I) may be a compound of formula (I'):

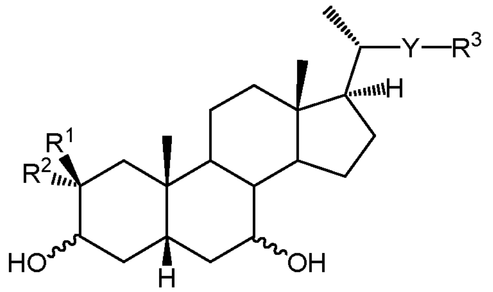

(I')

wherein

one of $R^1$ and $R^2$ is F and the other of $R^1$ and $R^2$ is H or F;

Y is a bond, or a $C_{1-20}$ alkylene, $C_{2-20}$ alkenylene or $C_{2-20}$ alkynylene linker group;

$R^3$ is $C(O)OR^{12}$, $C(O)NR^{12}R^{13}$, $S(O)_2R^{12}$, $OS(O)_2R^{12}$, $S(O)_2OR^{12}$, $OS(O)_2OR^{12}$, $S(O)_2NR^{12}R^{13}$, $C(O)NR^{12}S(O)_2R^{13}$, $NHC(O)NR^{12}S(O)2R^{13}$, $OP(O)(OR^{12})_2$, $C(O)NR^{12}[CH(R^{15})]_nR^{16}$ or $C(O)NR^{12}C(O)CH_2NR^{12}[CH(R^{15})]_nR^{16}$

each $R^{12}$ is independently H or $C_{1-6}$ alkyl optionally substituted by one or more halo or aryl groups;
$R^{13}$ is H, $C_{1-6}$ alkyl optionally substituted by one or more halo or aryl groups or a 5- or 6-membered carbocyclic ring optionally substituted with a substituent $R^{16}$; or $R^{12}$ and $R^{13}$ together with the nitrogen atom to which they are attached form a 5- or 6-membered heterocyclic ring optionally containing a further nitrogen atom, optionally substituted with one or more substituents selected from $CH_2C(O)OH$, $C(O)OH$, $S(O)_2OH$, =O or =N-OH groups and optionally fused to a phenyl group which is unsubstituted or substituted with one or more substituents selected from halo and nitro;
each $R^{15}$ is independently H or $C_{1-6}$ alkyl optionally substituted by one or more halo or aryl groups or a group $R^{14}$, where $R^{14}$ is a side chain of an amino acid;
n is 1, 2 or 3;
$R^{16}$ is selected from $C(O)OH$, $S(O)_2OH$, $OS(O)_2OH$ and $P(O)(OH)_2$;

or a pharmaceutically acceptable salt or isotopic variant thereof.

The compound of general formula (I) may be a compound of general formula (IA), (IB), (IC) or (ID):

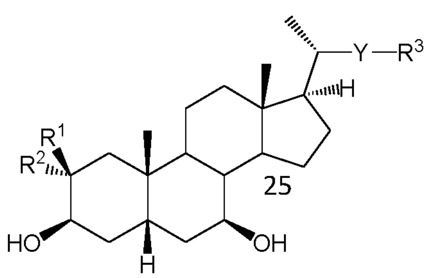

(IA)

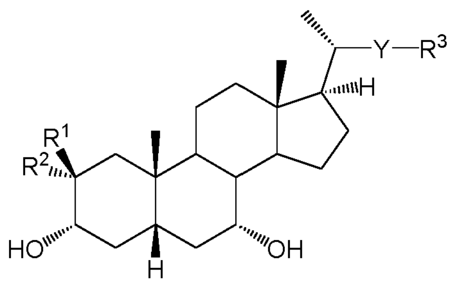

(IB)

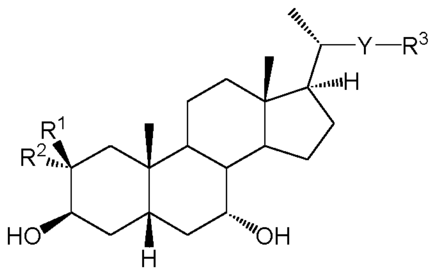

(IC)

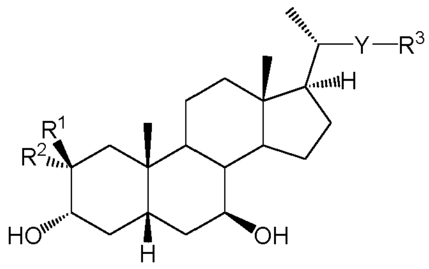

(ID)

wherein $R^1$, $R^2$, Y and $R^3$ are as defined above for general formula (I).

Some particularly suitable compounds of the invention are compounds of general formula (IA).

Other suitable compounds of the invention are compounds of general formula (IB).

Other suitable compounds of the invention are compounds of general formula (IC).

Other suitable compounds of the invention are compounds of general formula (ID).

In some suitable compounds of general formulae (I), (IA), (IB), (IC) and (ID), both $R^1$ and $R^2$ are F.

In other suitable compounds of general formulae (I), (IA), (IB), (IC) and (ID), $R^1$ is F and $R^2$ is H.

In still other suitable compounds of general formulae (I), (IA), (IB), (IC) and (ID), $R^1$ is H and $R^2$ is F.

Compounds in which both $R^1$ and $R^2$ are F are particularly suitable.

In compounds of general formulae (I), (IA), (IB), (IC) and (ID), Y is suitably a bond or a $C_{1-15}$ alkylene linker, or $C_{2-15}$ alkenylene linker. More suitably, Y is a bond or a $C_{1-12}$, $C_{1-10}$, $C_{1-8}$, $C_{1-6}$, $C_{1-4}$, $C_{1-3}$ or $C_{1-2}$ alkylene linker or a $C_{2-12}$, $C_{2-10}$, $C_{2-8}$, $C_{2-6}$, $C_{2-4}$, $C_{2-3}$ or $C_2$ alkenylene linker and is unsubstituted or substituted with an OH group.

In some suitable compounds of general formulae (I), (IA), (IB), (IC) and (ID), Y is bond, or a $C_{1-3}$ alkylene or $C_{2-3}$ alkenylene linker group. Suitably Y is $C_{1-3}$ alkylene or $C_{2-3}$ alkenylene.

More suitably, Y is bond, or a $C_{1-3}$ alkylene linker group. Still more suitably, Y is a $C_{1-3}$ alkylene linker group.

Examples of particularly suitable linkers Y include a bond, $-CH_2-$, $-CH_2CH_2-$, $-CH(OH)-CH_2-$, $-CH=CH-$ or $-CH=C(CH_3)-$, in particular, a bond, $-CH_2-$, $-CH_2CH_2-$, $-CH=CH-$ or $-CH=C(CH_3)-$, especially $-CH_2-$, $-CH_2CH_2-$, $-CH=CH-$ or $-CH=C(CH_3)-$, and especially $-CH_2CH_2-$.

As discussed above, in the compounds of general formulae (I), (IA), (IB), (IC) and (ID), $R^3$ is $C(O)O_{R}^{12}$, $C(O)NR^{12}R^{13}$, $S(O)_2R^{12}$, $OS(O)_2R^{12}$, $S(O)_2OR^{12}$, $OS(O)_2OR^{12}$, $S(O)_2NR^{12}R^{13}$, $C(O)NR^{12}S(O)_2R^{13}$, $NHC(O)NR^{12}S(O)_2R^{13}$, $OP(O)(OR^{12})_2$, $C(O)NR^{12}[CH(R^{15})]_nR^{16}$ or $C(O)NR^{12}C(O)CH_2NR^{12}[CH(R^{15})]_nR^{16}$.

In some cases, in the compounds of general formulae (I), (IA), (IB), (IC) and (ID), $R^3$ is more suitably $C(O)OR^{12}$, $OS(O)_2R^{12}$, $OS(O)_2OR^{12}$, $S(O)_2NR^{12}R^{13}$, $C(O)NR^{12}S(O)_2R^{13}$, $NR^{12}C(O)NR^{12}S(O)_2R^{13}$ or $C(O)NR^{12}[CH(R^{15})]_nR^{16}$; wherein $R^{12}$, $R^{13}$, $R^{15}$, n and $R^{16}$ are as defined above.

In this case, still more suitable compounds are those in which $R^3$ is $C(O)OR^{12}$, $C(O)NR^{12}CH(R^{14})C(O)OH$ or $C(O)NR^{12}CH(R^{15})CH(R^{15})S(O)_2OH$, wherein $R^{12}$ and $R^{14}$ are as defined above and $R^{15}$ is H or $C_{1-6}$ alkyl optionally substituted by one or more halo or aryl groups.

In other more suitable compounds, $R^3$ is $C(O)OR^{12}$, $(C(O)N(R^{12})(R^{13})$ or $C(O)NR^{12}[CH(R^{15})]_nR^{16}$; wherein $R^{12}$, $R^{13}$, $R^{15}$, $R^{16}$ and n are as defined above.

In the compounds of general formulae (I), (IA), (IB), (IC) and (ID), $R^{12}$ is suitably H, $C_{1-6}$ alkyl which may be unsubstituted or substituted as described above, more suitably H, benzyl or $C_{1-4}$ alkyl optionally substituted with $R^{16}$ or $N(R^{10})(R^{11})$, especially H or methyl or ethyl optionally substituted with $R^{16}$ or $N(R^{10})(R^{11})$.

In some compounds in which $R^3$ is $C(O)NR^{12}S(O)_2R^{13}$, $NHC(O)NR^{12}S(O)_2R^{13}$, $C(O)NR^{12}[CH(R^{15})]_nR^{16}$ or $C(O)NR^{12}C(O)CH_2NR^{12}[CH(R^{15})]_nR^{16}$, $R^{12}$ is more suitably H, methyl or ethyl, especially H or methyl.

In the compounds of general formulae (I), (IA), (IB), (IC) and (ID), $R^{13}$, when present, is suitably a 5- or 6-membered carbocyclyl or heterocyclyl optionally substituted with $R^{16}$ or =O, where =O substituents may be attached to a ring C or S atom; or phenyl optionally substituted with $R^{16}$.

Alternatively, in some suitable compounds of general formulae (I), (IA), (IB), (IC) and (ID) in which $R^3$ is $C(O)NR^{12}R^{13}$ or $S(O)_2NR^{12}R^{13}$, $R^{12}$ and $R^{13}$ together with the nitrogen atom to which they are attached may form a 5- or 6-membered heterocyclic ring optionally substituted with one or more substituents selected from $R^{16}$ and =O and optionally comprising one or more further heteroatoms selected from O, N and S.

In the compounds of general formulae (I), (IA), (IB), (IC) and (ID), $R^{16}$, when present, is more suitably is $C(O)OH$, $S(O)_2OH$, $S(O)_2(C_{1-6}$ alkyl) or $OS(O)_2OH$, especially $C(O)OH$ or $S(O)_2OH$.

When $R^{16}$ is is $C(O)OH$, $S(O)_2OH$, $OS(O)_2OH$ or $P(O)(OH)_2$, the compound of general formula (I), (IA), (IB), (IC) or (ID) may be in salt form. Suitable salts are as discussed above but metal salts are particularly suitable, for example sodium and potassium salts, especially sodium salts.

In some suitable compounds of of general formulae (I), (IA), (IB), (IC) and (ID), $R^3$ is $C(O)OR^{12}$, $C(O)NR^{12}R^{13}$ or $C(O)NR^{12}[CH(R^{15})]_nR^{16}$, wherein:

when $R^3$ is $C(O)OR^{12}$, each $R^{12}$ is independently H or $C_{1-6}$ alkyl optionally substituted by one or more substituents selected from halo, $OR^{10}$, $NR^{10}R^{11}$, $R^{16}$ and aryl;
each $R^{10}$ and $R^{11}$ is independently H or $C_{1-6}$ alkyl; or
when $R^3$ is $C(O)NR^{12}R^{13}$:
each $R^{12}$ is H or $C_{1-6}$ alkyl optionally substituted by one or more substituents selected from halo, $OR^{10}$ and $NR^{10}R^{11}$;
each $R^{10}$ and $R^{11}$ is independently H or $C_{1-6}$ alkyl; and
$R^{13}$ is a 1,1-tetrahydrothiopyran dioxide or a 1,1-tetrahydrothiophene dioxide; or
$R^{12}$ and $R^{13}$ together with the nitrogen atom to which they are attached form a 5- or 6-membered ring containing an $SO_2$ moiety or substituted with C(O)OH; or
when $R^3$ is $C(O)NR^{12}[CH(R^{15})]_nR^{16}$, $R^{12}$ is H or methyl, $R^{15}$ is H and either $R^{16}$ is C(O)OH and n is 1; or
$R^{16}$ is $S(O)_2OH$, $S(O)_2(C_{1-6}$ alkyl) or $OS(O)_2OH$; and n is 2 or 3.
or a pharmaceutically acceptable salt or isotopic variant thereof.

In some particularly suitable compounds of general formulae (I), (IA), (IB), (IC) and (ID), $R^3$ is $C(O)OR^{12}$.

Suitably in these compounds, $R^{12}$ is H or methyl or ethyl optionally substituted with $R^{16}$, and more suitably $R^{12}$ is H, $CH_2R^{16}$ or $-CH_2CH_2R^{16}$ where $R^{16}$ is as defined above but is especially $S(O)_2OH$. Still more suitably, $R^{12}$ is H.

Particularly suitable compounds of this type are those in which $R^3$ is C(O)OH and salts thereof as discussed above, for example metal salts such as sodium and potassium salts, especially sodium salts.

In compounds of general formulae (I), (IA), (IB), (IC) and (ID), where $R^3$ is $C(O)OR^{12}$ and $R^{12}$ is H or $C_{1-6}$ alkyl (e.g. methyl or ethyl) substituted by $R^{16}$ wherein $R^{16}$ is C(O)OH, $S(O)_2OH$, $OS(O)_2OH$ or $P(O)(OH)_2$, the compound may be in salt form. Suitable salts are as discussed above but metal salts are particularly suitable, for example sodium and potassium salts, especially sodium salts.

In other particularly suitable compounds of general formulae (I), (IA), (IB), (IC) and (ID), $R^3$ is $C(O)NR^{12}[CH(R^{15})]_nR^{16}$ and salts thereof as discussed above, for example metal salts such as sodium and potassium salts, especially sodium salts.

In such compounds, $R^{12}$ is more suitably H, methyl or methyl substituted with $R^{16}$, for example $-CH_2C(O)OH$. In some compounds of this type, $R^{12}$ is H; in other compounds of this type, $R^{12}$ is methyl; in still other compounds of this type, $R^{12}$ is $-CH_2R^{16}$. In this case, $R^{15}$ is suitably H and n is suitably 1 such that $R^3$ is $C(O)N(CH_2R^{16})_2$ and the two $R^{16}$ groups may be the same or different but are more suitably the same and are, for example C(O)OH.

When $R^3$ is $C(O)NR^{12}[CH(R^{15})]_nR^{16}$, it may be a group $C(O)NR^{12}CH(R^{14})C(O)OH$ or a group $C(O)NR^{12}[CH(R^{15})]_nR^{16}$, where $R^{12}$ is H or $C_{1-6}$ alkyl, more suitably H or $C_{1-3}$ alkyl, especially H or methyl; n is 2 or 3; each $R^{15}$ is H or $C_{1-6}$ alkyl optionally substituted by one or more halo or aryl groups, more suitably H or $C_{1-6}$ alkyl and still more suitably H; and $R^{16}$ is $S(O)_2OH$, $S(O)_2(C_{1-6}$ alkyl) or $OS(O)_2OH$, especially $S(O)_2OH$, $S(O)_2$(methyl) or $OS(O)_2OH$, especially $S(O)_2OH$.

When $R^3$ is $C(O)NR^{12}CH(R^{14})C(O)OH$, $R^{14}$ is especially the side chain of an amino acid selected from glycine, alanine, valine, leucine or isoleucine, i.e. $R^{14}$ is H, $CH_3$, $CH(CH_3)_2$ or $CH(CH_3)(C_2H_5)$. More suitably, $R^{14}$ is H. Particularly suitable $R^{12}$ moieties are as defined above. When $R^{12}$ is H and $R^{14}$ is H, $R^3$ is a glycine conjugate; and when $R^{12}$ is methyl and $R^{14}$ is H, $R^3$ is an N-methyl glycine conjugate. More suitably, $R^{12}$ is H and $R^3$ is a glycine conjugate.

When $R^3$ is $C(O)NR^{12}[CH(R^{15})]_nR^{16}$, it may be a group $C(O)NR^{12}CH(R^{15})CH(R^{15})S(O)_2OH$, wherein $R^{14}$ is a side chain of an amino acid and $R^{15}$ is H or $C_{1-6}$ alkyl optionally substituted by one or more halo or aryl groups. The compound may be in the form of a salt as discussed above, for example metal salts such as sodium and potassium salts, especially sodium salts.

When $R^3$ is $C(O)NR^{12}CH(R^{15})CH(R^{15})S(O)_2OH$, each $R^{15}$ is suitably H or $C_{1-6}$ alkyl. More suitably, both $R^{15}$ moieties are H. Particularly suitable $R^{12}$ moieties are as defined above but in particularly suitable compounds, $R^{12}$ is H or methyl. When $R^{12}$ is H and both $R^{15}$ moieties are H, $R^3$ is a taurine conjugate. When $R^{12}$ is methyl and both $R^{15}$ moieties are H, $R^3$ is an N-methyl taurine conjugate.

In some suitable compounds in which $R^3$ is $C(O)NR^{12}[CH(R^{15})]_nR^{16}$ each $R^{15}$ is independently H or $C_{1-4}$ alkyl optionally substituted as set out above. More suitably, $R^{15}$ is H or unsubstituted $C_{1-4}$ alkyl, still more suitably H, methyl or ethyl and especially H.

Alternatively, in compounds where $R^3$ is $C(O)NR^{12}[CH(R^{15})]_nR^{16}$ and n is 2 or 3, two $R^{15}$ groups may combine with the carbon atoms to which they are attached, and optionally with an intervening carbon atom where present, to form a carbocyclic ring as set out above. More suitably, the two $R^{15}$ groups are on adjacent carbon atoms. The carbocyclic ring thus formed is suitably a 5- to 7 membered ring, for example a 6-membered ring.

In other particularly suitable compounds of general formulae (I), (IA), (IB), (IC) and (ID), $R^3$ is $C(O)NR^{12}R^{13}$.

In some suitable compounds of this type, $R^{12}$ is H or $C_{1-4}$ alkyl optionally substituted with one or more

substituents, for example a single substituent, selected from $R^{16}$ and $NR^{10}R^{11}$, wherein $R^{16}$, $R^{10}$ and $R^{11}$ are as defined above. $R^{10}$ and $R^{11}$ may be the same or different and are more suitably selected from H and $C_{1-4}$ alkyl, especially $C_{1-3}$ alkyl. More suitable $R^{16}$ groups are as defined above.

In particularly suitable compounds in which $R^3$ is $C(O)NR^{12}R^{13}$, $R^{12}$ is H or $C_{1-3}$ alkyl substituted with a single $R^{16}$ substituent. More suitable $R^{16}$ groups are as defined above and the compound may be present in the form of a salt as discussed above, for example a metal salt such as a sodium or potassium salt, especially a sodium salt.

When $R^3$ is $C(O)NR^{12}R^{13}$, $R^{13}$ is suitably phenyl, or a 5- to 7-membered cycloalkyl or heterocyclyl group, any of which may optionally be substituted with a single $R^{16}$ substituent and where cycloalkyl and heterocyclyl groups may be substituted with one or more =O substituents.

More suitably, the cycloalkyl group is unsubstituted cyclopentyl, cyclohexyl or cycloheptyl.

More suitably, the heterocyclyl group is a 5- or 6-membered sulfur containing group such as tetrahydrothiophene and tetrahydrothiopyran, or oxides thereof, such as 1,1-dioxotetrahydrothiophine and 1,1-dioxotetrahydropyran.

In other suitable compounds in which $R^3$ is $C(O)NR^{12}R^{13}$, each $R^{12}$ is H or $C_{1-6}$ alkyl optionally substituted by one or more substituents selected from halo, $OR^{10}$ and $NR^{10}R^{11}$; each $R^{10}$ and $R^{11}$ is independently H or $C_{1-6}$ alkyl; and

$R^{13}$ is a 1,1-tetrahydrothiopyran dioxide or a 1,1-tetrahydrothiophene dioxide; or

$R^{12}$ and $R^{13}$ together with the nitrogen atom to which they are attached form a 5- or 6-membered ring containing an $SO_2$ moiety or substituted with C(O)OH.

In these compounds, $R^{12}$ is more suitably H and and $R^{13}$ is a 1,1-tetrahydrothiopyran dioxide ring. Alternatively, $R^{12}$ and $R^{13}$ together with the nitrogen atom to which they are attached form a 6-membered ring containing an $SO_2$ moiety, especially a thiomorpholine dioxide ring, or piperidine substituted with C(O)OH.

Particularly preferred $R^3$ groups include C(O)OH, $C(O)NHCH_2C(O)OH$, $C(O)N(CH_3)CH_2C(O)OH$, $C(O)NHCH_2CH_2S(O)_2OH$ and $C(O)N(CH_3)CH_2CH_2S(O)_2OH$, especially C(O)OH, $C(O)NHCH_2C(O)OH$, $C(O)NHCH_2CH_2S(O)_2OH$ and $C(O)N(CH_3)CH_2CH_2S(O)_2OH$.

In one embodiment, the compound of formula (I) is selected from the group consisting of:

2β-fluorochenodeoxycholic acid (Compound 1);
2β-fluoro-3β,7α-dihydroxy-5β-cholanic acid (Compound 2);
2α-fluoro-3β,7α-dihydroxy-5β-cholanic acid (Compound 3);
2α-fluoro-3β,7β-dihydroxy-5β-cholanic acid (Compound 4);
2α-fluoro-3α,7α-dihydroxy-5β-cholanic acid (Compound 5);
2α-fluoro-3α,7β-dihydroxy-5β-cholanic acid (Compound 6);
2,2-difluoro-3β,7β-dihydroxy-5β-cholanic acid (Compound 7);
2,2-difluoro-3α,7α-dihydroxy-5β-cholanic acid (Compound 8);
2,2-difluoro-3α,7β-dihydroxy-5β-cholanic acid (Compound 9);
*N*-(2,2-difluoro-3β,7β-dihydroxy-5β-cholan-24-amide)-ethylsulfonic acid (Compound 10);
*N*-(2,2-difluoro-3β,7β-dihydroxy-5β-cholan-24-amide)-propanoic acid (Compound 11);
*N*-(methyl),*N*-(2,2-difluoro-3β,7β-dihydroxy-5β-cholan-24-amide)-acetic acid (Compound 12);
*N*-(2,2-difluoro-3β,7β-dihydroxy-5β-cholan-24-amide)-*trans*-2-cyclohexane carboxylic acid (Compound 13);
1-(2,2-difluoro-3β,7β-dihydroxy-5β-cholan-24-oyl)-piperidine-3-carboxylic acid (Compound 14);
3-(2,2-difluoro-3β,7β-dihydroxy-5β-cholan-24-amide)-4-thiazolidine-carboxylic acid (Compound 15);
*N*-(2,2-difluoro-3β,7β-dihydroxy-5β-cholan-24-oyl)-morpholine (Compound 16);
*N*-(2,2-difluoro-3β,7β-dihydroxy-5β-cholan-24-amide)-methylcarboxylic acid (Compound 17)
*N*-(carboxymethyl)-*N*-(2,2-difluoro-3β,7β-dihydroxy-5β-cholan-24-oyl)-2-amino acetic acid (Compound 18);
*N*-(methyl)-*N*-(2,2-difluoro-3β,7β-dihydroxy-5β-cholan-24-amide) ethylsulfonic acid (Compound 19);
3-(2,2-difluoro-3β,7β-dihydroxy-5β-cholan-24-oyl) amino-propanesulfonic acid (Compound 20);
*N*-(2,2-difluoro-3β,7β-dihydroxy-5β-cholan-24-amide) methanesulfonic acid (Compound 21);
*N*-(2,2-difluoro-3β,7β-dihydroxy-5β-cholan-24-oyl)-2-aminoethyl sulfuric acid (Compound 22);
*O*-(2,2-difluoro-3β,7β-dihydroxy-5β-cholan-24-oyl)-2-hydroxy ethyl sulfonic acid (Compound 23);
*N*-(2,2-difluoro-3β,7β-dihydroxy-5β-cholan-24-oyl) aniline-2-sulfonic acid (Compound 24);
*N*-(cyclohexyl)-*N*-(2,2-difluoro-3β,7β-dihydroxy-5β-cholan-24-oyl)-3-amino-propanesulfonic acid (Compound 25);
N-(cyclohexyl)-*N*-(2,2-difluoro-3β,7β-dihydroxy-5β-cholan-24-oyl)-2-aminoethanesulfonic acid (Compound 26);
*N*-(2,2-difluoro-3β,7β-dihydroxy-5β-cholan-24-oyl) 2-aminoethyl methyl sulfone (Compound 27);
*N*-(ethyl)-*N*-(2,2-difluoro-3β,7β-dihydroxy-5β-cholan-24-oyl)-3-amino-tetrahydrothiophene dioxide (Compound 28);
*N*-(2-(diisopropylamino)ethyl)-*N*-(2,2-difluoro-3β,7β-dihydroxy-5β-cholan-24-oyl)-3-amino-tetrahydrothiophene dioxide (Compound 29);
*N*-(2,2-difluoro-3β,7β-dihydroxy-5β-cholan-24-oyl)-thiomorpholine-dioxide (Compound 30);

*N*-(2,2-difluoro-3β,7β-dihydroxy-5β-cholan-24-oyl) 1,1-dioxidotetrahydro-2H-thiopyran-3-ylamine (Compound 31); and

pharmaceutically acceptable salts thereof (where appropriate), especially metal salts, such as sodium or potassium salts, particularly sodium salts or (for Compound 18) the disodium salt.

Methods of preparing the compounds of general formula (I) are described below. These methods form a further aspect of the invention.

Compounds of general formulae (IB) and (IC) in which $R^1$ is F and $R^3$ is $C(O)OR^{12a}$, wherein $R^{12a}$ is $C_{1-6}$ alkyl optionally substituted by one or more halo or aryl groups, may be prepared from compounds of general formula (II):

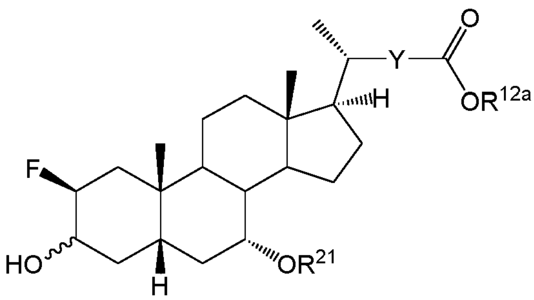

(II)

wherein Y and $R^3$ are as defined for general formula (I); $R^{12a}$ is $C_{1-6}$ alkyl optionally substituted by one or more halo or aryl groups; and $R^{21}$ is an OH protecting group which is acid labile;

by treatment with an acid, for example hydrochloric acid as described in General Procedure L below.

Suitable acid labile protecting groups $R^{21}$ include alkyl ethers, for example methoxymethyl.

Compounds of general formula (II) may be formed as a mixture of isomers of general formulae (IIB) and (IIC):

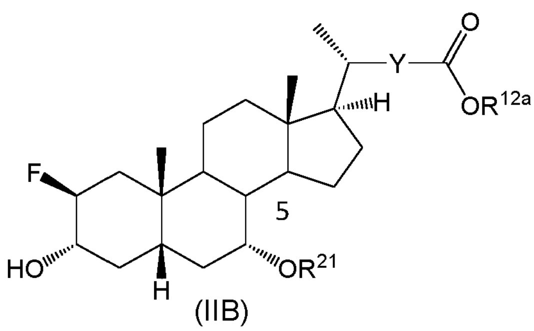

(IIB)

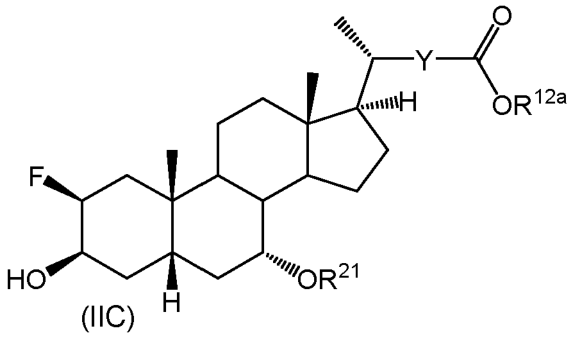

(IIC)

wherein Y and $R^3$ are as defined for general formula (I) and $R^{12a}$ and $R^{21}$ are as defined for general formula (II);

by reduction of a compound of general formula (III):

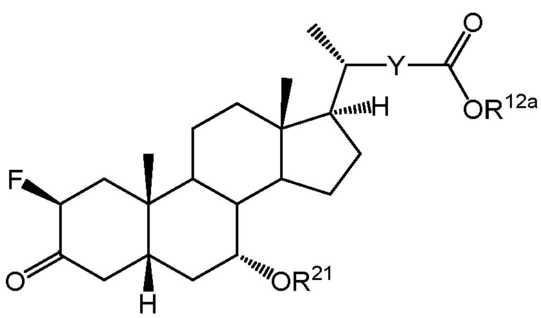

(III)

wherein Y and $R^3$ are as defined for general formula (I) and $R^{12a}$ and $R^{21}$ are as defined for general formula (II).

Removal of the protecting groups $R^{21}$ from the compounds of general formulae (IIB) and (IIC) as describes above yields compounds of general formulae (IB) and (IC) respectively.

Suitable reducing agents for the reduction of the compounds of general formula (III) include hydrides, for example sodium borohydride. The reduction may be carried out in an organic solvent such as tetrahydrofuran at a temperature of about 15 to 25 °C, suitably at room temperature.

Compounds of general formula (III) may be prepared from compounds of general formula (IV):

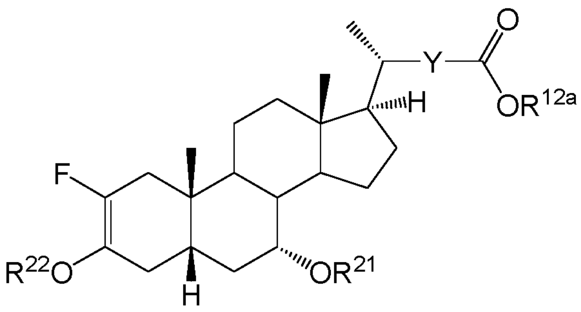

(IV)

wherein Y and $R^3$ are as defined for general formula (I); $R^{12a}$ and $R^{21}$ are as defined for general formula (II); and $R^{22}$ is an OH protecting group;

by fluorination using an agent such as Selectfluor®, (1-chloromethyl-4-fluoro-1,4-diazoniabicyclo[2.2.2]octane bis(tetrafluoroborate), which has the structure:

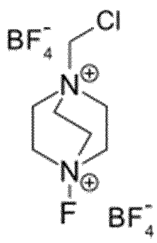

When using Selectfluor®, the reaction is suitably carried out at a temperature of 15 to 25 °C, typically at room temperature, in a polar organic solvent such as acetonitrile.

Suitable protecting groups $R^{22}$ include silyl protecting groups $Si(R^{23})_3$, wherein each $R^{23}$ is independently $C_{1-6}$ alkyl or phenyl.

Examples of $R^{22}$ groups include trimethylsilyl (TMS), triethylsilyl (TES), triphenylsilyl (TPS), tri-isopropylsilyl (TIPS), thexyldimethylsilyl (TDS), tert-butyldiphenylsilyl (TBDPS), *tert*butyldimethylsilyl (TBDMS or TBS), di-tert-butylmethylsilyl (DTBMS), diethylisopropylsilyl (DEIPS) and dimethylisopropylsilyl (DMIPS), in particular TMS, TES, TIPS, TBDMS and TBDPS.

Compounds of general formula (IV) may be prepared from compounds of general formula (V):

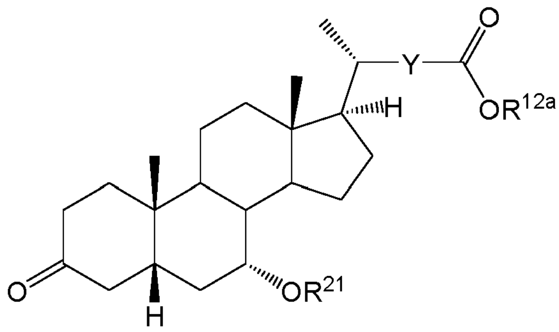

(V)

wherein Y and $R^3$ are as defined for general formula (I); $R^{12a}$ and $R^{21}$ are as defined for general formula (II); by reaction with a compound of general formula (VI):

$(R^{23})_3Si-OR^{24}$ (VI)

wherein each $R^{23}$ is independently as defined above and $R^{24}$ is a leaving group such as trifluoromethanesulfonate (triflate), toluene sulfonyl (tosyl) or methane sulfonyl (mesyl).

The reaction is suitably carried out under basic conditions, for example in the presence of a weak base such as triethylamine at a temperature of about 15 to 25 °C, suitably at room temperature.

Compounds of general formula (V) may be prepared from chenodeoxycholic acid by esterification of the carboxylic acid by reaction with an alcohol $R^{12a}OH$, for example as described in General Procedure A below, followed by protection of the 7-OH group, by reaction with a compound of general formula (VII):

$R^{21}$-X (VII)

wherein $R^{21}$ is as defined for general formula (II) and X is a leaving group, typically halo, for example chloro; for example as described in General Procedure K below. This may be followed by oxidation of the 3-OH group as described in General Procedure M below.

Compounds of general formula (I) in which $R^2$ is F and Y is C(O)$OR^{12a}$, wherein $R^{12a}$ is as defined above, may be prepared as set out below.

Compounds of general formulae (IA) and (IC) in which $R^2$ is F and $R^3$ is C(O)$OR^{12a}$ may be prepared by reduction of compounds of general formula (XIIa):

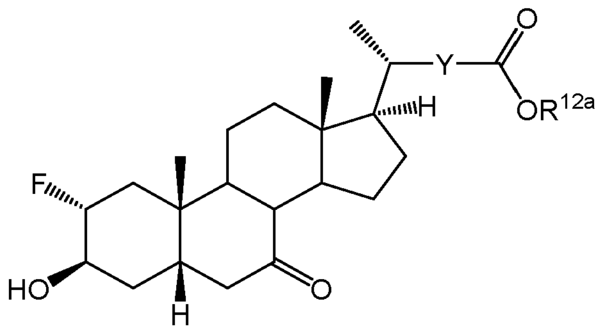

(XIIa)

wherein Y is as defined for general formula (I) and $R^{12a}$ is as defined for general formula (II).

Suitably, the reduction is carried out using a hydride, for example sodium borohydride, in the presence of cerium (III) chloride. The reaction is suitably carried out at a temperature of about 15 to 25 °C, suitably at room temperature.

The product obtained is a mixture of compounds of general formula (IA) and general formula (IC), which can be separated by conventional methods, for example by chromatography.

The compound of general formula (XIIa) may also be used to prepare compounds of general formulae (IB) and (ID) in which $R^2$ is F and $R^3$ is C(O)$OR^{12a}$. In this case, the compound of general formula (XIIa) may be reacted with a

carboxylic acid of general formula (XIII):

$R^{25}$-C(O)OH (XIII)

wherein $R^{25}$ is $C_{1-6}$ alkyl or benzyl, but more suitably benzyl;
in the presence of triphenyl phosphine and diethyl azodicarboxylate (DEAD) in a Mitsunobu type reaction to give a compound of general formula (XIV):

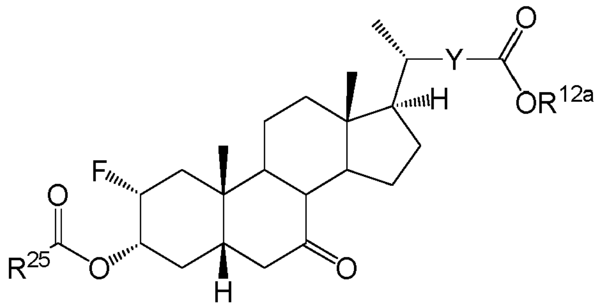

(XIV)

wherein Y is as defined for general formula (I); $R^{12a}$ is as defined for general formula (II) and $R^{25}$ is as defined for general formula (XIII).

The compound of general formula (XIV) may be hydrolysed using a mild base such as potassium carbonate to give a compound of general formula (XIIb):

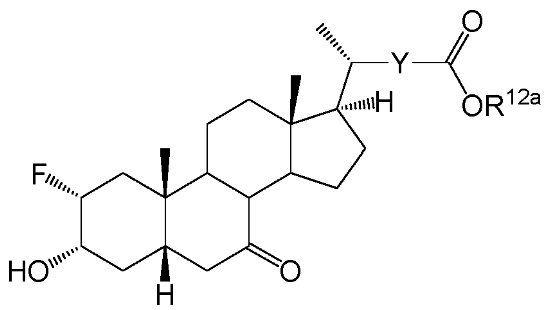

(XIIb)

wherein Y is as defined for general formula (I) and $R^{12a}$ is as defined for general formula (II).

The compound of general formula (XIIb) may then be reduced to give a mixture of compounds of general formula (IB) and general formula (ID) using the conditions described above for the reduction of the compound of general formula (XIIa).

A compound of general formula (XIIa) may be prepared from a compound of general formula (XIV):

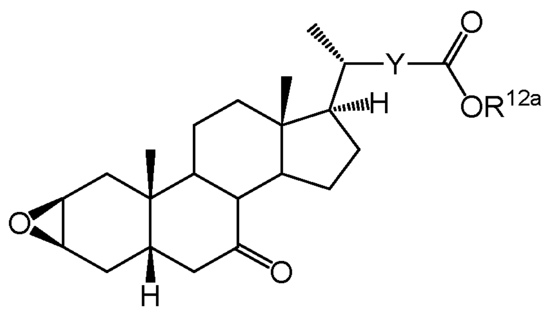

(XIV)

wherein Y is as defined for general formula (I) and $R^{12a}$ is as defined for general formula (II);
by reaction with hydrogen fluoride pyridine (HF.pyridine).

Suitably, the reaction is conducted in a dry organic solvent such as dichloromethane (DCM).

A compound of general formula (XIV) may be prepared by epoxidation of a compound of general formula (XV):

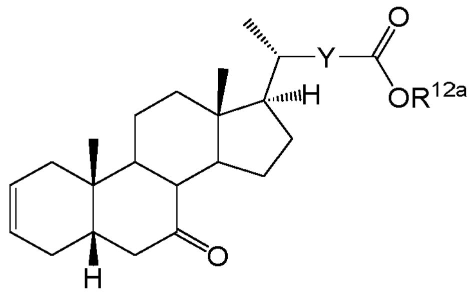

(XV)

wherein Y is as defined for general formula (I) and $R^{12a}$ is as defined for general formula (II).

A suitable oxidising agent is meta-chloroperbenzoic acid (mCPBA) and the reaction maybe carried out in an organic solvent such as DCM and at a temperature of about 15 to 25 °C, suitably at room temperature.

A compound of general formula (XV) may be prepared from a compound of general formula (XVI):

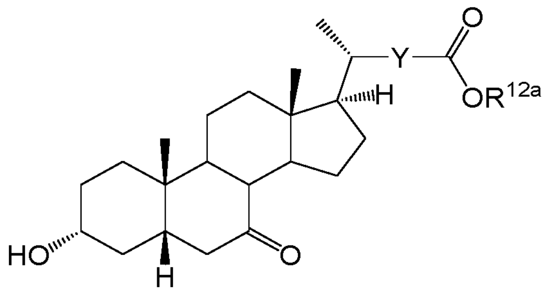

(XVI)

wherein Y is as defined for general formula (I) and $R^{12a}$ is as defined for general formula (II);
by an elimination reaction.

Trifluoromethanesulfonic anhydride (triflic anhydride) is an example of a suitably activated leaving group, which may be used in combination with a base such as dimethylaminopyridine (DMAP). The reaction may be carried out at a temperature of about 5 to 20 °C, suitably 10 to 15 °C.

Compounds of general formula (XVI) may be prepared from 7-ketolithocholic acid by esterification as described in General Procedure A below. 7-ketolithocholic acid is commercially available.

Compounds of general formula (IA) in which both $R^1$ and $R^2$ are fluoro and $R^3$ is $C(O)OR^{12a}$, wherein $R^{12a}$ is as defined for general formula (II) may be prepared from compounds of general formula (XXI):

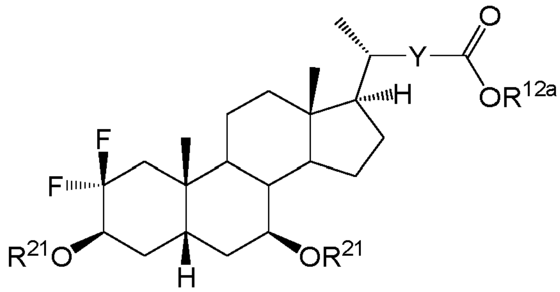

(XXI)

wherein Y is as defined for general formula (I) and $R^{12a}$ and $R^{21}$ are as defined for general formula (II); by reaction with an acid, for example hydrochloric acid, as described in General Procedure L below.

Compounds of general formula (IA) may be converted to compounds of general formulae (IB) and (ID) using the following procedure.

The compound of general formula (IA) may be oxidised to give a compound of general formula (XXII):

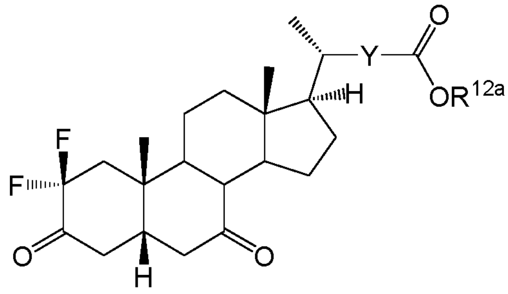

(XXII)

wherein Y is as defined for general formula (I) and $R^{12a}$ and $R^{21}$ are as defined for general formula (II).

Suitable oxidising agents for this process include Dess-Martin periodinane and the reaction conditions for this are as described below in General Procedure M.

The diketone of general formula (XXII) may then be reduced to give a mixture of Compounds of general formulae (IB) and (ID). Suitable reducing agents for this process include a hydride, for example sodium borohydride, in the presence of cerium (III) chloride as described in General Procedure B below. The compounds of general formulae (IB) and (ID) may be separated by conventional methods, for example chromatographic methods.

Compounds of general formula (XXI) may be prepared by fluorination of compounds of general formula (XXIII):

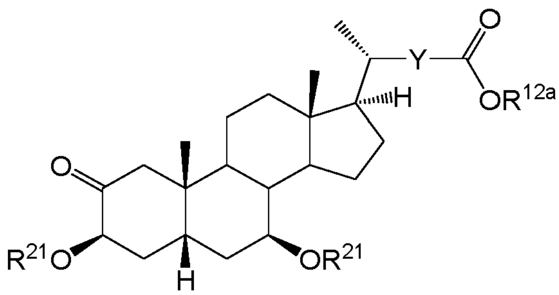

(XXIII)

wherein Y is as defined for general formula (I) and $R^{12a}$ and $R^{21}$ are as defined for general formula (II).

Suitable fluorinating agents for this reaction include N,N-diethylaminosulfur trifluoride (DAST). Reaction with DAST may take place in an organic solvent, for example dichloromethane at a temperature of about 15 to 25 °C, typically at room temperature.

Compounds of general formula (XXIII) may be prepared by oxidation of compounds of general formula (XXIV):

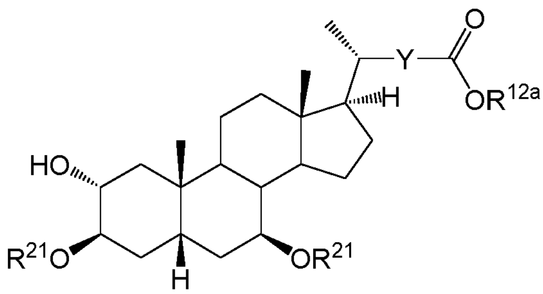

(XXIV)

wherein Y is as defined for general formula (I) and $R^{12a}$ and $R^{21}$ are as defined for general formula (II).

Suitable oxidising agents include Dess-Martin periodinane as described in General Procedure M below.

Compounds of general formula (XXIV) may be prepared by reaction of a compound of general formula (XXV):

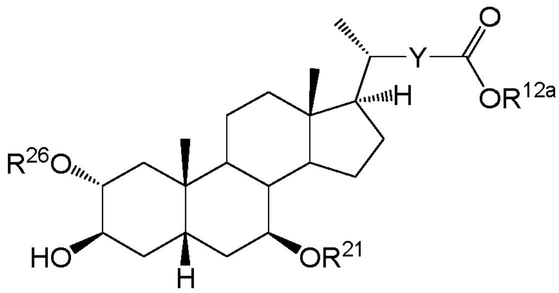

(XXV)

wherein Y is as defined for general formula (I); $R^{12a}$ and $R^{21}$ are as defined for general formula (II); and $R^{26}$ is a base labile protecting group;

with a compound of general formula (VII) as described above, followed by reaction with a base to remove the protecting group $R^{26}$.

Examples of protecting groups $R^{26}$ include acyl groups $R^{25}C(O)$-, wherein $R^{25}$ is as defined above for general formula (XIII).

The reaction of the compound of general formula (XXV) with the compound of general formula (VII) may be carried out using the method of General Procedure K below.

The protecting group $R^{26}$ may be removed by a base such as an alkoxide, for example a sodium or potassium alkoxide, typically a sodium alkoxide for example sodium methoxide or sodium ethoxide. Suitably the deprotection is carried out in an alcoholic solvent such as methanol or ethanol at a temperature of about 15 to 25°C, typically at room temperature.

The compound of general formula (XXV) may be prepared from a compound of general formula (XXVI):

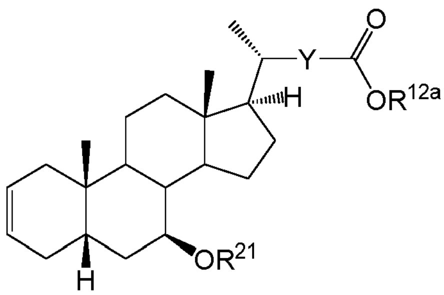

(XXVI)

wherein Y is as defined for general formula (I); $R^{12a}$ and $R^{21}$ are as defined for general formula (II);

by epoxidation to give a compound of general formula (XXVIa):

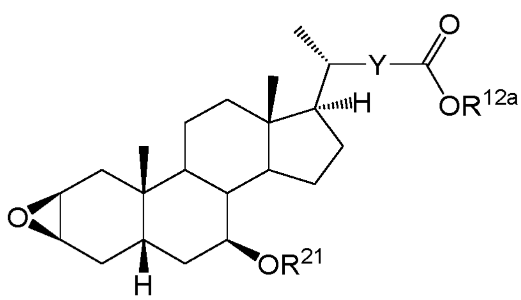

(XXVIa)

wherein Y is as defined for general formula (I); $R^{12a}$ and $R^{21}$ are as defined for general formula (II);
followed by ring opening by reaction with a compound of general formula (XXVII):

$R^{26}$-OH (XXVII)

wherein $R^{26}$ is as defined above for general formula (XXV).

A suitable oxidising agent is meta-chloroperbenzoic acid (m-CPBA) and the reaction maybe carried out in an organic solvent such as DCM and at a temperature of about 15 to 25 °C, suitably at room temperature. The reaction results in the production of an inseparable mixture of a Δ2β,3β-epoxide (XXVIa) and a Δ3β,4β-epoxide. On treatment of the mixture with a compound of general formula (XXVII), the compound of general formula (XXVIa) reacts to give the required product.

In the ring opening reaction, when the protecting group $R^{26}$ is an acyl group $R^{25}C(O)$-, the compound of formula (XXVII) is a compound of general formula (XIII) as defined above. Reaction of the compound of general formula (XXVIa) with a compound of general formula (XIII) may take place at elevated temperature, for example about 40 to 60°C, typically at about 50°C.

A compound of general formula (XXVI) may be prepared from a compound of general formula (XXVIII):

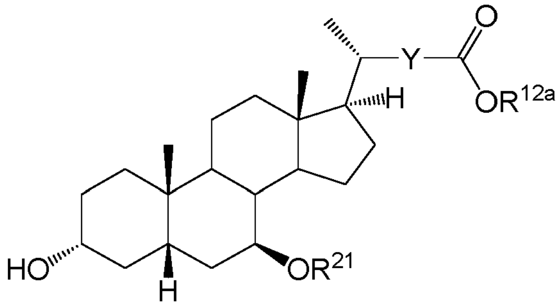

(XXVIII)

wherein Y is as defined for general formula (I); $R^{12a}$ and $R^{21}$ are as defined for general formula (II);
by an elimination reaction.

Trifluoromethanesulfonic anhydride (triflic anhydride) is an example of a suitably activated leaving group, which may be used in combination with a base such as lutidine. The reaction may be carried out at a temperature of about 5 to 20 °C, suitably 10 to 15°C.

A compound of general formula (XXVIII) may be prepared from a compound of general formula (XXIX):

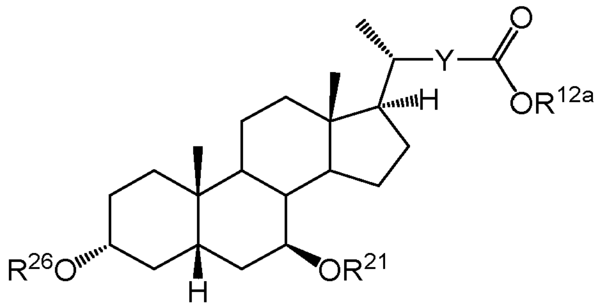

(XXIX)

wherein Y is as defined for general formula (I); $R^{12a}$ and $R^{21}$ are as defined for general formula (II); and $R^{26}$ is as defined for general formula (XXV);
by reaction with a compound of general formula (XXVII) as defined above.

Suitably, the compound of general formula (XXVII) is a carboxylic acid of general formula (XIII) such that $R^{26}$ is an acyl group of formula $R^{25}C(O)$-.

The reaction may take place in an alcoholic solvent such as methanol or ethanol at a temperature of about 15 to 25°C, typically at room temperature.

A compound of general formula (XXIX) may be prepared from a compound of general formula (XXX):

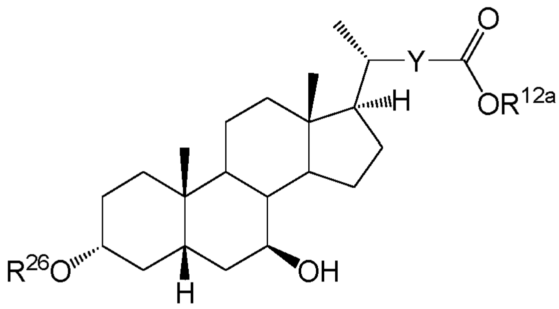

(XXX)

wherein Y is as defined for general formula (I); $R^{12a}$ is as defined for general formula (II); and $R^{26}$ is as defined for general formula (XXV);

by reaction with a compound of general formula (VII). Suitable reaction conditions are as described in General Procedure K below.

A compound of general formula (XXX) may be prepared from a compound of general formula (XXXI):

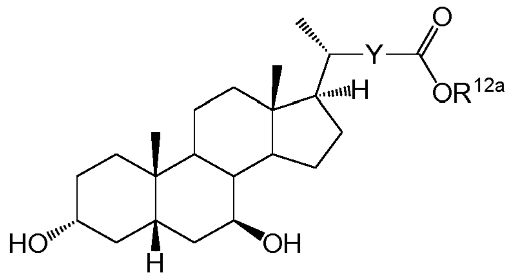

(XXXI)

wherein $R^{12a}$ is as defined for general formula (II);

by reaction with a compound of general formula (XXVII) or, more usually, with a compound of general formula (XXXII):

$(R^{26})_2O$ (XXXIII)

wherein $R^{26}$ is as defined for general formula (XXV).

When $R^{26}$ is an acyl group $R^{26}$, the reagent of general formula (XXXIII) is a carboxylic anhydride.

The compound of general formula (XXXI) is an ester of UDCA, which may be prepared from UDCA by reaction with an alcohol $R^{12a}OH$, for example as described in General Procedure A.

Compounds of general formulae (I), (IA), (IB), (IC) and (ID) in which $R^3$ is $C(O)OR^{12a}$, wherein $R^{12a}$ is $C_{1-6}$ alkyl optionally substituted by one or more halo or aryl groups, may be converted to other compounds of general formulae(I), (IA), (IB), (IC) and (ID).

Compounds of general formulae (I), (IA), (IB), (IC) and (ID) in which $R^3$ is C(O)OH may be prepared by hydrolysis of the equivalent compound in which $R^3$ is $C(O)OR^{12a}$. The hydrolysis may be acid or base hydrolysis. Base hydrolysis is often more suitable and may be conducted, for example, using an alkali metal hydroxide such as lithium, sodium or potassium hydroxide, more usually lithium hydroxide. Base hydrolysis is described in General Procedure C below.

Compounds of general formula (I) in which $R^3$ is $C(O)NR^{12}R^{13}$ may be prepared from the carboxylic acid by reaction with an amine of formula $H-NR^{12}R^{13}$ wherein $R^{12}$ and $R^{13}$ are as defined above for general formula (I); in a suitable solvent with heating. Suitably, the reaction is carried out in the presence of a coupling reagent and under basic conditions, for example in the presence of an amine such as diisopropylethylamine (DIPEA) or triethylamine (TEA) and in an organic solvent such as DMF as described in General Procedure Q below.

Suitable coupling reagents include known peptide coupling agents such as O-(Benzotriazol-1-yl)-N,N,N',N'-tetramethyluronium hexafluorophosphate (HBTU), O-(Benzotriazol-1-yl)- N,N,N',N'-tetramethyluronium tetrafluoroborate (TBTU), O-(7-Azabenzotriazol-1-yl)-N,N,N',N'-tetramethyluronium hexafluorophosphate (HATU), O-(7-Azabenzotriazol-1-yl)- N,N,N',N'-tetramethyluronium tetrafluoroborate (TATU), (Benzotriazol-1-yloxy)tris(dimethylamino)phosphonium hexafluorophosphate (BOP), (Benzotriazol-1-yloxy)tripyrrolidinophosphonium hexafluorophosphate (PyBOP) carbodiimides such as 1-ethyl-3-(3-dimethylaminopropyl)carbodiimide (EDCI) and triazoles such as 1-hydroxy-7-azabenzotriazole (HOAt) or hydroxybenzotriazole (HOBt); and chloroformates such as isobutyl chloroformate.

Amines of formula H-$NR^{12}R^{13}$ are known and are readily available or may be prepared by methods known to those of skill in the art.

Compounds of general formula (I) in which $R^7$ is C(O)$NR^{12}R^{13}$ or $OS(O)_2OR^{12}$ may also be prepared by methods similar to those described by Festa et al., J. Med. Chem., 2014, 57, 8477-8495.

Compounds of general formulae (I), (IA), (IB), (IC) and (ID) in which $R^3$ is C(O)$NR^{12}[CH(R^{15})]_nR^{16}$ may be prepared from compounds of general formulae (I), (IA), (IB), (IC) and (ID) in which $R^3$ is C(O)OH by reaction with a compound of general formula (XL):

$$HNR^{12}[CH(R^{15})]_nR^{16} \qquad (XL)$$

wherein $R^{12}$, $R^{15}$, n and $R^{16}$ are as defined above;
in the presence of a coupling reagent and under basic conditions, for example in the presence of an amine such as diisopropylethylamine (DIPEA) or triethylamine (TEA) and in an organic solvent such as DMF as described in General Procedure Q below. Suitable coupling agents are as described above.

For example, compounds of general formulae (I), (IA), (IB), (IC) and (ID) in which $R^3$ is C(O)$NR^{12}CH(R^{14})C(O)OH$ may be prepared from compounds of general formulae (I), (IA), (IB), (IC) and (ID) in which $R^3$ is C(O)OH by reaction with an amino acid of general formula (XLI):

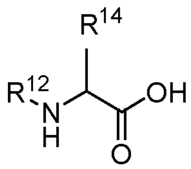

(XLI)

wherein $R^{12}$ and $R^{14}$ are as defined above;
in the presence of a coupling reagent and under basic conditions, for example in the presence of an amine such as diisopropylethylamine (DIPEA) or triethylamine (TEA) and in an organic solvent such as DMF as described in General Procedure Q below. Suitable coupling agents are as described above, with HATU being particularly suitable.

Suitably, in the compound of general formula (XLI), $R^{12}$ is H or methyl and $R^{14}$ is H.

Similarly, compounds of general formulae (I), (IA), (IB), (IC) and (ID) in which $R^3$ is C(O)$NR^{12}CH(R^{15})CH(R^{15})S(O)_2OH$ may be prepared from compounds of general formulae (I), (IA), (IB), (IC) and (ID) in which $R^3$ is C(O)OH by reaction with a compound of general formula (XLII):

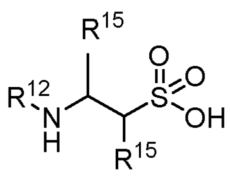

(XLII)

wherein $R^{12}$ and $R^{15}$ are as defined above;
in the presence of a coupling reagent and under basic conditions, for example in the presence of an amine such as diisopropylethylamine (DIPEA) or triethylamine (TEA) and in an organic solvent such as DMF as described in General Procedure Q below. Suitable coupling agents are as described above, with HATU and isobutyl chloroformate being particularly suitable.

Suitably, in the compound of general formula (XLII), $R^{12}$ is H or methyl and each $R^{15}$ is H.

Amino acids of general formula (XLI) and taurine and its derivatives of general formula (XLII) are well known and are readily available or may be synthesised by methods known in the art.

Compounds of general formulae (I), (IA), (IB), (IC) and (ID) in which $R^3$ is C(O)OH may be converted to compounds in which $R^3$ is $C(O)NR^{12}S(O)_2R^{13}$, wherein $R^{13}$ is as defined above, by reaction with a compound of formula:

$$NHR^{12}S(O)_2R^{13}$$

wherein $R^{12}$ and $R^{13}$ are as defined above, in the presence of a coupling reagent and under basic conditions, for example in the presence of an amine such as diisopropylethylamine (DIPEA) or triethylamine (TEA) and in an organic solvent such as DMF as described in General Procedure Q below. Suitable coupling agents are as described above, with 1-ethyl-3(3-dimethylaminopropyl)carbodiimide (EDCI) being particularly suitable.

Compounds of general formulae (I), (IA), (IB), (IC) and (ID) in which $R^3$ is $NHC(O)NR^{12}S(O)_2R^{13}$ may be prepared from compounds of general formulae (I), (IA), (IB), (IC) and (ID) in which $R^3$ is C(O)OH by a process as shown in Scheme 1:

Scheme 1

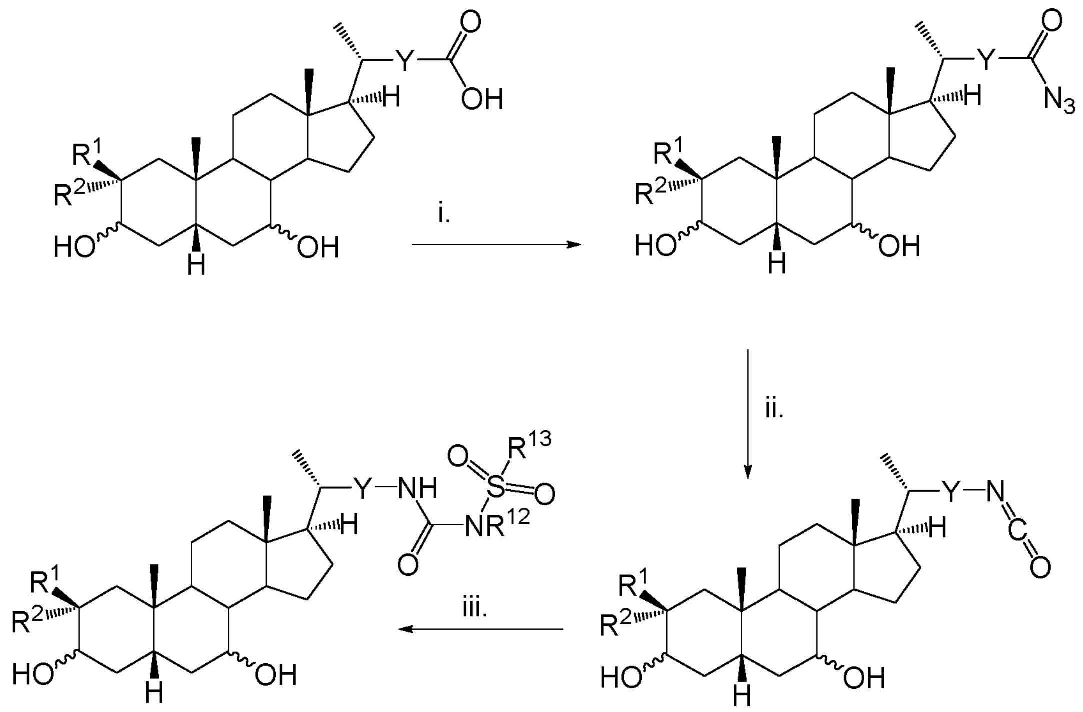


i diphenyl phosphoryl azide / triethylamine
ii heat
iii $R^{13}SO_2NHR^{12}$

Compounds of general formulae (I), (IA), (IB), (IC) and (ID) in which $R^3$ is $S(O)_2OR^{12}$ may be synthesised from compounds of general formulae (I), (IA), (IB), (IC) and (ID) in which $R^3$ is C(O)OH. The compound in which $R^3$ is C(O)OH may first be reacted with a $C_{1-6}$ alkanoyl or benzoyl chloride or with a $C_{1-6}$ alkanoic anhydride to protect any OH groups. The protected compound may then be reacted with a reducing agent such as a hydride, suitably lithium aluminium hydride or sodium borohydride in order to reduce the carboxylic acid group to OH. The alcohol group may be replaced by a halogen, for example bromine or iodine, for example using the triphenyl phosphine/imidazole/halogen method described by Classon et al., J. Org. Chem., 1988, 53, 6126-6130. The halogenated compound may then be reacted with sodium sulphite in an alcoholic solvent to give a compound with a $SO_3^-$ $Na^+$ substituent.

Compounds of general formulae (I), (IA), (IB), (IC) and (ID) in which $R^3$ is $OS(O)_2OR^{12}$ can be obtained by protecting the OH groups of a compound of general formulae (I), (IA), (IB), (IC) or (ID) in which $R^3$ is $C(O)OR^{12}$ using any suitable protecting group; reducing the carboxylic acid or ester to obtain an alcohol and reacting this with chlorosulfonic acid in the presence of a base such as triethylamine to yield the protected triethylamine salt of the compound in which $R^3$ is

is $OS(O)_2OR^{12}$. The protecting groups can be removed using base hydrolysis.

Reaction of the alcohol with a sulfonyl chloride yields a compound of general formula (I), (IA), (IB), (IC) or (ID) in which $R^3$ is $OS(O)_2R^{12}$.

Compounds of general formulae (I), (IA), (IB), (IC) and (ID) in which $R^3$ is $S(O)_2R^{12}$ can be obtained from the alcohol by reaction with Lawesson's reagent followed by oxidation of the resultant product.

Surprisingly, it has been shown that the compounds of the invention are able to restore mitochondrial function and can cross the blood brain barrier. They are therefore of use in the treatment of neurodegenerative disorders including Parkinson's disease, mild cognitive impairment, dementia (including Alzheimer's disease, vascular dementia and dementia with Lewy bodies), Huntington's disease and amyotrophic lateral sclerosis (motor neurone disease).

The compounds of the invention are surprisingly active when compared with bile acids with slightly different substitutions on the A and B rings.

In a further aspect of the invention, there is provided a compound of general formula (I) for use in medicine.

There is also provided a compound of general formula (I) for use in the treatment or prevention of a neurodegenerative disorder.

The invention also provides the use of a compound of general formula (I) in the preparation of an agent for the treatment or prevention of a neurodegenerative disorder.

The invention further provides a method for the treatment or prevention of a neurodegenerative disorder, the method comprising administering to a patient in need of such treatment an effective amount of a compound of general formula (I).

Examples of neurodegenerative disorders include Parkinson's disease, mild cognitive impairment, dementia (including Alzheimer's disease, vascular dementia and dementia with Lewy bodies), Huntington's disease, amyotrophic lateral sclerosis (motor neurone disease), progressive supranuclear palsy and Wilson's disease. Disorders which are particularly suitable for treatment with the compounds of the present invention include Parkinson's disease, mild cognitive impairment, dementia (including Alzheimer's disease, vascular dementia and dementia with Lewy bodies), Huntington's disease and amyotrophic lateral sclerosis and especially Parkinson's disease, mild cognitive impairment and dementia (including Alzheimer's disease, vascular dementia and dementia with Lewy bodies).

Compounds of general formula (IA) and (ID) are particularly effective in the treatment or prevention of Parkinson's disease, especially compounds of general formula (IA) and (ID) in which both $R^1$ and $R^2$ are F.

Examples of particularly suitable compounds for use in treating Parkinson's disease include the compound of general formula (IA) which is 2,2-difluoro-3β,7β-dihydroxy-5β-cholanic acid (Compound 7) and the compound of general formula (ID) which is 2,2-difluoro-3α,7β-dihydroxy-5β-cholanic acid (Compound 9). 2,2-Difluoro-3β,7β-dihydroxy-5β-cholanic acid (Compound 7) is particularly suitable.

Compounds of general formula (IB) are particularly effective in the treatment or prevention of dementia, for example Alzheimer's disease. This is especially the case for compounds of general formula (IB) in which both $R^1$ and $R^2$ are F.

Suitably, when the neurodegenerative disorder is a dementia, especially Alzheimer's disease, the compound of general formula (IB) is 2,2-difluoro-3α,7α-dihydroxy-5β-cholanic acid (Compound 8).

The compounds of general formula (I) will generally be administered as part of a pharmaceutical composition.

Therefore, in a further aspect of the invention, there is provided a pharmaceutical composition comprising a compound of general formula (I) and a pharmaceutically acceptable excipient or carrier.

The composition may be formulated for administration by any route, for example parenteral, including intravenous, intramuscular, subcutaneous or intradermal; or oral, rectal, nasal, topical (including eye drops, topical administration to the lung, buccal and sublingual) or vaginal administration.

More suitably, the composition is formulated for parenteral administration or for topical administration to the lung (by inhalation).

The composition may be prepared by bringing into association the above defined active agent with the carrier. In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of general formula (I) in conjunction or association with a pharmaceutically acceptable carrier or vehicle.

Formulations for oral administration in the present invention may be presented as: discrete units such as capsules, sachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion; or as a bolus etc.

In some cases, the compositions may be formulated for delayed, slow or controlled release of the compound of general formula (I).

For compositions for oral administration (e.g. tablets and capsules), the term "acceptable carrier" includes vehicles such as common excipients e.g. binding agents, for example syrup, acacia, gelatin, sorbitol, tragacanth,

polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate, stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

For topical application to the skin, compounds of general formula (I) may be made up into a cream, ointment, jelly, solution or suspension etc. Cream or ointment formulations that may be used for the drug are conventional formulations well known in the art, for example, as described in standard text books of pharmaceutics such as the British Pharmacopoeia.

Topical administration to the lung may be achieved by use of an aerosol formulation. Aerosol formulations typically comprise the active ingredient suspended or dissolved in a suitable aerosol propellant, such as a chlorofluorocarbon (CFC) or a hydrofluorocarbon (HFC). Suitable CFC propellants include trichloromonofluoromethane (propellant 11), dichlorotetrafluoromethane (propellant 114), and dichlorodifluoromethane (propellant 12). Suitable HFC propellants include tetrafluoroethane (HFC-134a) and heptafluoropropane (HFC-227). The propellant typically comprises 40%-99.5% e.g. 40%-90% by weight of the total inhalation composition. The formulation may comprise excipients including co-solvents (e.g. ethanol) and surfactants (e.g. lecithin, sorbitan trioleate and the like). Other possible excipients include polyethylene glycol, polyvinylpyrrolidone, glycerine and the like. Aerosol formulations are packaged in canisters and a suitable dose is delivered by means of a metering valve (e.g. as supplied by Bespak, Valois or 3M or alternatively by Aptar, Coster or Vari).

Topical administration to the lung may also be achieved by use of a non-pressurised formulation such as an aqueous solution or suspension. These may be administered by means of a nebuliser e.g. one that can be hand-held and portable or for home or hospital use (ie non-portable). The formulation may comprise excipients such as water, buffers, tonicity adjusting agents, pH adjusting agents, surfactants and co-solvents. Suspension liquid and aerosol formulations (whether pressurised or unpressurised) will typically contain the compound of the invention in finely divided form, for example with a $D_{50}$ of 0.5-10 $\mu$m e.g. around 1-5 $\mu$m. Particle size distributions may be represented using $D_{10}$, $D_{50}$ and $D_{90}$ values. The $D_{50}$ median value of particle size distributions is defined as the particle size in microns that divides the distribution in half. The measurement derived from laser diffraction is more accurately described as a volume distribution, and consequently the $D_{50}$ value obtained using this procedure is more meaningfully referred to as a $Dv_{50}$ value (median for a volume distribution). As used herein Dv values refer to particle size distributions measured using laser diffraction. Similarly, $D_{10}$ and $D_{90}$ values, used in the context of laser diffraction, are taken to mean $Dv_{10}$ and $Dv_{90}$ values and refer to the particle size whereby 10% of the distribution lies below the $D_{10}$ value, and 90% of the distribution lies below the $D_{90}$ value, respectively.

Topical administration to the lung may also be achieved by use of a dry-powder formulation. A dry powder formulation will contain the compound of the disclosure in finely divided form, typically with a mass mean diameter (MMAD) of 1-10 $\mu$m or a $D_{50}$ of 0.5-10 $\mu$m e.g. around 1-5 $\mu$m. Powders of the compound of the invention in finely divided form may be prepared by a micronization process or similar size reduction process. Micronization may be performed using a jet mill such as those manufactured by Hosokawa Alpine. The resultant particle size distribution may be measured using laser diffraction (e.g. with a Malvern Mastersizer 2000S instrument). The formulation will typically contain a topically acceptable diluent such as lactose, glucose or mannitol (preferably lactose), usually of comparatively large particle size e.g. a mass mean diameter (MMAD) of 50 $\mu$m or more, e.g. 100 $\mu$m or more or a $D_{50}$ of 40-150 $\mu$m. As used herein, the term "lactose" refers to a lactose-containing component, including $\alpha$-lactose monohydrate, $\beta$-lactose monohydrate, $\alpha$-lactose anhydrous, $\beta$-lactose anhydrous and amorphous lactose. Lactose components may be processed by micronization, sieving, milling, compression, agglomeration or spray drying. Commercially available forms of lactose in various forms are also encompassed, for example Lactohale® (inhalation grade lactose; DFE Pharma), InhaLac®70 (sieved lactose for dry powder inhaler; Meggle), Pharmatose® (DFE Pharma) and Respitose® (sieved inhalation grade lactose; DFE Pharma) products. In one embodiment, the lactose component is selected from the group consisting of $\alpha$-lactose monohydrate, $\alpha$-lactose anhydrous and amorphous lactose. Preferably, the lactose is $\alpha$-lactose monohydrate.

Dry powder formulations may also contain other excipients. Thus in one embodiment a dry powder formulation according the present disclosure comprises magnesium or calcium stearate. Such formulations may have superior chemical and/or physical stability especially when such formulations also contain lactose.

A dry powder formulation is typically delivered using a dry powder inhaler (DPI) device. Example dry powder delivery systems include SPINHALER®, DISKHALER®, TURBOHALER®, DISKUS®, SKYEHALER®, ACCUHALER® and CLICKHALER®. Further examples of dry powder delivery systems include ECLIPSE, NEXT, ROTAHALER, HANDIHALER, AEROLISER, CYCLOHALER, BREEZHALER/NEOHALER, MONODOSE, FLOWCAPS, TWINCAPS, X-CAPS, TURBOSPIN, ELPENHALER, MIATHALER, TWISTHALER, NOVOLIZER, PRESSAIR, ELLIPTA, ORIEL dry powder inhaler, MICRODOSE, PULVINAL, EASYHALER, ULTRAHALER, TAIFUN, PULMOJET, OMNIHALER, GYROHALER, TAPER, CONIX, XCELOVAIR and PROHALER.

In one embodiment a compound of general formula (I) is provided as a micronized dry powder formulation, for example comprising lactose of a suitable grade.

Thus, as an aspect of the invention there is provided a pharmaceutical composition comprising a compound of general formula (I) in particulate form in combination with particulate lactose, said composition optionally comprising magnesium stearate.

In one embodiment a compound of general formula (I) is provided as a micronized dry powder formulation, comprising lactose of a suitable grade and magnesium stearate, filled into a device such as DISKUS. Suitably, such a device is a multidose device, for example the formulation is filled into blisters for use in a multi-unit dose device such as DISKUS.

In another embodiment a compound of general formula (I) is provided as a micronized dry powder formulation, for example comprising lactose of a suitable grade, filled into hard shell capsules for use in a single dose device such as AEROLISER.

In another embodiment a compound of general formula (I) is provided as a micronized dry powder formulation, comprising lactose of a suitable grade and magnesium stearate, filled into hard shell capsules for use in a single dose device such as AEROLISER.

In another embodiment a compound of general formula (I) is provided as a fine powder for use in an inhalation dosage form wherein the powder is in fine particles with a $D_{50}$ of 0.5-10 μm e.g. around 1-5 μm, that have been produced by a size reduction process other than jet mill micronisation e.g. spray drying, spray freezing, microfluidisation, high pressure homogenisation, super critical fluid crystallisation, ultrasonic crystallisation or combinations of these methods thereof, or other suitable particle formation methods known in the art that are used to produce fine particles with an aerodynamic particle size of 0.5-10 μm. The resultant particle size distribution may be measured using laser diffraction (e.g. with a Malvern Mastersizer 2000S instrument). The particles may either comprise the compound alone or in combination with suitable other excipients that may aid the processing. The resultant fine particles may form the final formulation for delivery to humans or may optionally be further formulated with other suitable excipients to facilitate delivery in an acceptable dosage form.

The compound of the invention may also be administered rectally, for example in the form of suppositories or enemas, which include aqueous or oily solutions as well as suspensions and emulsions and foams. Such compositions are prepared following standard procedures, well known by those skilled in the art. For example, suppositories can be prepared by mixing the active ingredient with a conventional suppository base such as cocoa butter or other glycerides. In this case, the drug is mixed with a suitable nonirritating excipient which is solid at ordinary temperatures but liquid at the rectal temperature and will therefore melt in the rectum to release the drug. Such materials are cocoa butter and polyethylene glycols.

Parenteral formulations will generally be sterile.

The medical practitioner, or other skilled person, will be able to determine a suitable dosage for the compound of general formula (I), and hence the amount of the compound of the invention that should be included in any particular pharmaceutical formulation (whether in unit dosage form or otherwise).

Compounds of general formula (I) may be used in combination with one or more other active agents which are useful in the treatment or prophylaxis of neurodegenerative disorders.

Therefore, in a further aspect of the invention, there is provided a product comprising a compound of general formula (I) and an additional agent useful in the treatment or prevention of a neurodegenerative disorder as a combined preparation for simultaneous, sequential or separate use in the treatment or prevention of a neurodegenerative disorder as described above.

The invention will now be described in greater detail with reference to the following examples and to the figures in in which:

**Figure 1** (Figures 1A, 1 B and 1C) shows data from fibroblast cell lines from controls and from 6 sporadic Parkinson's disease (sPD) patients. Controls are compared with untreated sPD, fibroblasts, sPD fibroblasts incubated with UDCA and sPD fibroblasts incubated with Compound 7; Figure 1A: basal oxygen consumption; Figure 1B: maximal

respiratory rate; Figure 1C: ATP linked respiration.

**Figure 2** shows the extracellular acidification rate in fibroblast cell lines from controls and from 6 sporadic Parkinson's disease (sPD) patients. Control fibroblasts are compared with untreated sPD, fibroblasts, sPD fibroblasts incubated with UDCA and sPD fibroblasts incubated with Compound 7.

**Figure 3** shows mitochondrial respiratory chain complex I activity in homogenate from a MTPT mouse which was either untreated or treated with Compound 7 at a dose of 4 mg/kg or 12 mg/kg

**Figure 4** shows a comparison of mitochondrial respiratory chain complex I activity in left striatum mouse brain homogenate from a mouse which is treated with vehicle, with vehicle + 12mg Compound 7, with MPTP or with MTPT plus 1mg, 4mg or 12mg Compound 7.

**Abbreviations**

| | |
|---|---|
| AcOH | Acetic acid |
| Boc | *t*-butyloxycarbonyl |
| $^t$BuOH | *t*-butanol |
| BzOH | Benzoic acid |
| Calcd | Calculated |
| *m*CPBA | *Meta*-chloroperbenzoic acid |
| d | Days |
| DAST | Diethylaminosulfur trifluoride |
| DCM | Dichloromethane |
| DEAD | Diethyl azodicarboxylate |
| DIPA | di-isopropyl alcohol |
| DIPEA | *N,N*-diisopropylethylamine |
| DMF | *N,N*-dimethylformamide |
| EDC | 1-Ethyl-3-(3-dimethylaminopropyl)carbodiimide |
| Equiv | Equivalents |
| EtOAc | Ethyl acetate |
| EtOH | Ethanol |
| $Et_3N$ | Triethylamine |
| h | Hours |
| HATU | (1-[Bis(dimethylamino)methylene]-1H-1,2,3-triazolo[4,5-b]pyridinium 3-oxid hexafluorophosphate |
| HOBt | Hydroxybenzotriazole |
| HPLC | High performance liquid chromatography |
| MeOH | Methanol |
| Mol | Moles |
| MOM | Methoxymethyl |
| NaOMe | Sodium methoxide |
| PE | Petroleum ether |
| PPh3 | triphenylphosphine |
| RM | Reaction mixture |
| RT | Room temperature |

(continued)

| | |
|---|---|
| sat | Saturated |
| SM | Starting material |
| $Tf_2O$ | Trifluromethanesulfonic anhydride (triflic anhydride) |
| THF | Tetrahydrofuran |
| TLC | Thin layer chromatograpy |
| UDCA | Ursodeoxycholic acid |

**General Procedures**

**General procedure A for 24-carboxylic acid protection as methyl ester**

The method of Pelliciari was used (ACS Med. Chem. Lett. 2012, 3, 273-277). Free bile acid (25.0 g, 64 mmol, 1 equiv) was dissolved in HPLC grade MeOH (20 volumes) before adding *p*-toluene sulfonic acid (0.1 equiv) and sonicating at 30 °C for 2 h. Once deemed complete by TLC analysis the solvent was removed *in vacuo,* before dissolving the residue in EtOAc (16 volumes), washing the organics with sat. $NaHCO_3$ (x2), water and brine. The organic phase was then dried ($Na_2SO_4$) and concentrated to yield the methyl ester.

**General procedure B for 3-keto/7-keto reduction using $NaBH_4$/$CeCl_3$**

Using the conditions of Černý (Steroids 2012, 77, 1233-1241). To a solution of the ketone (1 equiv) and $CeCl_3$ (1.2 equiv) in MeOH (~50 volumes) and EtOAc (2 mL) was added $NaBH_4$ (1.1 equiv) over the course of 5 min. The solution was stirred for 30 min, at which point further $NaBH_4$ (1 equiv) was added and stirred for a further 30 min to drive the reaction towards completion. Reaction quenched with ice cold 2M HCl and the aqueous washed with EtOAc (x2). The combined organic were washed with sat $NaHCO_3$ and water, dried ($Na_2SO_4$) and concentrated then purified by column chromatography to afford both the $\alpha$-OH and $\beta$-OH epimers.

**General procedure C for saponification of methyl ester using LiOH in MeOH**

To a solution of the methyl ester (43 mg, 0.11 mmol, 1 equiv) in MeOH (~70 volumes) was added 2M LiOH (10 equiv) and the solution allowed to stir until complete at RT. Solvent removed *in vacuo* and the crude residue acidified with 2M HCl, before extracting with EtOAc (x2). Combined organics washed with water and brine, dried ($Na_2SO_4$) and concentrated to yield the free acid.

**General procedure D for saponification of methyl ester using LiOH in THF**

The methyl ester (1.0 equiv.) was dissolved in THF (~ 20 volumes) and added LiOH (2M in $H_2O$,10 equiv.). After stirring at room temperature until complete, the reaction mixture was reduced *in vacuo,* the resulting residue was acidified with 2M HCl and the aqueous phase was extracted with EtOAc (x2). The combined organic phases were then washed with water and brine before drying over $Na_2SO_4$ and reducing *in vacuo* to afford the free acid.

**General procedure E for methanolysis of acetate/benzoate**

The acetate/benzoate protected bile acid (3.1 g, 6.12 mmol, 1 equiv) was dissolved in dry MeOH (~10 volumes) before the addition of 25% NaOMe in MeOH (~6 volumes) and the RM stirred at RT. On completion the reaction was acidified to pH 4-5 with 2M HCl and diluted with $H_2O$. The aqueous phase was extracted with DCM (x2), combined organics washed with $NaHCO_3$, dried ($Na_2SO_4$) and concentrated to afford the deprotected material. Used without further purification.

**General procedure F for saponification of ester using NaOH**

To a round-bottom flask were added the ester (1.0 eq), NaOH (12.95 eq) and MeOH (HPLC grade, 8.35 mL/mol). The reaction mixture was stirred at room temperature overnight. Upon completion indicated by TLC analysis, the solvent was removed under reduced pressure. The residue was diluted with water, acidified with aqueous HCl and extracted with EtOAc (× 3). The combined organic layer was washed with brine, dried over Na2SO4, filtered and concentrated *in vacuo* to

afford the desired compound (purification by flash chromatography if needed).

**General procedure G for reduction of ketone derivatives using $NaBH_4$**

To a round bottom flask were added the ketone (1.0 eq), sodium borohydride (2.4 eq) and anhydrous THF (~40 vol). The reaction mixture was stirred at room temperature overnight, quenched with $H_2O$ and extracted with ethyl acetate (× 3). The combined organic layer was washed with brine, dried over $Na_2SO_4$, filtered and concentrated *in vacuo.* The crude was purified by flash chromatography, and by HPLC if needed.

**General procedure K for protection of secondary alcohol as a MOM ether**

To a solution of starting secondary alcohol (1 equiv) in dry DCM (~17 volumes) was added DIPEA (3 equiv) and MOM-Cl (5 equiv) at 0 °C. The reaction mixture was warmed to room temperature before allowing to stir overnight. Once complete, the reaction mixture was quenched with water (~3.4 mL/mmol) and methanol (~3.4 mL/mmol) before separating the layers and extracting the aqueous with EtOAc (x4) and washing the combined organics with brine (x2). The organic phase was then dried ($Na_2SO_4$) and concentrated *in vacuo* to yield the crude material. The crude was purified by flash chromatography (HPLC if needed) to afford the desired compound.

**General procedure L for cleavage of MOM-group using HCl**

The MOM protected material (1.5 g, 1.76 mmol, 1 equiv) was dissolved in MeOH (~30 volumes) and 2M HCl (~5.7 mL/mmol), then the mixture was warmed to 70 °C for 5 hr. Reaction mixture was cooled, and concentrated *in vacuo,* azeotroping to complete dryness (MeOH×3, $CHCl_3$×1) to yield the desired material.

**General procedure M for secondary alcohol oxidation using Dess-Martin Periodinane**

To a solution of starting secondary alcohol (1.0 equiv.) in dichloromethane at 0 °C was added Dess-Martin periodinane (~1.2 equiv.) portion-wise over 10 mins. After 18 hours warming to RT, the reaction was deemed complete by TLC and the reaction mixture was quenched by the addition of sat. $Na_2S_2O_3$ solution and sat. $NaHCO_3$ solution. The aqueous phase was separated and extracted with dichloromethane (x3) and the combined organic fractions were washed with sat. $NaHCO_3$ solution, water and brine, dried over $MgSO_4$, filtered and concentrated *in vacuo* to afford the crude desired product. The crude was purified by flash chromatography (HPLC if needed) to afford the desired compound.

**General procedure N for hydrogenation/hydrogenolysis using catalyst**

To a round-bottom flask was added benzyl protected bile acid (1.0 eq), catalyst [Pd/C or $PtO_2$] (10 mol%) and solvent [MeOH, EtOH, etc]. The reaction mixture was degassed with hydrogen gas and then stirred under $H_2$ at atmospheric pressure or high pressure for 16-72 hours. The catalyst was filtered through Celite and the filtrate was concentrated and purified by flash chromatography.

**General procedure O for preparation of silyl enol ether from ketone derivatives**

To a round-bottom flask were added DIPA (12.6 eq) and THF (1.25 mL/mmol). The solution was cooled to -78 °C, then n-butyllithium (2.5 M in hexanes, 12 eq) was added dropwise and stirred at -78 °C for 30 min. TMSCI (10 eq) was added and stirred for 20 min. A solution of the ketone derivative (1 eq) in THF (6.5 mL/mmol) was then added dropwise in 10 min and stirred at this temperature for 45 min, followed by the addition of triethylamine (18 eq) and stirred for 1 h. The reaction mixture was warmed to -20 °C, quenched with saturated $NaHCO_3$ solution and warmed to room temperature in 2 h. The organic layer was separated and the aqueous layer was extracted with ethyl acetate (x3). The combined organic layer was washed with saturated $NaHCO_3$ solution, water, brine, dried over $Na_2SO_4$, filtered and concentrated to afford the desired sily enol ether intermediate which was used for further reaction without any purification.

**General procedure P for eletrophilic fluorination of silyl enol ether using Selectfluor in DMF**

To a round bottom flask were the silyl enol ether derivative (1.0 eq), DMF (2 mL/mmol) and a solution of Selectfluor (1.5 eq) in DMF (3 mL/mmol) at 0 °C. The reaction mixture was stirred at room temperature overnight, quenched with $H_2O$ and extracted with ethyl acetate (× 4). The combined organic layer was washed with brine, dried over $Na_2SO_4$, filtered and concentrated *in vacuo.* The crude was purified by flash chromatography, and by HPLC if needed.

**General procedure Q for the formation of Conjugates**

Fluorinated bile acid (1 equiv.) was dissolved in dry DMF (12 vol) with stirring under argon. HATU (1 equiv.), DIPEA (3.0 equiv.) and amino acid (1.1 equiv.) added and the reaction stirred at RT for 16 h. Upon completion, the reaction mixture was dry loaded directly onto silica and purified via C18 chromatography (gradient elution of MeOH in $H_2O$, 0-100%) to yield the conjugate as either a DIPEA salt or free acid. DIPEA salts were then treated with a sodium ion exchange column to yield the desired sodium salt of the compounds as residues.

**Example 1 - Synthesis of 2β-Fluoro Compounds**

**A. Methyl 3α,7α-dihydroxy-5β-cholanoate (1A.1)**

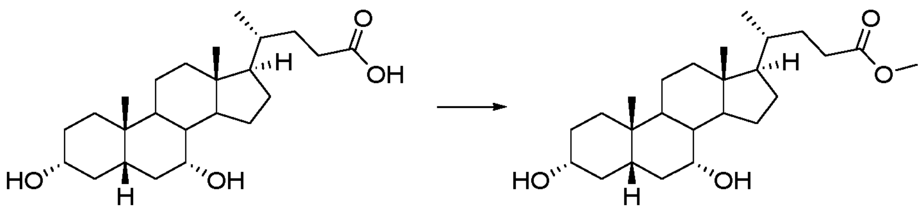

1A.1

The method of Pellicari was used (ACS Med. Chem. Lett. 2012, 3, 273-277). CDCA (25.0 g, 64 mmol, 1 equiv) was dissolved in HPLC grade MeOH (500 mL) before adding p-toluene sulfonic acid (1.21 g, 6.4 mmol, 0.1 equiv) and sonicating at 30 °C for 2 h. Once deemed complete by TLC analysis the solvent was removed in vacuo, before dissolving the residue in EtOAc (400 mL), washing the organics with sat. $NaHCO_3$ (2 × 150 mL), water (250 mL) and brine (250 mL). The organic phase was then dried ($Na_2SO_4$) and concentrated to yield target compound as a white/pale yellow solid (26.0 g, quantitative). (General procedure A).

**$^1$H NMR** (400 MHz, $CDCl_3$): δ 3.84 (1H, q, J = 2.4 Hz), 3.66 (3H, s), 3.44 (1H, tt, J = 10.9, 4.5 Hz), 2.34 (1H, ddd, J = 15.5, 11.0, 5.0 Hz), 2.28-2.15 (2H, m), 2.12-0.97 (26H, m), 0.93 (3H, d, J = 6.2 Hz), 0.90 (3H, s), 0.65 (3H, s) ppm.

**LRMS** ($ESI^+$) m/z : 429.1 $[M+Na]^+$.

B. **Methyl 7α-hydroxy-3-oxo-5β-cholanoate (1B.1)**

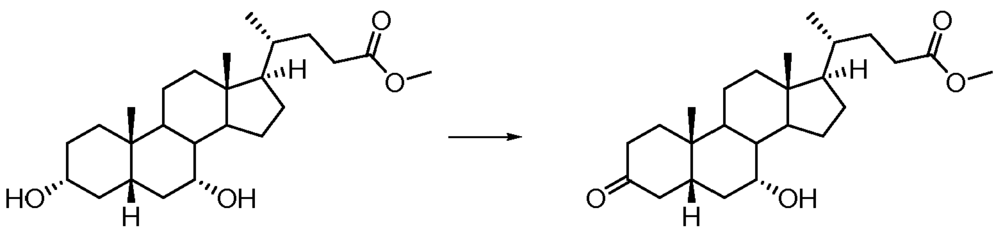

1B.1

To a solution of methyl 3α,7α-dihydroxy-5β-cholanoate (10.0 g, 24.6 mmol, 1 equiv) in water (25 mL) and t-butanol (100 mL) was added KBr (5.9 g, 49.0 mmol, 2 equiv), $KHCO_3$ (24.6 g, 246 mmol, 10 equiv) and TEMPO (5.0 g, 32.0 mmol, 1.3 equiv). The solution was cooled to 0 °C before adding ≈11% NaClO solution (54.2 mL, 73.2 mmol, 3.0 equiv) portion wise over the course of 6 h. The reaction was quenched with slow addition of sodium thiosulfate solution (300 mL, 1.2 M, 350 mmol). The aqueous was extracted with EtOAc (2×300 mL), which were combined and washed with brine (300 mL) and water (300 mL) before drying ($Na_2SO_4$) and removing the solvent in vacuo. The resulting bright red thick oily crude (15 g) was purified using flash chromatography (PE/EtOAc : 80:20→65:35) to yield a white solid (6.5 g, 16.0 mmol, 66%).

**$^1$H NMR** (400 MHz, $CDCl_3$): δ 3.93 (1H, br s), 3.67 (3H, s), 3.40 (1H, t, J = 14.4 Hz), 2.46-1.10 (30H, m), 1.01 (3H, s), 0.95 (3H, d, J = 6.6 Hz), 0.71 (3H, s) ppm.

**LRMS** ($ESI^+$) m/z : 422.1 $[M+NH_4]^+$, 427.1 $[M+Na]^+$.

C. **Methyl 7α-methoxymethoxyl-3-oxo-5β-cholanoate (1C.1)**

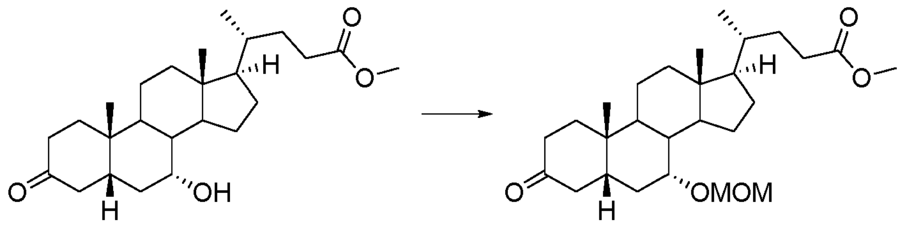

1B.1 1C.1

To a solution of methyl 7α-hydroxy-3-oxo-5β-cholanoate (3.0 g, 7.41 mmol, 1 equiv) in dry DCM (50 mL) was added DIPEA (3.83 mL, 22.2 mmol, 3 equiv) and MOM-Cl (2.82 mL, 37.1 mmol, 5 equiv) at 0 °C. The reaction mixture was warmed to room temperature before allowing to stir overnight. Once complete, the reaction mixture was quenched with water (25 mL) and methanol (25 mL) before separating the layers and extracting the aqueous with EtOAc (4×75 mL) and washing the combined organics with brine (2×150 mL). The organic phase was then dried ($Na_2SO_4$) and concentrated in vacuo to yield 3.8 g of crude material which was purified by flash chromatography (PE/EtOAc : 75:25) yielding a white solid (3.10 g, 6.9 mmol, 93%).

**$^1$H NMR** (400 MHz, $CDCl_3$): δ 4.68 (1H, d, J = 6.8 Hz), 4.55 (1H, d, J = 6.8 Hz), 3.72-3.62 (4H, m), 3.43-3.28 (4H, m), 2.49-1.05 (27H, m), 1.03 (3H, s), 0.94 (3H, d, J = 6.4 Hz), 0.69 (3H, s) ppm.

**LRMS** ($ESI^+$) m/z : 449.3 $[M+H]^+$, 471.1 $[M+Na]^+$.

D. **Methyl 7α-methoxymethoxyl-3-trimethylsilyloxy-5β-chol-2-eneoate (1D.1) and methyl 7α-methoxymethoxyl-3-trimethylsilyloxy-5β-chol-3-eneoate (ID.2)**

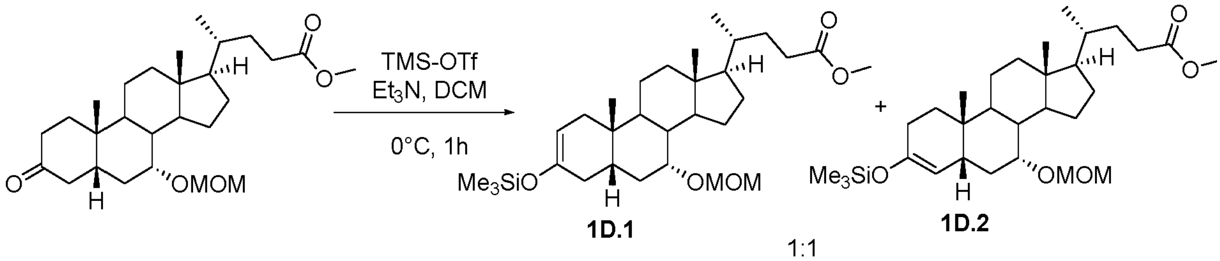


**1D.1** **1D.2**

1:1

Following method of Barlow et al (Eur. J. Med. Chem. 2011, 46, 1545-1554). To a solution of methyl 7α-methoxymethoxyl-3-oxo-5β-cholanoate (1.0 g, 2.23 mmol, 1 equiv) in dry DCM (20 mL) at 0 °C was added $Et_3N$ (0.62 mL, 4.46 mmol, 2 equiv) and trimethylsilyl triflate (0.44 mL, 2.45 mmol, 1.1 equiv). The reaction mixture was allowed to stir for 1 hr before diluting with further DCM (150 mL) and quenching with sat. $NaHCO_3$ (100 mL). The layers were separated and the aqueous was extracted with further DCM (3×100 mL), which were combined and washed with brine (150 mL), dried ($Na_2SO_4$) and concentrated to yield a colourless oil (1.2 g) which contained methyl 7α-methoxymethoxyl-3-trimethylsilyloxy-5β-chol-2-eneoate (IA.1) and methyl 7α-methoxymethoxyl-3-trimethylsilyloxy-5β-chol-3-eneoate (1A.2) in a roughly 1:1 ratio. This crude material was used in subsequent steps without further purification.

**$^1$H NMR** (400MHz, $CDCl_3$): δ 4.80-4.44 (3H, m), 3.67 (3H, s), 3.66-3.56 (1H, m), 3.40 (1.5H, s), 3.36 (1.5H, s), 2.51-1.08 (31H, m), 0.99-0.95 (3H, m), 0.93 (3H, d, J = 6.2 Hz), 0.66 (1.5 H, br. s), 0.65 (1.5 H, br. s), 0.21-0.15 (9H, m) ppm.

**E. Methyl 2β-fluoro-7α-methoxymethoxyl-3-oxo-5β-cholanoate (1E.1) and methyl 4β-fluoro-7α-methoxymethoxyl-3-oxo-5β-cholanoate (1E.2)**

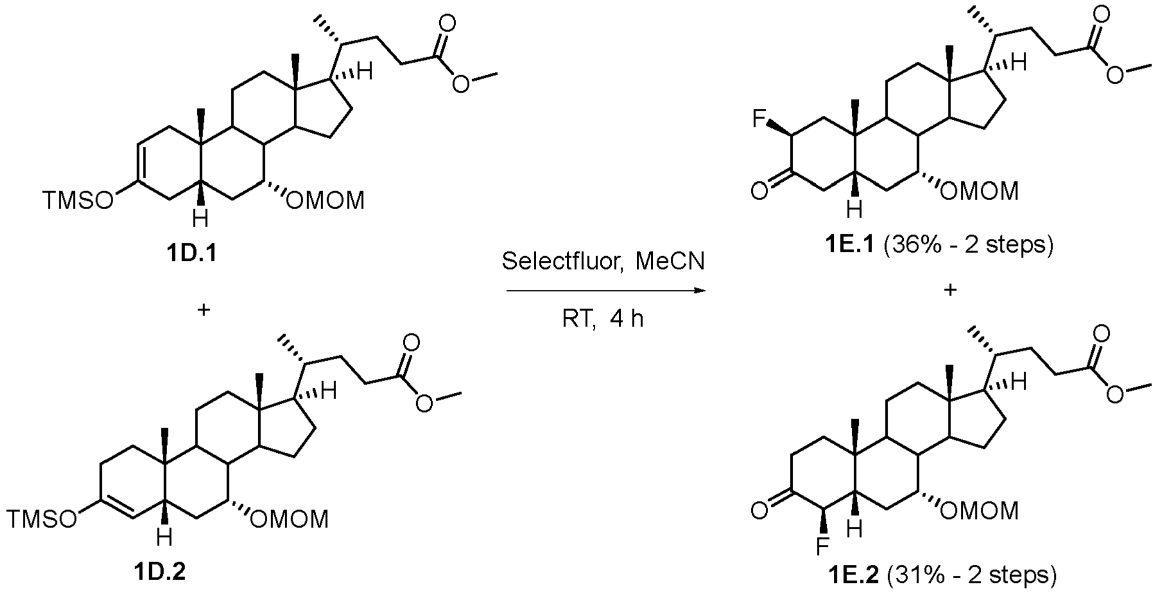


1D.1 + 1D.2 → 1E.1 (36% - 2 steps) + 1E.2 (31% - 2 steps)

Following the method of Fujimoto et al (Bioorg. Med. Chem. Lett. 2011, 21, 6409-6413). To a solution of 7α-methoxymethoxyl-3-trimethylsilyloxy-5β-chol-2-eneoate and methyl 7α-methoxymethoxyl-3-trimethylsilyloxy-5β-chol-3-eneoate (1.10 g, 2.2 mmol, 1 equiv) in dry acetonitrile was added Selectfluor® (1.20 g, 3.3 mmol, 1.5 equiv), allowing the reaction mixture to stir at room temperature for 4 h. The solvent was removed *in vacuo* before diluting with EtOAc (100 mL) and water (100 mL). The layers were separated before extracting the aqueous with further EtOAc (2×100 mL). The combined organics were then washed with brine (150 mL), dried ($Na_2SO_4$) and concentrated to yield 1.05 g of a pale yellow solid crude. The crude material was purified using flash chromatography (PE/EtOAc : 80:20) to yield methyl 2β-fluoro-7α-methoxymethoxyl-3-oxo-5β-cholanoate as a white solid (377 mg, 0.81 mmol, 36% over two steps) and methyl 4β-fluoro-7α-methoxymethoxyl-3-oxo-5β-cholanoate as a white solid (321 mg, 0.69 mmol, 31% over two steps).

**1E.1: $^1$H NMR** (400MHz, $CDCl_3$): δ 5.05 (1H, ddd, J = 49.4, 13.5, 5.6 Hz), 4.67 (1H, d, J = 6.8 Hz), 4.54 (1H, d, J = 6.8 Hz), 3.67 (4H, s), 3.48 (1H, t, J = 13.9 Hz), 3.37 (3H, s), 2.52 (1H, dt, J = 12.7, 6.1 Hz), 2.42-1.12 (27H, m), 1.08 (3H, s), 0.95 (3H, d, J = 6.6 Hz), 0.69 (3H, s) ppm.

**$^{19}$F NMR** ($CDCl_3$, 376MHz): δ -195.19 (ddt, J = 49.2, 9.6, 6.2 Hz) ppm.

**LRMS** ($ESI^+$) m/z : 484.2 $[M+NH_4]^+$, 489.1 $[M+Na]^+$.

**1E.2: $^1$H NMR** (400MHz, $CDCl_3$): δ 5.78 (1H, dd, J = 46.8, 11.7 Hz), 4.77 (1H, d, J = 6.8 Hz), 4.59 (1H, d, J = 6.8 Hz), 3.78 (1H, q, J = 2.7 Hz), 3.67 (3H, s), 3.40 (3H, s), 2.52 (1H, td, J = 14.4, 4.9 Hz), 2.42 1.11 (24H, m), 1.07 (3H, s), 0.94 (3H, d, J = 6.4 Hz), 0.70 (3H, s) ppm.

**$^{19}$F NMR** ($CDCl_3$, 376MHz): δ -200.67 (ddd, J = 46.8, 12.1, 6.9 Hz) ppm.

**LRMS** ($ESI^+$) : m/z 484.2 $(M+NH_4)^+$, 489.1 $(M+Na)^+$.

**F. Methyl 2β-fluoro-3α-hydroxy-7α-methoxymethoxyl-5β-cholanoate (1F.1) and methyl 2β-fluoro-3β-hydroxy-7α-methoxymethoxyl-5β-cholanoate (1F.2)**

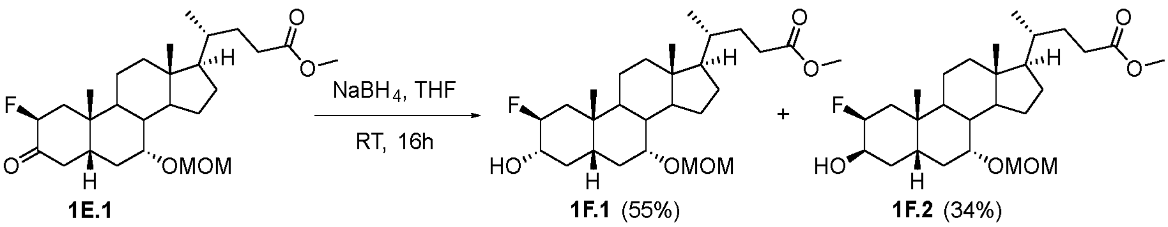


1E.1 → 1F.1 (55%) + 1F.2 (34%)

To a solution of methyl 2β-fluoro-7α-methoxymethoxyl-3-oxo-5β-cholanoate (260 mg, 0.56 mmol, 1 equiv) in anhydrous tetrahydrofuran (20 mL) was added sodium borohydride (64 mg, 1.70 mmol, 3 equiv) and the reaction mixture allowed to stir overnight at room temperature. Once deemed compete by TLC analysis the reaction was diluted with EtOAc (150 mL) and quenched with water (100 mL), separating the layers before extracting the aqueous with further EtOAc (2×100 mL). The combined organics were then washed with water (150 mL) and brine (150 mL) before drying ($Na_2SO_4$)

and concentrating *in vacuo* to yield 306 mg of a pale crude oil. The crude was purified by flash chromatography (PE/EtOAc: 65:35) to yield methyl 2β-fluoro-3α-hydroxy-7α-methoxymethoxyl-5β-cholanoate as a white solid (144 mg, 0.307 mmol, 55%) and methyl 2β-fluoro-3β-hydroxy-7α-methoxymethoxyl-5β-cholanoate as a colourless gum (88 mg, 0.19 mmol, 34%).

**1F.1: $^1$H NMR** (400MHz, $CDCl_3$): δ 4.68 (1H, d, J = 6.8 Hz), 4.54 (1H, d, J = 7.1 Hz), 4.40 (1H, dddd, J = 52.3, 12.0, 8.6, 4.4 Hz), 3.66 (3H, s), 3.62 - 3.47 (3H, m), 3.37 (3H, s), 2.48-1.01 (27H, m), 0.99 (3H, s), 0.92 (3H, d, J = 6.4 Hz), 0.64 (3H, s) ppm.

**$^{19}$F NMR** ($CDCl_3$, 376MHz): δ -187.47 (ddq, J = 52.3, 12.7, 7.1 Hz) ppm.

**LRMS** ($ESI^+$) m/z : 486.1 $[M+NH_4]^+$, 491.2 $[M+Na]^+$.

**1F.2: $^1$H NMR** (400MHz, $CDCl_3$): δ 4.72-4.51 (3H, m), 4.20-4.09 (1H, m), 3.66 (3H, s), 3.59 (1H, d, J = 2.4 Hz), 3.38 (3H, s), 2.45 (1H, ddd, J = 15.2, 12.4, 2.2 Hz), 2.35 (1H, ddd, J = 15.0, 10.3, 5.1 Hz), 2.22 (1H, ddd, J = 15.6, 9.5, 6.5 Hz), 2.06-1.05 (25H, m), 1.02 (3H, s), 0.93 (3H, d, J = 6.6 Hz), 0.65 (3H, s) ppm.

$^{19}$F NMR ($CDCl_3$, 376MHz): δ -187.31 (dquin, J = 47.2, 7.5 Hz) ppm.

LRMS ($ESI^+$) m/z : 486.0 $[M+NH_4]^+$, 491.1 $[M+Na]^+$.

**G. 2β-fluorochenodeoxycholic acid (Compound 1)**

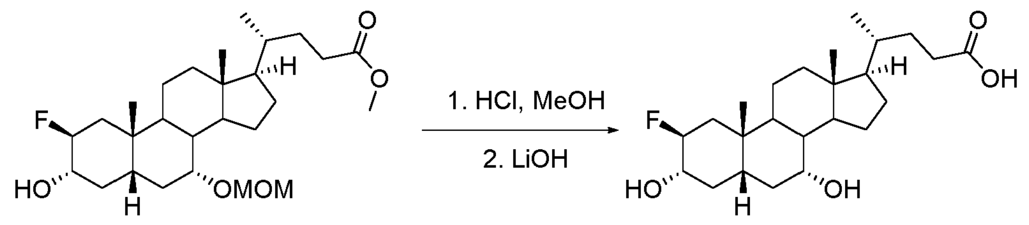


**1F.1** **Compound 1** (70%)

Using general procedure L, followed by general procedure C, methyl 2β-fluoro-3α-hydroxy-7α-methoxymethoxyl-5β-cholanoate (1F.1; 118 mg, 0.25 mmol, 1 equiv) was deprotected to yield 2β-fluorochenodeoxycholic acid **(Compound 1)** as a pale yellow solid (72 mg, 0.18 mmol, 70%).

**Compound 1: $^1$H NMR** (400MHz, $CD_3OD$): δ 4.32 (1H, dddd, J = 52.5, 12.5, 8.6, 4.0 Hz), 3.78 (1H, q, J = 2.3 Hz), 3.44 (1H, tdd, J = 12.0, 8.7, 5.0 Hz), 2.43 (1H, q, J = 13.2 Hz), 2.33 (1H, ddd, J = 15.5, 11.0, 5.0 Hz), 2.25-2.11 (2H, m), 2.04 (1H, dt, J = 12.4, 2.9 Hz), 1.99-1.04 (22H, m), 1.00 (3H, s), 0.97 (3H, d, J = 6.5 Hz), 0.70 (3H, s) ppm.

**$^{19}$F NMR** (376MHz, $CD_3OD$): δ -186.77 (ddq, J = 52.2, 11.9, 7.8 Hz) ppm.

**LRMS** ($ESI^+$) m/z : 393.1 $[M+H-H_2O]^+$, 821.2 $[2M+H]^+$, 843.3 $[2M+Na]^+$.

H. **2β-fluoro-3β,7α-dihydroxy-5β-cholanic acid (Compound 2)**

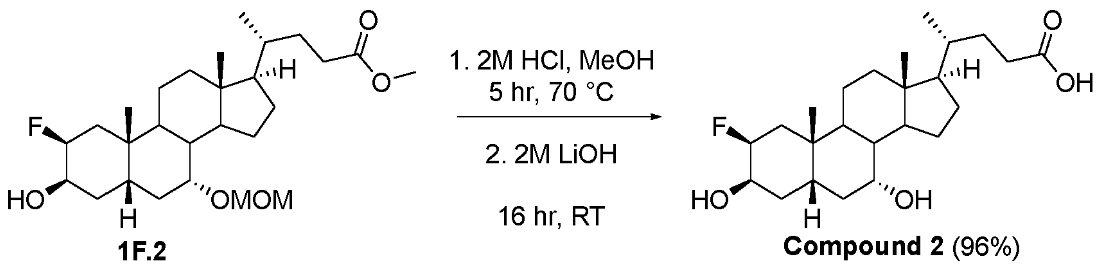


**1F.2** **Compound 2** (96%)

Using general procedure L, followed by general procedure C, methyl 2β-fluoro-3β-hydroxy-7α-methoxymethoxyl-5β-cholanoate (1F.2; 25 mg, 0.053 mmol, 1 equiv) was deprotected to yield 2β-fluoro-3β,7α-dihydroxy-5β-cholanic acid **(Compound 2)** as a gummy solid (21 mg, 0.05 mmol, 96%).

**Compound 2: $^1$H NMR** (400MHz, Acetone-$D_6$): δ 10.42 (1H, br. s), 4.58 (1H, dddd, J = 47.7, 12.1, 4.4, 2.8 Hz), 4.12-4.00 (1H, m), 3.80 (1H, q, J = 2.4 Hz), 3.59 (1H, br. s), 3.29 (1H, br. s), 2.61 (1H, ddd, J = 15.3, 12.8, 2.3 Hz), 2.34 (1H, ddd, J = 15.5, 11.0, 5.0 Hz), 2.21 (1H, ddd, J = 15.5, 9.6, 6.4 Hz), 2.03-1.03 (31H, m), 1.01 (3H, s), 0.97 (3H, d, J = 6.5 Hz), 0.70 (3H, s) ppm;

**$^{19}$F NMR** (376MHz, Acetone-$D_6$): δ -186.90 (dquin, J = 47.6, 7.6 Hz) ppm.
**LRMS** ($ESI^+$) m/z : 393.4 $[M+H-H_2O]^+$, 373.4 $[M+H-H_2O-HF]^+$, 355.5 $[M+H-2H_2O-HF]^+$.

**Example 2 - Synthesis of 2α-Fluoro Compounds**

A. **Methyl 7-oxo-5β-chol-2-eneoate (2A.1) and methyl 7-oxo-5β-chol-3-eneoate (2A.2)**

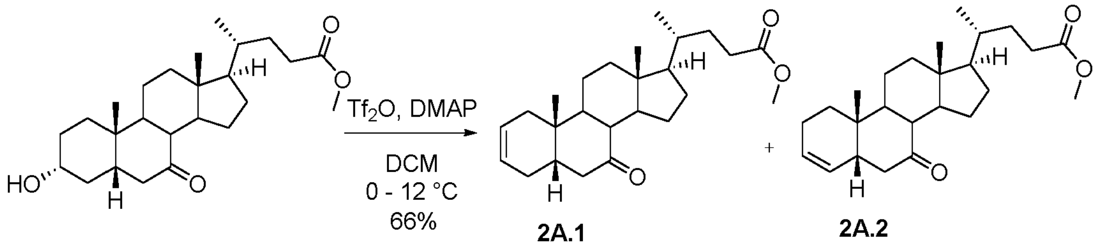


**2A.1** **2A.2**

Methyl 3α-hydroxyl-7-oxo-5β-cholanoate (60 g, 148 mmol, 1.0 equiv; synthesised from 7-ketolithocholic acid using procedure A) and DMAP (30 g, 122 mmol, 2.0 equiv) were dissolved in DCM (500 mL) and cooled to 0 °C on ice. Triflic anhydride (26.1 mL, 156 mmol, 1.05 equiv) was then added over the course of 15 mins. The reaction was stirred at 0 °C for 2 hours, although there was no reaction progress. Reaction was then slowly warmed to 10-12 °C and progress monitored *via* TLC. Deemed complete after 2 h, RM quenched with 2M HCl (500 mL) and stirred at RT for 10 mins. Layers separated and aqueous extracted with brine (500 mL), dried ($Na_2SO_4$) and concentrated to yield 78 g of a brown gummy solid. Crude purified *via* flash chromatography (Petrol ether/EtOAc : 95/5→90:10) to yield a mixture of alkenes methyl 7-oxo-5β-chol-2-eneoate and methyl 7-oxo-5β-chol-3-eneoate as a colourless gum (37.5 g, 97 mmol, 66%).
**2A.1 and 2A.2: $^1$H NMR** (400MHz, $CDCl_3$): δ 5.67-5.30 (2H, m), 3.65 (3H, s), 2.89-2.79 (1H, m), 2.58-2.48 (1H, m), 2.44-1.26 (21H, m), 1.23 (2H, s), 1.21 (1H, s), 0.91-0.88 (3H, m), 0.65 (3H, m) ppm.
**LRMS** ($ESI^+$) m/z : 387.2 $[M+H]^+$, 404.2 $[M+NH_4]^+$.

**B. Methyl 2β,3β-epoxy-7-oxo-5β-cholanoate (2B.1) and methyl 3β,4β-epoxy-7-oxo-5β-cholanoate (2B.2)**

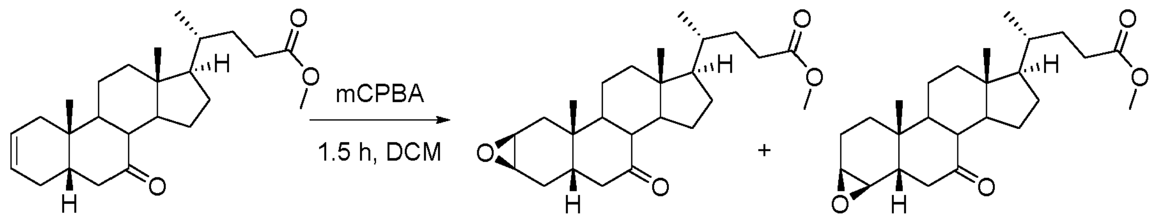


**2A.1** +Δ3,4-isomer **2A2** **2B.1** (~11%) **2B.2** (~22%)

The mixture of alkenes methyl 7-oxo-5β-chol-2-eneoate and methyl 7-oxo-5β-chol-3-eneoate (20 g, 51.8 mmol, 1 equiv) was dissolved in DCM (200 mL) at room temperature, before the addition of mCPBA (19.1 g, 77.7 mmol, 1.5 equiv). The reaction was deemed complete after 1.5 h, with the mixture changing from a solution to a suspension over the course of the reaction. The reaction was quenched with sat. aq. $Na_2S_2O_3$ (150 mL) and allowed to stir for 30 mins. Further DCM (200 mL) and $H_2O$ (150 mL) added to aid solvation. Layers separated and aqueous extracted with further DCM (200 mL), then the combined organics were washed with sat. aq. $NaHCO_3$ (200 mL) and dried ($Na_2SO_4$) and concentrated to yield 20.5 g of a pale yellow, gummy solid. Crude purified *via* flash chromatography (Petrol ether/EtOAc : 92.5:7.5→92:8→80:10→88:12→80:20) to yield the pure Δ3β,4β-epoxide (2.00 g) along with 80% pure Δ2β,3β-epoxide (1.85 g) and a significant amount of mixed fractions (8.5 g). The mixed fractions were re-purified (Petrol ether/EtOAc: 93:7→92:8→91:9→80:10→88:12→85:15→80:20) to yield the pure Δ3β,4β-epoxide (0.8 g) along with 80% pure Δ3β,4β-epoxide (2.15 g) and 60% pure Δ2,3-epoxide (1.30 g). Overall, methyl 2β,3β-epoxy-7-oxo-5β-cholanoate was isolated as a white crystalline solid (~2.3 g, 5.8 mmol, 11%), along with methyl 3β,4β-epoxy-7-oxo-5β-cholanoate as a white solid (~4.5 g, 11.3 mmol, 22%)
**2B.1: $^1$H NMR** (400MHz, $CDCl_3$): δ 3.63 (3H, s), 3.13-3.09 (1H, m), 2.98 (1H, dd, J = 5.3, 4.4 Hz), 2.78 (1H, dd, J = 12.3, 4.3 Hz), 2.39-2.11 (5H, m), 1.91 (6H, m), 1.61-1.17 (10H, m), 1.13 (3H, m), 1.09-0.92 (2H, m), 0.89 (3H, d, J = 6.5 Hz),

0.63 (3H, s) ppm.

**LRMS** ($ESI^+$) m/z : 403.1 $[M+H]^+$, 425.2 $[M+Na]^+$, 403.1 $[M+H-MeCN]^+$.

**2B.2: $^1$H NMR** (400MHz, $CDCl_3$): δ 3.64 (3H, s), 3.16-3.13 (1H, m), 2.89 (1H, dd, J = 12.6, 7.0 Hz,), 2.82 (1H, d, J = 3.8 Hz), 2.42-1.17 (24H, m), 1.13 (3H, s), 0.89 (3H, d, J = 6.5 Hz), 0.64 (3H, s) ppm.

**LRMS** ($ESI^+$) m/z : 403.2 $[M+H]^+$, 425.2 $[M+Na]^+$.

C. **Methyl 2α-fluoro-3β-hydroxy-7-oxo-5β-cholanoate (2C.1)**

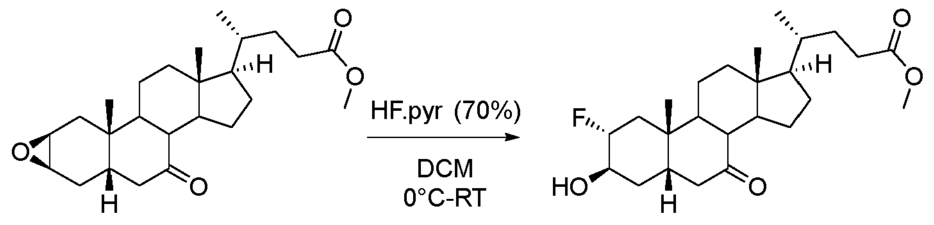


**2B.1** **2C.1** (80%)

To a solution of methyl 2β,3β-epoxy-7-oxo-5β-cholanoate (830 mg, 2.06 mmol, 1 equiv) in dry DCM (25 mL) was cooled to 0 °C, before adding 70% HF.pyridine (830 μL) and allowing to warm to RT. Deemed complete after 2 d, reaction cooled to 0 °C again and carefully quenched with drop-wise addition of saturated $NaHCO_3$ (20 mL). Layers separated and aqueous extracted with further DCM (20 mL); combined organics washed with 2M HCl and brine (30 mL each), dried ($Na_2SO_4$) and concentrated to 840 mg of a white foamy solid. Crude purified *via* flash chromatography (PE/EtOAc : 70:30) to yield methyl 2α-fluoro-3β-hydroxy-7-oxo-5β-cholanoate as a gummy solid (700 mg, 1.66 mmol, 80%).

**$^1$H NMR** (400MHz, $CDCl_3$): δ .53 (1H, dq, J = 47.0, 2.6 Hz), 4.04-3.96 (1H, m), 3.65 (3H, s), 2.87 (1H, dd, J = 12.7, 6.1 Hz), 2.42-1.25 (25H, m), 1.22 (3H, s), 1.20-1.00 (3H, m), 0.90 (3H, d, J = 6.4 Hz), 0.64 (3H, s) ppm.

**$^{19}$F NMR** ($CDCl_3$, 376MHz): δ -184.60 (tt, J = 48.6, 8.7 Hz) ppm;.

**LRMS** ($ESI^+$) m/z : 423.1 $[M+H]^+$, 445.1 $[M+Na]^+$, 845.5 $[2M+H]^+$.

D. **Methyl 2α-fluoro-3α-benzoyloxy-7-oxo-5β-cholanoate (2D.1)**

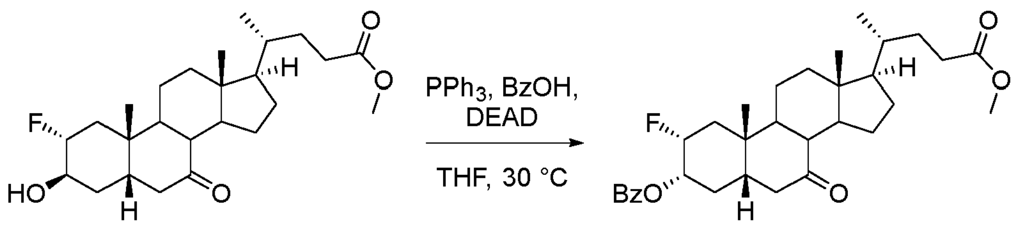


**2C.1** **2D.1**

To a solution of methyl 2α-fluoro-3β-hydroxy-7-oxo-5β-cholanoate (1.05 g, 2.5 mmol, 1 equiv), $PPh_3$ (980 mg, 3.7 mmol, 1.5 equiv) and benzoic acid (450 mg, 3.7 mmol, 1.5 equiv) in dry THF (25 mL) was added DEAD (650 μL, 3.7 mmol, 1.5 equiv). The solution was allowed to stir at 30°C over the weekend, at which point crude $^{19}$F NMR indicated roughly 40% conversion to desired benzoate. Further $PPh_3$, BzOH and DEAD (1.5 equiv each) was added and reaction allowed to stir O/N, at which point conversion was ≈60%. Further $PPh_3$, benzoic acid and DEAD (0.5 equiv each) added, stirred overnight and 80% conversion reached. More $PPh_3$, benzoic acid and DEAD (0.5 equiv each) added and stirred O/N once more, although no further progress noted. Solvent removed *in vacuo* and crude bright yellow material separated *via* flash chromatography (PE/EtOAc : 98:2→95:5→85:15→ 70:30→0:100) to yield 285 mg of methyl 2α-fluoro-3α-benzoyloxy-7-oxo-5β-cholanoate (≈90% pure) along with 1.28 g of additional mixed fractions.

**$^1$H NMR** (400MHz, $CDCl_3$): δ 8.04 (2H, dd, J = 7.8, 1.2 Hz), 7.56 (1H, tt, J = 7.6, 1.2 Hz), 7.44 (2H, t, J = 7.8 Hz), 5.12-4.79 (2H, m), 3.67 (3H, s), 2.92 (1H, dd, J = 12.6, 5.9 Hz), 2.53-1.29 (21H, m), 1.26 (3H, s), 1.24-1.04 (4H, m), 0.93 (3H, d, J = 6.4 Hz), 0.67 (3H, s) ppm.

**$^{19}$F NMR** ($CDCl_3$, 376MHz): δ -199.45 (tdd, J = 49.9, 28.6, 8.7 Hz) ppm.

**LRMS** ($ESI^+$) m/z : 527.2 $[M+H]^+$, 544.1 $[M+NH_4]^+$, 549.1 $[M+Na]^+$.

E. **Methyl 2α-fluoro-3α-hydroxy-7-oxo-5β-cholanoate (2E.1)**

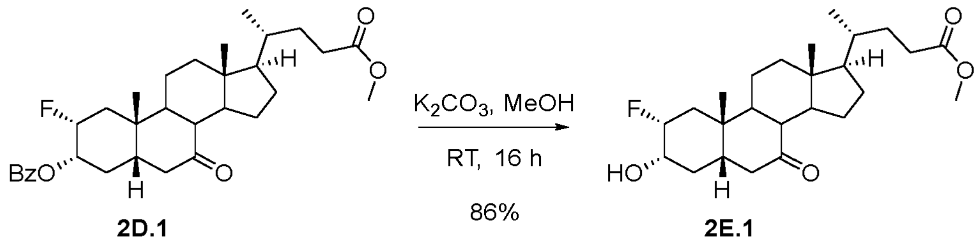


2D.1 2E.1

Using the method of Zhao et al (Eur. J. Org. Chem., 2005, 2005, 4414-4427). A mixture of methyl 2α-fluoro-3α-benzoyloxy-7-oxo-5β-cholanoate (400 mg, 0.76 mmol, 1 equiv) and potassium carbonate (20 mg, 0.15 mmol, 0.2 equiv) were suspended in dry MeOH (20 mL) and allowed to stir for 16 h at RT. After 16 h reaction mixture had formed a colourless solution, and was deemed complete by TLC analysis. Solvent removed *in vacuo* and crude residue taken up between EtOAc/$H_2O$ (5 mL each) and aqueous extracted with further EtOAc (2×5 mL). Combined organics dried ($Na_2SO_4$) and concentrated to yield 320 mg of a pale gum. Crude purified *via* flash chromatography (PE/acetone : 70:30) to yield methyl 2α-fluoro-3α-hydroxy-7-oxo-5β-cholanoate (275 mg, 0.65 mmol, 86%) as a gummy solid. **$^1$H NMR** (400MHz, $CDCl_3$): δ 4.71 (1H, d, J = 52.0 Hz), 3.62 (3H, s), 3.59-3.45 (1H, m), 2.84 (1H, dd, J = 12.5, 6.0 Hz), 2.43 (1H, d, J = 8.3 Hz), 2.39-2.26 (3H, m), 2.24-2.08 (2H, m), 2.01-1.20 (18H, m), 1.18 (3H, s), 1.14-1.02 (3H, m), 0.88 (3H, d, J = 6.5 Hz), 0.61 (3H, s) ppm.
**$^{19}$F NMR** ($CDCl_3$, 376MHz): δ -202.32 (tdd, J = 51.2, 29.5, 8.7 Hz) ppm.
**LRMS** (ESI$^+$) m/z : 423.4 [M+H]$^+$.

F. **Methyl 2α-fluoro-3β,7α-dihydroxy-5β-cholanoate (2F.1) and methyl 2α-fluoro-3β,7β-dihydroxy-5β-cholanoate (2F.2)**

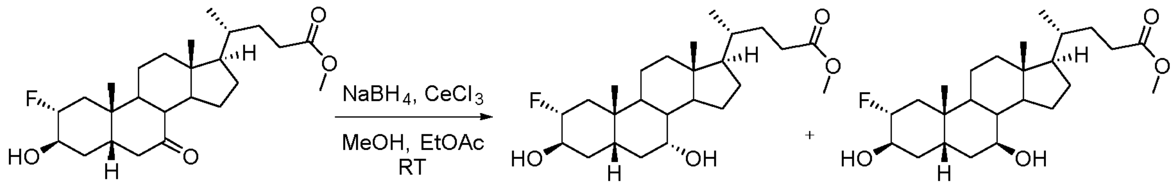


2C.1 **2F.1** (46%) **2F.2** (35%)

Using general procedure B, methyl 2α-fluoro-3β-hydroxy-7-oxo-5β-cholanoate (300 mg, 0.71 mmol, 1 equiv) was reduced. Crude material purified *via* flash chromatography (PE/EtOAc : 65:35→55:45) to yield methyl 2α-fluoro-3β,7α-dihydroxy-5β-cholanoate (140 mg, 0.33 mmol, 46%) and methyl 2α-fluoro-3β,7β-dihydroxy-5β-cholanoate (107 mg, 0.25 mmol, 35%).
**2F.1: $^1$H NMR** (400MHz, $CDCl_3$): δ 4.55 (1H, dq, J = 47.4, 2.8 Hz), 4.00 (1H, dq, J = 7.2, 3.4 Hz), 3.86 (1H, q, J = 2.6 Hz), 3.66 (3H, s), 2.72 (1H, tt, J = 14.3, 2.4 Hz), 2.35 (1H, ddd, J = 15.5, 11.0, 5.0 Hz), 2.22 (1H, ddd, J = 15.7, 9.4, 6.5 Hz), 2.11-1.06 (27H, m), 0.97 (3H, s), 0.92 (3H, d, J = 6.5 Hz), 0.66 (3H, s) ppm.
**$^{19}$F NMR** (376MHz, $CDCl_3$): δ -184.70 (tt, J = 48.6, 8.7 Hz) ppm;
**LRMS** (ESI$^+$) m/z : 447.3 [M+Na]$^+$.
**2F.2: $^1$H NMR** (400MHz, $CDCl_3$): δ 4.50 (1H, dq, J = 47.3, 2.8 Hz), 3.95 (1H, dq, J = 7.2, 3.4 Hz), 3.63 (3H, s), 3.55 (1H, td, J = 9.7, 5.1 Hz), 2.32 (1H, ddd, J = 15.4, 10.2, 5.0 Hz), 2.19 (1H, ddd, J = 15.6, 9.4, 6.5 Hz), 2.11-1.02 (27H, m), 0.96 (3H, s), 0.90 (3H, d, J = 6.4 Hz), 0.65 (3H, s) ppm.
**$^{19}$F NMR** (376MHz, $CDCl_3$): δ -184.43 (tt, J = 47.7, 8.7 Hz) ppm.
**LRMS** (ESI$^+$) m/z : 447.2 [M+Na]$^+$.

G. **Methyl 2α-fluoro-3α,7α-dihydroxy-5β-cholanoate (2G.1) and methyl 2α-fluoro-3α,7β-dihydroxy-5β-cholanoate (2G.2)**

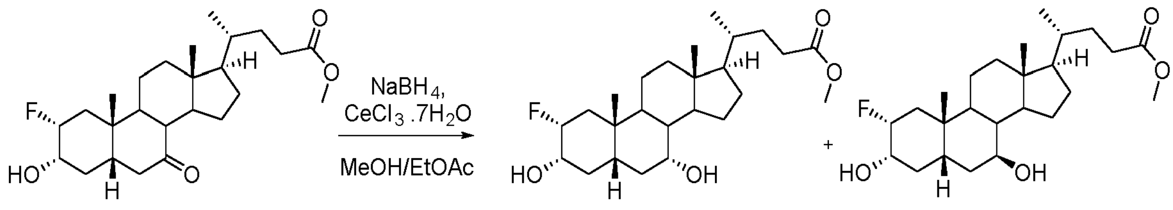


**2E.1** **2G.1** (27%) **2G.2** (17%)

Using general procedure B, methyl 2α-fluoro-3α-hydroxy-7-oxo-5β-cholanoate (270 mg, 0.64 equiv, 1 equiv) was reduced. Crude purified *via* flash chromatography (PE/acetone : 75:25) to yield 33mg of pure 7α-OH analogue along with 140 mg of a mixture of both 7α-OH and 7β-OH epimers. The mixture was re-purified *via* flash chromatography (PE/EtOAc : 60:40→50:50) to yield further pure methyl 2α-fluoro-3α,7α-dihydroxy-5β-cholanoate (total - 74 mg, 0.17 mmol, 27%) and pure methyl 2α-fluoro-3α,7β-dihydroxy-5β-cholanoate (45 mg, 0.11 mmol, 17%), both as gummy solids.

**2G.1: $^1$H NMR** (400MHz, $CDCl_3$): δ 4.67 (1H, d, J = 52.1 Hz), 3.78 (1H, q, J = 2.4 Hz), 3.59 (3H, s), 3.37 (1H, dddd, J = 28.5, 12.0, 4.4, 2.5 Hz), 2.46 (1H, q, J = 13.0 Hz), 2.37-2.23 (2H, m), 2.20-2.10 (1H, m), 2.04-0.88 (27H, m), 0.88-0.83 (6H, m), 0.59 (3H, s) ppm.

**$^{19}$F NMR** ($CDCl_3$, 376MHz): δ -202.71 (tdd, J = 52.0, 27.7, 8.7 Hz) ppm.

**LRMS** ($ESI^+$) m/z : 407.4 $[M+H-H_2O]^+$, 387.3 $[M+H-H_2O-HF]^+$.

**2G.2: $^1$H NMR** (400MHz, $CDCl_3$): δ 4.74 (1H, d, J = 52.1 Hz), 3.66 (3H, s), 3.63-3.35 (2H, m), 2.43-2.29 (2H, m), 2.28-2.14 (1H, m), 2.08-1.00 (29H, m), 0.96 (3H, s), 0.92 (3H, d, J = 6.2 Hz), 0.67 (3H, s) ppm.

**$^{19}$F NMR** ($CDCl_3$, 376MHz): δ -202.49 (tdd, J = 52.0, 29.5, 8.7 Hz) ppm.

**LRMS** ($ESI^+$) m/z 407.4 $[M+H-H_2O]^+$, 387.3 $[M+H-H_2O-HF]^+$.

H. **2α-fluoro-3β,7α-dihydroxy-5β-cholanic acid (Compound 3)**

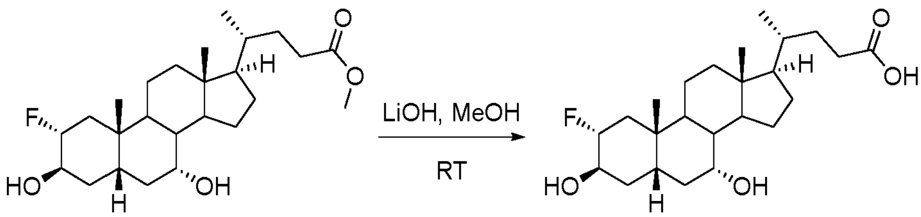


**2F.1** **Compound 3** (94%)

Using general procedure C, methyl 2α-fluoro-3β,7α-dihydroxy-5β-cholanoate (105 mg, 0.25 mmol, 1 equiv) was hydrolysed to yield 2α-fluoro-3β,7α-dihydroxy-5β-cholanic acid **(Compound 3)** (96 mg, 0.23 mmol, 94%) as a pale solid.

**$^1$H NMR** (400MHz, acetone-$D_6$): δ 10.51 (1H, br. s), 4.58 (1H, dq, J = 48.0, 2.7 Hz), 3.96 (1H, dq, J = 7.2, 3.4 Hz), 3.91 (1H, q, J = 2.8 Hz), 2.89 (1H, tt, J = 14.2, 2.6 Hz), 2.43 (1H, ddd, J = 15.5, 10.7, 5.3 Hz), 2.30 (1H, ddd, J = 15.0, 9.4, 6.7 Hz), 2.20-2.06 (4H, m), 2.01-1.13 (21H, m), 1.11-0.97 (6H, m), 0.78 (3H, s) ppm.

**$^{19}$F NMR** (376MHz, acetone-$D_6$): δ -184.37 (tt, J = 49.4, 8.7 Hz) ppm.

**LRMS** ($ESI^-$) m/z : 409.1 $[M-H]^-$, 819.5 $[M-H]^-$.

I. **2α-fluoro-3β,7β-dihydroxy-5β-cholanic acid (Compound 4)**

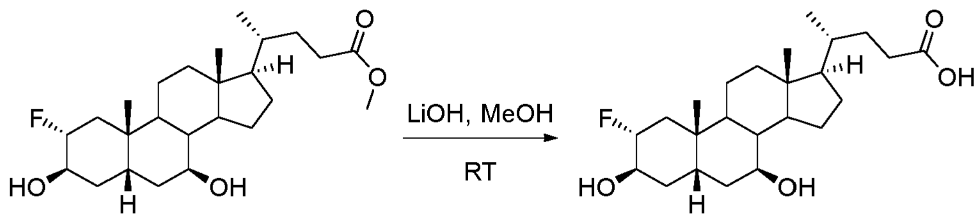


**2F.2** **Compound 4** (93%)

Using general procedure C, methyl 2α-fluoro-3β,7β-dihydroxy-5β-cholanoate (90 mg, 0.21 mmol, 1 equiv) was hydrolysed to yield 2α-fluoro-3β,7β-dihydroxy-5β-cholanic acid **(Compound 4)** (80 mg, 0.19 mmol, 93%) as a colourless solid.

**$^1$H NMR** (400MHz, acetone-$D_6$): δ 10.43 (br. s), 4.58 (1H, dq, J = 48.0, 3.1 Hz), 3.99 (1H, dq, J = 7.1, 3.3 Hz), 3.57 (1H, tdd, J = 10.2, 5.0, 1.0 Hz), 2.42 (1H, ddd, J = 15.5, 11.0, 5.0 Hz), 2.29 (1H, ddd, J = 15.8, 9.2, 6.8 Hz), 2.22-2.06 (4H, m), 2.04-1.14 (23H, m), 1.07 (3H, s), 1.05 (3H, d, J = 6.6 Hz), 0.79 (3H, s) ppm.

**$^{19}$F NMR** (376MHz, acetone-$D_6$): δ -184.29 (tt, J = 49.4, 8.7 Hz) ppm.

**LRMS** (ESI$^-$) m/z : 409.1 $[M-H]^-$, 819.5 $[2M-H]^-$.

J. **2α-fluoro-3α,7α-dihydroxy-5β-cholanic acid (Compound 5)**

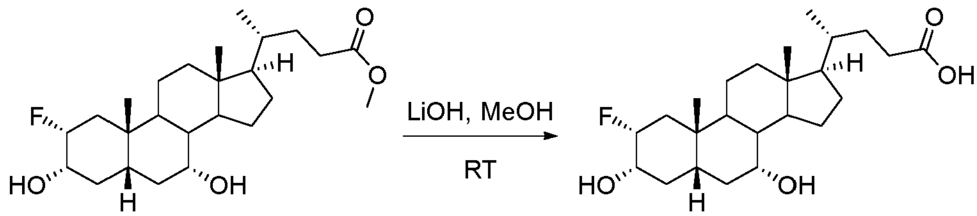


**2G.1** **Compound 5** (93%)

Using general procedure C, methyl 2α-fluoro-3α,7α-dihydroxy-5β-cholanoate (74 mg, 0.17 mmol, 1 equiv) was hydrolysed to yield 2α-fluoro-3a,7a-dihydroxy-5β-cholanic acid **(Compound 5)** (65 mg, 0.16 mmol, 93%) as a colourless solid.

**$^1$H NMR** (400MHz, acetone-$D_6$): δ 4.65 (dq, J = 52.3, 1.7 Hz), 3.81 (1H, q, J = 2.8 Hz), 3.40 (1H, dddd, J = 29.7, 12.0, 3.9, 2.1 Hz), 2.69 (1H, q, J = 12.6 Hz), 2.39-2.16 (3H, m), 2.02-1.01 (25H, m), 0.98-0.91 (6H, m), 0.69 (3H, s) ppm.

**$^{19}$F NMR** (376MHz, acetone-$D_6$): δ -200.79 (tdd, J = 51.2, 29.5, 8.7 Hz) ppm.

**LRMS** (ESI$^-$) m/z : 841.4 $[2M+H]^+$, 393.4 $[M+H-H_2O]^+$, 375.4 $[M+H-H_2O-HF]^+$, 373.4 $[M+H-2H_2O]^+$.

K. **2α-fluoro-3α,7β-dihydroxy-5β-cholanic acid (Compound 6)**

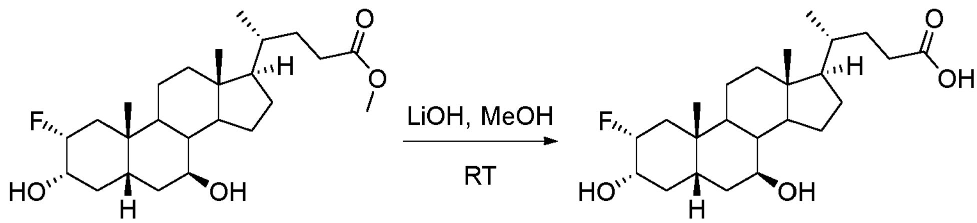


**2G.2** **Compound 6** (97%)

Using general procedure C, methyl 2α-fluoro-3α,7β-dihydroxy-5β-cholanoate (44 mg, 0.10 mmol, 1 equiv) was hydrolysed to yield 2α-fluoro-3α,7β-dihydroxy-5β-cholanic acid **(Compound 6)** (40 mg, 0.97 mmol, 97%) as a colourless solid.

**$^1$H NMR** (400MHz, acetone-$D_6$): δ 4.67 (1H, dq, J = 52.1, 1.7 Hz), 3.61-3.42 (2H, m), 2.39-2.15 (3H, m), 2.02-1.06 (29H, m), 0.99 -0.93 (6H, m), 0.70 (3H, s) ppm.

**$^{19}$F NMR** (376MHz, acetone-$D_6$): δ -200.58 (tdd, J = 50.7, 30.3, 6.9 Hz) ppm.

**LRMS** (ESI$^-$) m/z : 393.4 $[M+H-H_2O]^+$, 375.4 $[M+H-H_2O-HF]^+$, 373.4 $[M+H-2H_2O]^+$.

**Example 3** - **Synthesis of 2,2-difluorinated analogues**

**A. Methyl 3α,7β-dihydroxy-5β-cholanoate (3A.1)**

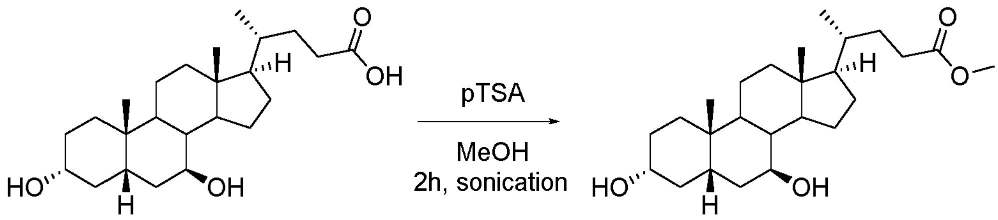


UDCA 3A.1 (quantitative)

Using general procedure A, UDCA (100 g, 250 mmol, 1 equiv) was protected to yield methyl 3α,7β-dihydroxy-5β-cholanoate as a white solid (103 g, 250 mmol, quantitative).

**$^1$H NMR** (400MHz, $CDCl_3$): δ 3.66 (3H, s), 3.73-3.63 (2H, m), 3.58 (2H, td, J = 10.4, 5.3 Hz), 2.35 (1H, ddd, J = 15.3, 10.1, 4.8 Hz), 2.21 (1H, ddd, J = 15.6, 9.6, 6.4 Hz), 1.99 (1H, dt, J = 12.3, 2.8 Hz), 1.95-0.97 (26H, m), 0.94 (3H, s), 0.92 (3H, d, = 6.4 Hz), 0.67 (3H, s) ppm.

**LRMS** ($ESI^+$) m/z : 389.5 $[M+H-H_2O]^+$, 371.5 $[M+H-2H_2O]^+$.

Data consistent with literature (except m.p.); see J. Ren, Y. Wang, J. Wang, J. Lin, K. Wei, R. Huang, *Steroids* **2013,** *78*, 53-58.

B. **Methyl 3α-acetoxy-7β-hydroxy-5β-cholanoate (3B.1)**

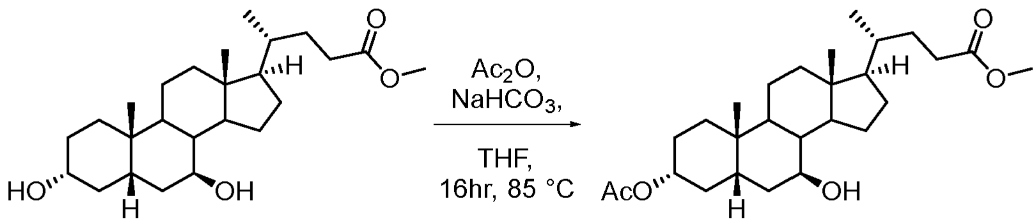


**3A.1** **3B.1** (76%)

Methyl 3α,7β-dihydroxy-5β-cholanoate (30.0 g, 73.8 mmol, 1 equiv), acetic anhydride (35 mL, 369 mmol, 1 equiv) and $NaHCO_3$ (37.2 g, 443 mmol, 6 equiv) were taken up in THF (600 mL) and the reaction mixture was warmed to 85 °C overnight. Reaction mixture was cooled, filtered and the supernatant concentrated *in vacuo* to yield a crude residue. This was taken up in EtOAc and brine (300 mL each), the layers were then separated and the aqueous extracted with further EtOAc (2×200 mL). The combined organics were dried ($Na_2SO_4$) and concentrated to yield 37 g of clear gum/liquid. The crude was purified *via* flash chromatography (pet ether/EtOAc: 85:15→80:20→70:30) to yield methyl 3α-acetoxy-7β-hydroxy-5β-cholanoate as a gummy solid (25.3 g, 56.4 mmol, 76%).

**$^1$H NMR** (400MHz, $CDCl_3$): δ 4.64 (1H, tt, J = 10.5, 5.5 Hz), 3.64 (3H, s), 3.55 (1H, ddd, J = 11.5, 8.7, 5.1 Hz), 2.33 (1H, ddd, J = 15.5, 11.0, 5.0 Hz), 2.20 (1H, ddd, J = 15.6, 9.6, 6.4 Hz), 2.00 (3H, s), 1.97-0.98 (24H, m), 0.93 (3H, s), 0.90 (3H, d, J = 6.4 Hz), 0.65 (3H, s) ppm.

**LRMS** ($ESI^+$) m/z : 471.5 $[M+Na]^+$, 371.4 $[M+H-H_2O-HOAc]^+$.

C. **Methyl 3α-acetoxy-7β-methoxymethoxyl-5β-cholanoate (3C.1)**

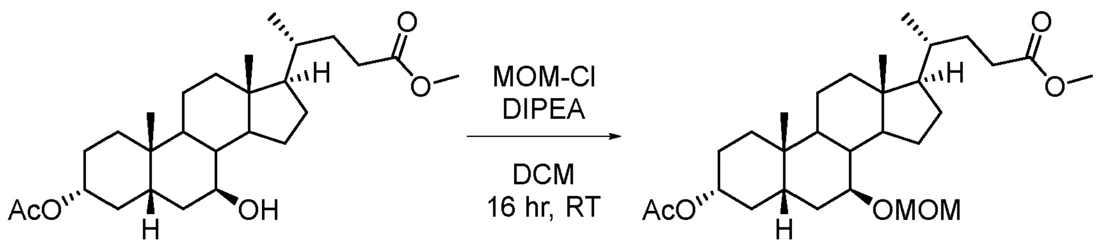


**3B.1** **3C.1** (79%)

Using general procedure K, methyl 3α-acetoxy-7β-hydroxy-5β-cholanoate (72 g, 160.5 mmol) was protected as a MOM ether. Crude purified *via* flash chromatography (pet ether/EtOAc: 85:15→80:20→70:30→60:40) to yield methyl 3α-acetoxy-7β-methoxymethoxyl-5β-cholanoate as a gummy solid (62 g, 126 mmol, 79%).

**$^1$H NMR** (400MHz, $CDCl_3$): δ 4.70-4.61 (1H, m), 4.60 (2H, s), 3.64 (3H, s), 3.39-3.25 (4H, m), 2.33 (ddd, J = 15.5, 11.0, 5.0 Hz), 2.19 (1H, ddd, J = 15.5, 9.6, 6.4 Hz), 2.00 (3H, s), 1.90-0.98 (25H, m), 0.94 (3H, s), 0.90 (3H, d, J = 6.4 Hz), 0.65 (3H, s) ppm.

**LRMS** ($ESI^+$) m/z : 515.5 $[M+Na]^+$, 371.5 $[M+H-HOCH_2OCH_3-HOAc]^+$.

D. **Methyl 3α-hydroxy-7β-methoxymethoxyl-5β-cholanoate (3D.1)**

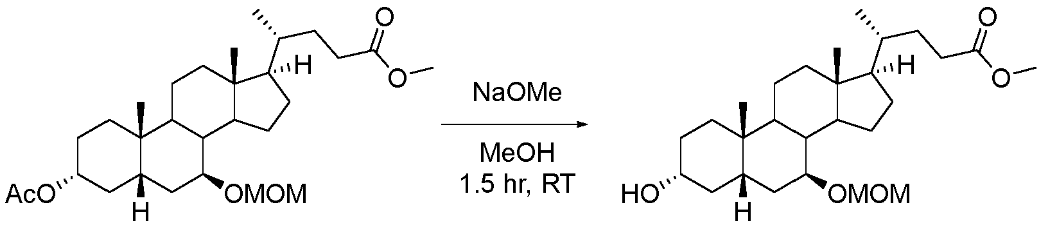


**3C.1** **3D.1** (quantitative)

Using general procedure E, methyl 3α-acetoxy-7β-methoxymethoxyl-5β-cholanoate (82 g, 166 mmol, 1 equiv) was hydrolysed to yield methyl 3α-hydroxy-7β-methoxymethoxyl-5β-cholanoate as a pale yellow gum (75 g, 166 mmol, quantitative yield).

**$^1$H NMR** (400MHz, $CDCl_3$): δ 4.61 (2H, s), 3.65 (3H, s), 3.56 (1H, tt, J = 10.5, 5.0 Hz), 3.41-3.25 (4H, m), 2.34 (1H, ddd, J = 15.5, 11.0, 5.0 Hz), 2.20 (1H, ddd, J = 15.5, 9.6, 6.4 Hz), 2.02-1.90 (1H, m), 1.89-1.72 (6H, m), 1.70-0.97 (19H, m), 0.94 (3H, s), 0.90 (3H, d, J = 6.4 Hz), 0.66 (3H, s) ppm.

**LRMS** ($ESI^+$) m/z : 371.5 $[M+H-HOCH_2OCH_3-H_2O]^+$.

E. **Methyl 7β-methoxymethoxyl-5β-chol-2-enoate (3E.1) and methyl 7β-methoxymethoxyl-5β-chol-3-enoate (3E.2)**

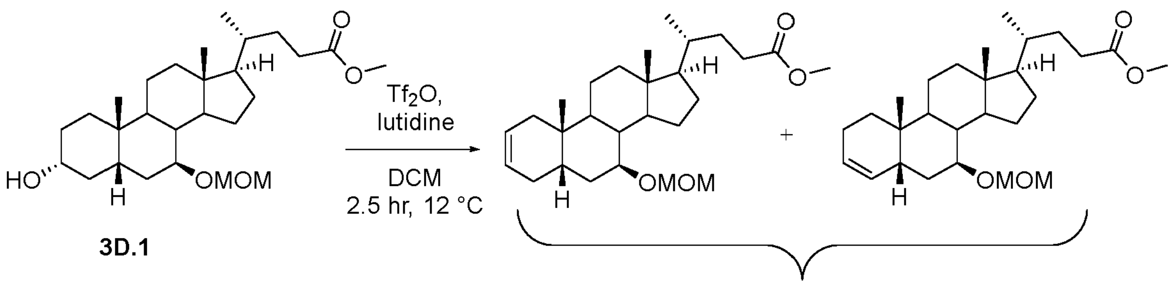


**3D.1**

**3E.1 + 3E.2** (89%)

Methyl 3α-hydroxy-7β-methoxymethoxyl-5β-cholanoate (75 g, 166 mmol, 1 equiv) was dissolved in DCM (650 mL) and cooled to 5 °C on ic, before the addition of lutidine (58 mL< 500 mmol, 3 equiv) and $Tf_2O$ (31 mL, 183 mmol, 1.1 equiv). Reaction mixture warmed to 8-10 °C for 1 h however reaction incomplete, further lutidine (25 mL) and $Tf_2O$ (15 mL), and RM further warmed to 12-14 °C for a further 1.5 h. Reaction deemed complete by TLC analysis. Reaction mixture dry loaded onto silica, and purified *via* flash chromatography (pet ether/EtOAc : 98:2→97:3→95:5) to yield an inseparable mixture of methyl 7β-methoxymethoxyl-5β-chol-2-enoate and methyl 7β-methoxymethoxyl-5β-chol-3-enoate as a pale yellow gum (64.1 g, 148 mmol, 89%).

**3E.1/3E.2: $^1$H NMR** (400MHz, $CDCl_3$): δ 5.74-5.34 (2H, m), 4.68-4.62 (2H, m), 3.66 (3H, s), 3.37 (3H, s), 3.13 (1H, td, J = 10.2, 5.0 Hz), 2.32 (1H, ddd, J = 15.5, 10.3, 5.0 Hz), 2.26-2.16 (1H, m), 2.15-1.00 (27H, m), 0.98 (2H, s), 0.92 (2H, d, J = 6.4 Hz), 0.86 (2H, d, J = 6.6 Hz), 0.68 (3H s) ppm.

F. **Methyl 2α-acetoxy-3β-hydroxy-7β-methoxymethoxyl-5β-cholanoate (3F.1) and methyl 3β,4β-epoxy-7β-methoxymethoxyl-5β-cholanoate (3F.2)**

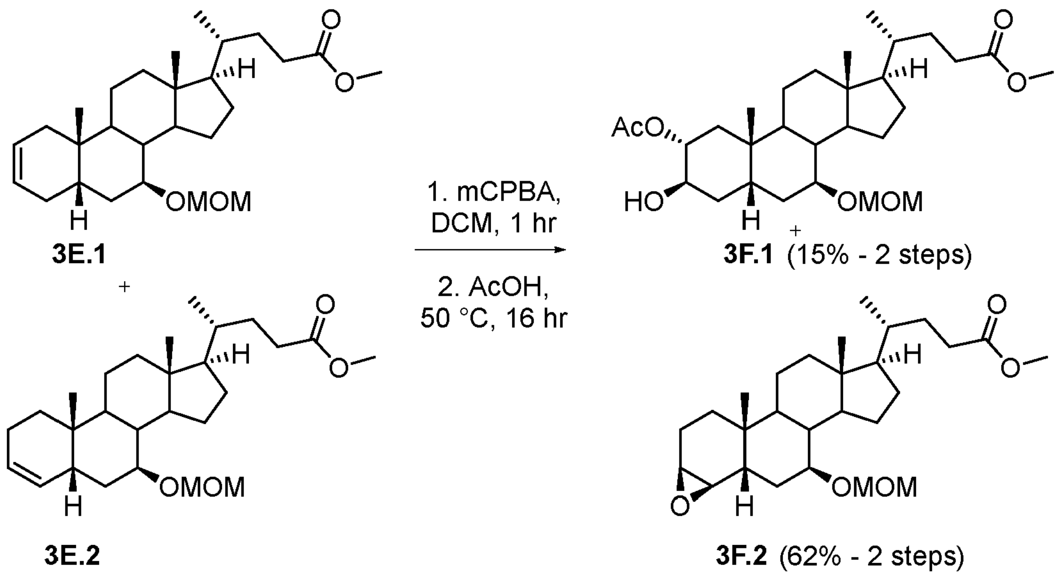


3E.1 + 3E.2

3F.1 (15% - 2 steps) + 3F.2 (62% - 2 steps)

A mixture of methyl 7β-methoxymethoxyl-5β-chol-2-enoate and methyl 7β-methoxymethoxyl-5β-chol-3-enoate (63.0 g, 146 mmol, 1 equiv), along with mCPBA (54.0 g, 1.5 equiv) was dissolved in DCM and stirred for 1 h at RT. RM quenched with sat. aq. $Na_2S_2O_3$ (250 mL) and stirred for 20 min at RT. Layers separated and aqueous extracted with DCM (300 mL). Combined organics washed with sat. aq. $NaHCO_3$ (300 mL), dried ($Na_2SO_4$) and concentrated, to yield 72 g of a pale yellow gum containing an inseparable mixture of Δ2β,3β- and Δ3β,4β-epoxides (assume quantitative yield). This mixture was then dissolved in AcOH (600 mL), and warmed to 50 °C for 16 hr. The reaction mixture was concentrated *in vacuo,* then azeotroped (EtOAc×3, DCM×1), before the crude was purified *via* flash chromatography (pet ether/EA : 85:15→80:20→70:30→60:40→50:50) to yield methyl 2α-acetoxy-3β-hydroxy-7β-methoxymethoxyl-5β-cholanoate as a gummy solid (11.1 g, 21.9 mmol, 15% - 2 steps) and methyl 3β,4β-epoxy-7β-methoxymethoxyl-5β-cholanoate as a gummy solid (40.5 g, 90 mmol, 62% - 2 steps).

**3F.1:** $^1$**H NMR** (400MHz, $CDCl_3$): δ 4.74 (1H, q, J = 3.9 Hz), 4.62 (2H, s), 3.79 (1H, q, J = 3.7 Hz), 3.64 (3H, s), 3.36-3.31 (4H, m), 2.33 (1JH, ddd, J = 15.5, 11.0, 5.0 Hz), 2.20 (1H, ddd, J = 15.5, 9.6, 6.4 Hz), 2.03 (3H, s), 2.01-0.99 (29H, m), 0.98 (3H, s), 0.90 (3H, d, J = 6.4 Hz), 0.65 (3H, s) ppm.

**LRMS** ($ESI^+$) m/z : 531.6 $[M+Na]^+$, 387.4 $[M+H-HOCH_2OCH_3-HOCH_2OCH_3]^+$.

**3F.2:** $^1$**H NMR** (400MHz, $CDCl_3$): δ 4.64 (2H, s), 3.64 (3H, s), 3.35 (3H, s), 3.19 (1H, br. s), 3.10 (1H, td, J = 10.8, 4.5 Hz), 2.88 (1H, d, J = 3.7 Hz), 2.32 (1H, ddd, J = 15.5, 11.0, 5.0 Hz), 2.19 (1H, ddd, J = 15.5, 9.6, 6.4 Hz), 2.12-2.04 (1H, m), 2.03-0.95 (25H, m), 0.90 (3H, d, J = 6.4 Hz), 0.87 (3H, s), 0.65 (3H, s) ppm.

**LRMS** ($ESI^+$) m/z : 417.4 [M, partial -MOM cleavage]$^+$, 387.4 $[M+H-HOCH_2OCH_3]^+$.

G. **Methyl 2α-acetoxy-3β,7β-dimethoxymethoxyl-5β-cholanoate (3G.1)**

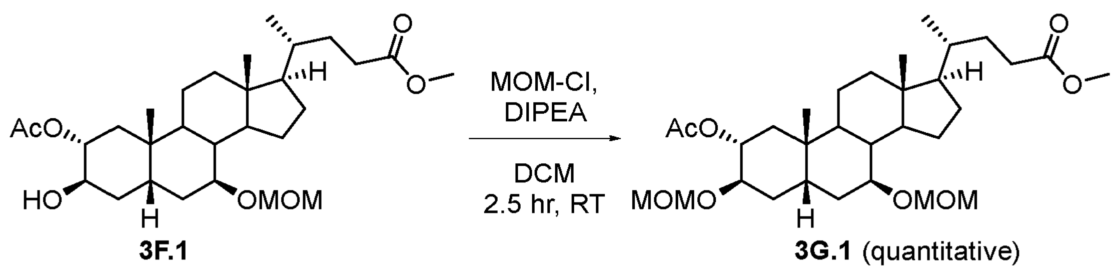


3F.1 → 3G.1 (quantitative)

Using general procedure K, methyl 2α-acetoxy-3β-hydroxy-7β-methoxymethoxyl-5β-cholanoate (11.0 g, 21.6

mmol, 1 equiv) was protected as the MOM derivative to yield methyl 2α-acetoxy-3β,7β-dimethoxymethoxyl-5β-cholanoate as a pale yellow oil/gum (13.0 g, quantitative).

**$^1$H NMR** (400MHz, $CDCl_3$): δ 4.83 (1H, q, J = 3.1 Hz), 4.62 (2H, s), 4.62 (2H, s), 3.67 (1H, q, J = 2.9 Hz), 3.63 (3H, s), 3.33 (3H, s), 3.33 (3H, s), 3.32-3.30 (1H, m), 2.32 (1H, ddd, J = 15.5, 11.0, 5.0 Hz), 2.19 (1H, ddd, J = 15.6, 9.4, 6.5 Hz), 2.01 (3H, s), 1.99-1.25 (25H, m), 0.95 (3H, s), 0.89 (3H, d, J = 6.4 Hz), 0.65 (3H, s) ppm.

**LRMS** ($ESI^+$) m/z : 575.6 $[M+Na]^+$, 491.6 $[M-HOCH_2OCH_3]^+$, 429.5 $[M-2HOCH_2OCH_3]^+$.

H. **Methyl 2α-hydroxy-3β,7β-dimethoxymethoxyl-5β-cholanoate (3H.1)**

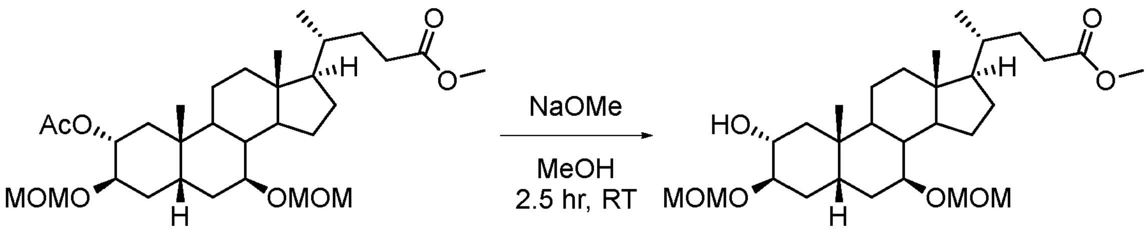


**3G.1** **3H.1** (85%)

Using general procedure E, methyl 2α-acetoxy-3β,7β-dimethoxymethoxyl-5β-cholanoate (13.0 g, 21.6 mmol, 1 equiv) was methanolysed to yield methyl 2α-hydroxy-3β,7β-dimethoxymethoxyl-5β-cholanoate as a pale yellow gum (9.5 g, 18.3 mmol, 85%).

**$^1$H NMR** (400MHz, $CDCl_3$): δ 4.65 (1H, d, J = 6.8 Hz), 4.62 (1H, d, J = 6.8 Hz), 4.60 (1H, d, J = 6.8 Hz), 4.57 (1H, d, J = 6.8 Hz), 3.65-3.59 (4H, m), 3.43-3.36 (1H, m), 3.34 (3H, s), 3.33-3.31 (1H, m), 3.31 (3H, s), 2.96 (1H, br. s), 2.29 (1H, ddd, J = 15.6, 10.5, 5.0 Hz), 2.16 (1H, ddd, J = 15.6, 9.4, 6.5 Hz), 1.97-1.23 (21H, m), 1.16-0.96 (4H, m), 0.92 (3H, s), 0.86 (3H, d, J = 6.4 Hz), 0.61 (3H, s) ppm.

**LRMS** ($ESI^+$) m/z : 533.7 $[M+Na]^+$, 399.5 $[M-HOCH_2OCH_3-H_2O-OMe]^+$, 387.4 $[M+H-2HOCH_2OCH_3]$.

I. **Methyl 2-oxo-3β,7β-dimethoxymethoxyl-5β-cholanoate (3I.1)**

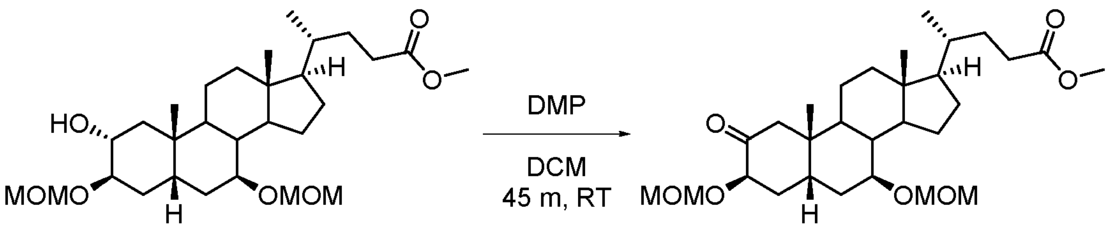


**3H.1** **3I.1** (93%)

Using general procedure M, methyl 2α-hydroxy-3β,7β-dimethoxymethoxyl-5β-cholanoate (9.2 g, 18.0 mmol 1 equiv) was oxidised, then purified *via* flash chromatography (pet ether/EtOAc : 80:20→70:30→65:35) to yield methyl 2-oxo-3β,7β-dimethoxymethoxyl-5β-cholanoate as a pale gummy solid (8.5 g, 16.7 mmol, 93%).

**$^1$H NMR** (400MHz, $CDCl_3$): δ 4.62 (2H, d, J = 7.0 Hz), 4.58 (2H, d, J = 7.0 Hz), 3.76 (1H, t, J = 2.6 Hz), 3.63 (3H, s), 3.34 (3H, s), 3.32 (3H, s), 3.31-3.23 (1H, m), 2.64 (1H, d, J = 12.8 Hz), 2.38-2.03 (5H, m), 2.01-1.25 (14H, m), 1.21-1.10 (2H, m), 1.09 (3H, s), 0.87 (3H, d, J = 6.4 Hz), 0.63 (3H, s) ppm.

**LRMS** ($ESI^+$) m/z : 531.6 $[M+Na]^+$, 477.6 $[M-OMe]^+$, 415.5 $[M-HOCH_2OCH_3-OMe]^+$.

J. **Methyl 2,2-difluoro-3β,7β-dimethoxymethoxyl-5β-cholanoate (3J.1) and methyl 2-fluoro-3β,7β-dimethoxymethoxyl-5β-chol-1-enoate (3J.2)**

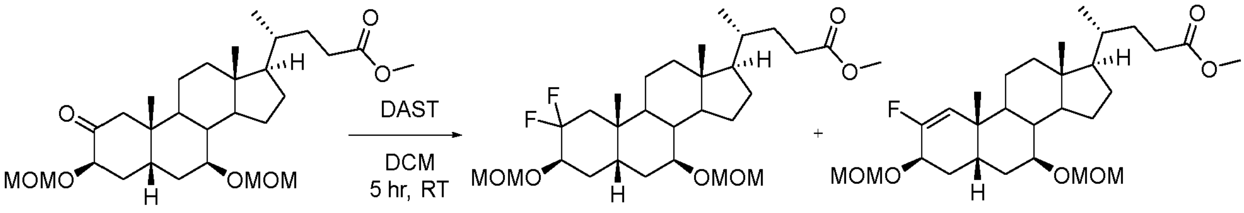


3I.1 3J.1 (21%) 3J.2 (12%)

Methyl 2-oxo-3β,7β-dimethoxymethoxyl-5β-cholanoate (8.0 g, 15.7 mmol, 1 equiv) was dissolved in DCM (40 mL) before the addition of DAST (1004 mL, 78.6 mmol, 5 equiv) and the reaction mixture stirred at RT for 5 hr. Mixture was then diluted with DCM (100 mL) before adding dropwise to an ice-cold sat. aq. solution of $NaHCO_3$ (150 mL), then stirred for 20 mins. Layers were separated then aqueous was extracted with DCM (100 mL), combined organics were then dried ($Na_2SO_4$) and concentrated to yield 7.5 g of a pale brown gum/oil. Crude purified *via* flash chromatography (pet ether/EtOAc : 90:10→85:15→80:20) to yield methyl 2,2-difluoro-3β,7β-dimethoxymethoxyl-5β-cholanoate (1.75 g, 3.3 mmol, 21%) along with methyl 2-fluoro-3β,7β-dimethoxymethoxyl-5β-chol-1-enoate (970 mg, 1.9 mmol, 12%) both as gummy solids.

**3J.1: $^1$H NMR** (400MHz, $CDCl_3$): δ 4.72 (1H, d, J = 6.6 Hz), 4.66 (1H, d, J = 6.6 Hz), 4.63 (2H, s), 3.81 (1H, br. s), 3.66 (3H, s), 3.38 (3H, s), 3.36 (3H, s), 3.31-3.21 (1H, s), 2.34 (1H, ddd, J = 15.6, 10.5, 5.0 Hz), 2.21 (1H, ddd, J = 15.6, 9.4, 6.5 Hz), 2.12 (1H, br. s), 2.05-1.08 (25H, m), 1.04 (3H, s), 0.92 (3H, d, J = 6.4 Hz), 0.67 (3H, s) ppm.

**$^{19}$F NMR** (376MHz, $CDCl_3$): δ -99.89 (d, J = 259.0 Hz), -102.89 (ddt, J = 250.6, 40.7, 5.0 Hz) ppm

**LRMS** ($ESI^+$) m/z : 553.5 $[M+Na]^+$, 437.5 $[M-HOCH_2OCH_3-OCH_3]^+$.

**3J.2: $^1$H NMR** (400MHz, $CDCl_3$): δ 5.39 (1H, d, J = 17.7 Hz), 4.71 (1H, d, J = 6.8 Hz), 4.69 (1H, d, J = 6.8 Hz), 4.64 (2H, s), 4.12-4.08 (1H, m), 3.66 (3H, s), 3.40 (3H, s), 3.36 (3H, s), 3.27-3.17 (1H, m), 2.34 (1H, ddd, J = 15.6, 10.5, 5.0 Hz), 2.21 (1H, ddd, J = 15.6, 9.4, 6.5 Hz), 2.12-1.14 (24H, m), 1.12 (3H, s), 1.10-0.96 (2H, m), 0.91 (3H, d, J = 6.4 Hz), 0.68 (3H, s) ppm.

**$^{19}$F NMR** (376MHz, $CDCl_3$): δ -115.57 (dt, J = 17.0, 8.5 Hz) ppm.

**LRMS** ($ESI^+$) m/z : 448.6$[M+ H-HOCH_2OCH_3]^+$, 387.4 $[M+H-2HOCH_2OCH_3]^+$.

K. **Methyl 2,2-difluoro-3β,7β-dihydroxy-5β-cholanoate (3K.1)**

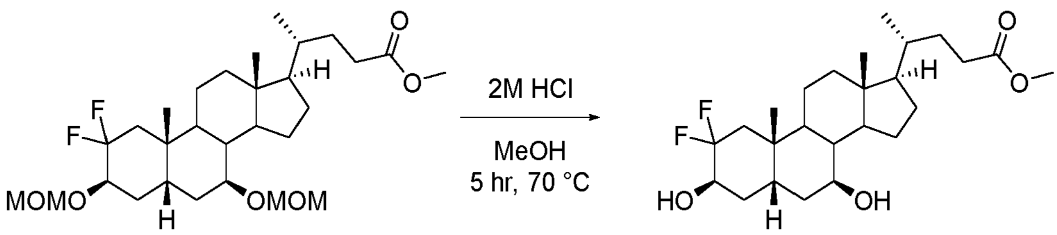


3J.1 3K.1 (quantitative)

Following general procedure L, methyl 2,2-difluoro-3β,7β-dimethoxymethoxyl-5β-cholanoate (1.5 g, 1.76 mmol, 1 equiv) deprotected to yield methyl 2,2-difluoro-3β,7β-dihydroxy-5β-cholanoate as a gummy solid (1.3 g, quantitative yield).

**$^1$H NMR** (400MHz, $CDCl_3$): δ 3.89 (1H, t, J = 5.7 Hz), 3.67 (3H, s), 3.54 (1H, ddd, J = 11.6, 9.2, 5.1 Hz), 2.36 (1H, ddd, J = 15.6, 10.5, 5.0 Hz), 2.22 (1H, ddd, J = 15.6, 9.4, 6.5 Hz), 2.17-1.07 (27H, m), 1.05 (3H, s), 0.93 (3H, d, J = 6.4 Hz), 0.69 (3H, s) ppm.

**$^{19}$F NMR** (376MHz, $CDCl_3$): δ -100.05 (d, J = 251.4 Hz), -105.16 (ddt, J = 252.1, 40.5, 7.2 Hz) ppm.

**LRMS** ($ESI^+$) m/z : 425.5 $[M+H-H_2O]^+$, 405.5 $[M+H-H_2O-HF]^+$.

L. **Methyl 2,2-difluoro-3,7-dioxo-5β-cholanoate (3L.1) + hydrate (3L.2)**

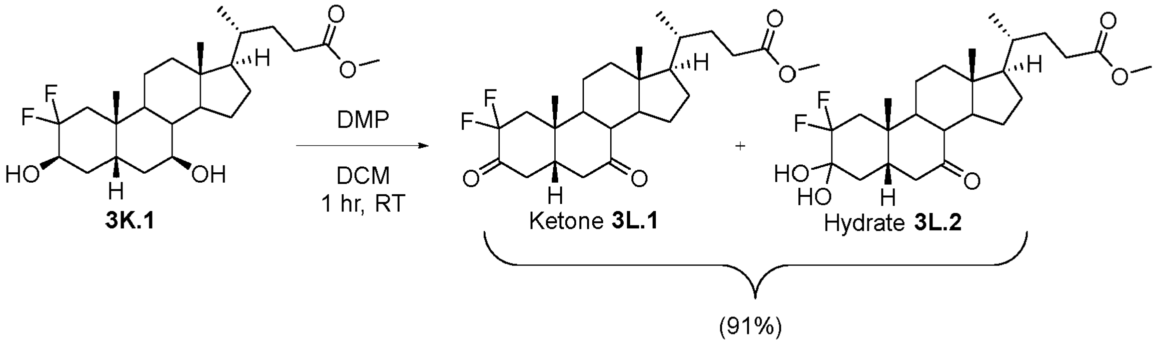


Using general procedure M, methyl 2,2-difluoro-3β,7β-dihydroxy-5β-cholanoate (1.0 g, 2.26 mmol, 1 equiv) was oxidised to yield a mixture of methyl 2,2-difluoro-3,7-dioxo-5β-cholanoate + hydrate (900 mg, 2.05 mmol, 91% - combined yield).

**3L.1** **$^1$H NMR** (400MHz, $CDCl_3$): δ 3.67 (3H, s), 2.93 (1H, ddd, J = 13.2, 5.5, 0.8 Hz), 2.77-2.63 (2H, m), 2.52-1.38 (23H, m), 1.37 (3H, s), 1.35-0.96 (6H, m), 0.94 (3H, d, J = 6.5 Hz), 0.70 (3H, s) ppm.

**$^{19}$F NMR** (376MHz, $CDCl_3$): δ -104.37 (ddd, J = 263.6, 39.9, 12.1 Hz), -111.15 (dq, J = 263.6, 5.0 Hz) ppm.

**LRMS** ($ESI^+$) m/z : 439.5 $[M+H]^+$.

**3L.2 LRMS** ($ESI^+$) m/z : 457.5 $[M+H]^+$.

M. **Methyl 2,2-difluoro-3α,7α-dihydroxy-5β-cholanoate (3M.1) + methyl 2,2-difluoro-3α,7β-dihydroxy-5β-cholanoate (3M.2)**

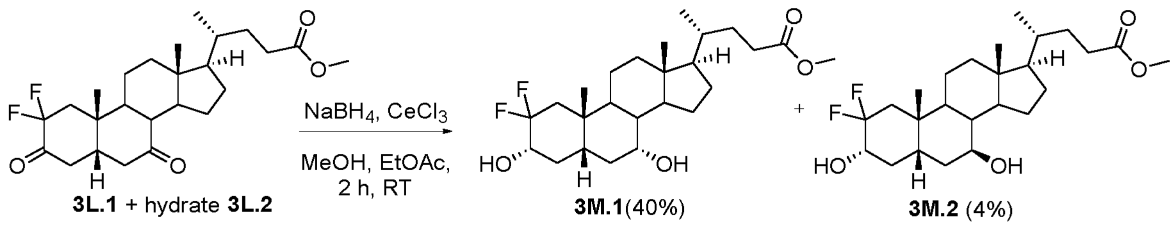


Using general procedure B, a mixture of methyl 2,2-difluoro-3,7-dioxo-5β-cholanoate and the hydrate (750 mg, 1.71 mmol, 1 equiv) were reduced. Crude was purified *via* flash chromatography (petrol ether/EtOAc : 70:30→60:40→50:50) to yield methyl 2,2-difluoro-3α,7α-dihydroxy-5β-cholanoate (300 mg, 0.68 mmol, 40%) and methyl 2,2-difluoro-3α,7β-dihydroxy-5β-cholanoate (26 mg, 0.06 mmol, 4%).

**3M.1: $^1$H NMR** (400MHz, $CDCl_3$): δ 3.86 (1H, q, J = 2.7 Hz), 3.67 (3H, s), 3.65-3.56 (1H, m), 2.54 (1H, q, J = 13.2 Hz), 2.47-2.31 (2H, m), 2.28-2.17 (1H, m), 2.05-1.04 (19H, m), 1.00 (3H, s), 0.94 (3H, d, J = 6.5 Hz), 0.67 (3H, s) ppm.

**$^{19}$F NMR** (376MHz, $CDCl_3$): δ -101.58 (dquin, J = 235.8, 5.2 Hz), -119.95 (dddd, J = 235.8, 39.9, 20.8, 10.4 Hz) ppm.

**LRMS** ($ESI^+$) m/z : 425.5 $[M+H-H_2O]^+$.

**3M.2: $^1$H NMR** (400MHz, $CDCl_3$): δ 3.73 (1H, ddt, J = 19.7, 10.9, 5.4 Hz), 3.67 (3H, s), 3.57 (1H, ddd, J = 11.3, 9.5, 5.1 Hz), 2.43-2.30 (2H, m), 2.22 (2H, ddd, J = 15.7, 9.4, 6.6 Hz), 2.04-1.05 (31H, m), 1.02 (3H, s), 0.93 (3H, d, J = 6.4 Hz), 0.68 (3H, s) ppm.

**$^{19}$F NMR** (376MHz, $CDCl_3$): δ -101.31 (dquin, J = 237.6, 5.2 Hz), -119.06 (dddd, J = 237.6, 38.2, 19.0, 10.2 Hz) ppm.

**LRMS** ($ESI^+$) m/z : 425.5 $[M+H-H_2O]^+$.

N. **2,2-difluoro-3β,7β-dihydroxy-5β-cholanic acid (Compound 7)**

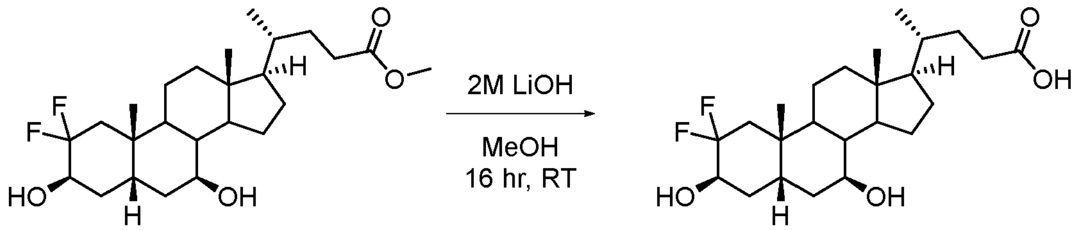


3K.1 Compound 7 (83%)

Using general procedure C, methyl 2,2-difluoro-3β,7β-dihydroxy-5β-cholanoate (60 mg, 0.14 mmol, 1 equiv) was hydrolysed to yield 2,2-difluoro-3β,7β-dihydroxy-5β-cholanic acid **(Compound 7)** as a pale solid (50 mg, 0.12 mmol, 83%).

**$^1$H NMR** (400MHz, $CDCl_3$): δ 3.89 (1H, br. s), 3.55 (1H, ddd, J = 11.4, 9.2, 5.2 Hz), 2.39 (1H, ddd, J = 15.6, 10.5, 5.0 Hz), 2.26 (1H, ddd, J = 15.8, 9.4, 6.5 Hz), 2.18-1.07 (26H, m), 1.05 (3H, s), 0.94 (3H, d, J = 6.5 Hz), 0.69 (3H, s) ppm.

**$^{19}$F NMR** (376MHz, $CDCl_3$): δ -100.00 (d, J = 253.2 Hz), -105.12 (ddt, J = 251.4, 41.6, 8.0 Hz) ppm.

**LRMS** ($ESI^+$) m/z : 411.5 $[M+H-H_2O]^+$, 391.5 $[M+H-H_2O-HF]^+$.

O. **2,2-difluoro-3α,7α-dihydroxy-5β-cholanic acid (Compound 8)**

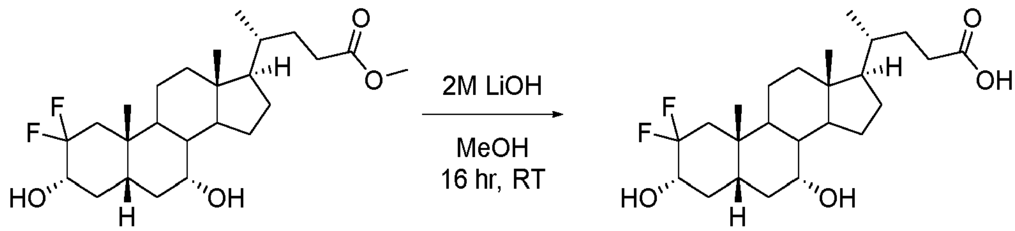


3M.1 Compound 8 (96%)

Using general procedure C, methyl 2,2-difluoro-3α,7α-dihydroxy-5β-cholanoate (75 mg, 0.17 mmol, 1 equiv) was hydrolysed to yield 2,2-difluoro-3a,7a-dihydroxy-5β-cholanic acid**(Compound 8)** as a white solid (70 mg, 0.16 mmol, 96%).

**$^1$H NMR** (400MHz, $CDCl_3$): δ 33.86 (1H, q, J = 2.0 Hz), 3.62 (1H, ddt, J = 19.7, 10.9, 5.4 Hz), 2.54 (1H, q, J = 13.0 Hz), 2.44-2.35 (2H, m), 2.25 (1H, ddd, J = 15.9, 9.6, 6.5 Hz), 2.02-1.02 (25H, m), 0.99 (3H, s), 0.94 (3H, d, J = 6.4 Hz), 0.67 (3H, s) ppm.

**$^{19}$F NMR** (376MHz, $CDCl_3$): δ -119.67 (d, J = 235.8 Hz), -119.67 (dddd, J = 235.8, 38.2, 19.1, 10.4 Hz) ppm.

**LRMS** ($ESI^+$) m/z : 411.5 $[M+H-H_2O]^+$, 393.4 $[M+H-2H_2O]^+$.

P **2,2-difluoro-3,7-dioxo-5β-cholanic acid (Comparative Compound E) + hydrate (3P.1)**

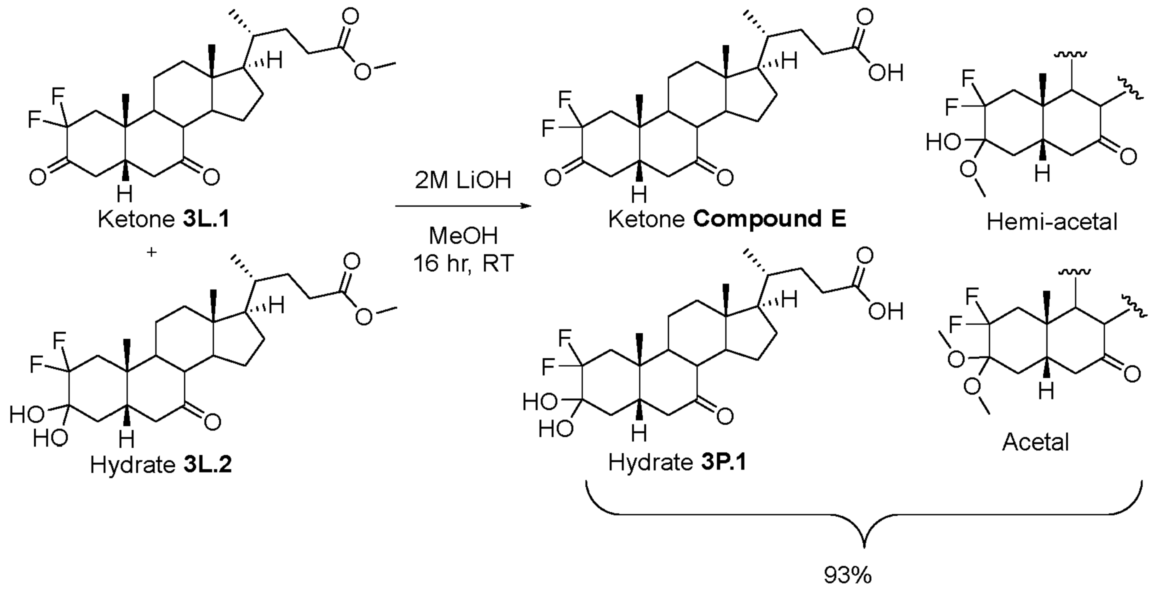


Ketone **3L.1** + Hydrate **3L.2**

Ketone **Compound E**

Hemi-acetal

Hydrate **3P.1**

Acetal

Using general procedure C, methyl 2,2-difluoro-3,7-dioxo-5β-cholanoate and the hydrate (60 mg, 0.14 mmol, 1 equiv) were hydrolysed to yield 2,2-difluoro-3,7-dioxo-5β-cholanic acid **(Compound E)** and the hydrate as a white solid, as a mixture of ketone/hydrate/acetal adducts (55 mg, 0.13mmol, 93%).

**$^1$H NMR** (400MHz, $CD_3CN$): δ 2.93 (1H, dd, J = 13.1, 5.9 Hz), 2.90-2.84 (1H, m), 2.70-1.97 (9H, m), 1.93-0.93 (19H, m), 0.91 (2H, d, J = 6.4 Hz), 0.89 (1H, d, J = 6.4 Hz), 0.67 (2H, s), 0.64 (1H, s) ppm.

**$^{19}$F NMR** (376MHz, $CD_3CN$): δ -103.96 (1F, ddd, J = 261.6, 38.1, 15.0 Hz), -108.59 (0.2F, ddd, J = 246.2, 39.0, 11.3 Hz), -110.91 (1F, dq, J = 263.6, 5.2 Hz), -113.71--112.69 (0.1F, dq, J = 246.2, 5.2 Hz), -116.15 (0.1F, dq, J = 246.2, 5.2 Hz), -117.09 (0.2F, dq, J = 246.2, 5.2 Hz) ppm.

**LRMS** ($ESI^+$) m/z : **Ketone** : 425.5 $[M+H]^+$; **Hydrate** : 443.5 $[M+H]^+$; **Hemi-acetal** : 457.7 $[M+H]^+$, 439.5 $[M+H-H_2O]^+$; **Acetal** : 443.5 $[M+H]^+$, 439.5 $[M+H-MeOH]^+$.

Q. **2,2-difluoro-3α,7β-dihydroxy-5β-cholanic acid (Compound 9)**

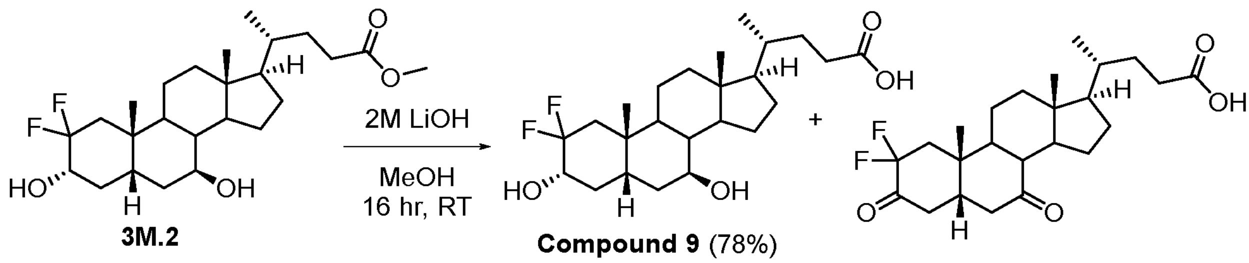


**3M.2**

**Compound 9** (78%) +

Using general procedure C, methyl 2,2-difluoro-3α,7β-dihydroxy-5β-cholanoate (25 mg, 0.06 mmol, 1 equiv) was hydrolysed to yield 2,2-difluoro-3α,7β-dihydroxy-5β-cholanic acid**(Compound 9)** as a gummy solid (20 mg, 0.05 mmol, 78%).

**$^1$H NMR** (400MHz, $CD_3OD$): δ 3.69 (1H, ddt, J = 21.0, 11.0, 5.0 Hz), 3.44 (1H, ddd, J = 11.5, 9.8, 5.0 Hz), 2.38-2.26 (2H, m), 2.25-2.14 (2H, m), 2.08-1.06 (32H, m), 1.03 (3H, s), 0.96 (3H, d, J = 6.5 Hz), 0.72 (3H, s) ppm.

**$^{19}$F NMR** (376MHz, $CD_3OD$): δ -101.64 (dquin, J = 239.3, 5.0 Hz), -120.18 (dddd, J = 239.3, 38.2, 20.8, 10.4 Hz) ppm.

**LRMS** ($ESI^+$) m/z : 411.4 $[M+H-H_2O]^+$, 393.3 $[M+H-2H_2O]^+$.

**Example 4 - Synthesis of Conjugates of Compound 7**

A. **Sodium *N*-(2,2-difluoro-3β,7β-dihydroxy-5β-cholan-24-amide)-ethylsulfonic acid (Compound 10)**

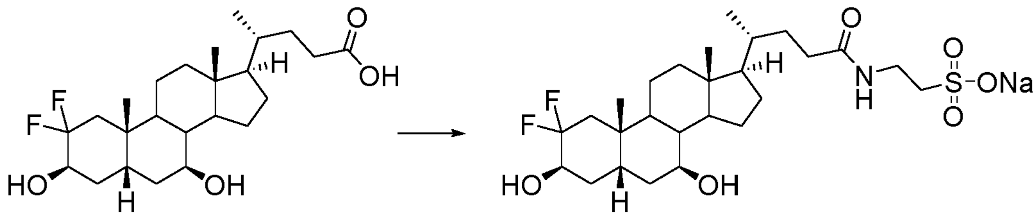

Compound 7

Using general procedure Q, 2,2-difluoro-3a,7a-dihydroxy-5β-cholanic acid (7.4 mg, 0.017 mmol) was conjugated to yield sodium *N*-(2,2-difluoro-3β,7β-dihydroxy-5β-cholan-24-amide)-ethylsulfonic acid as a clear residue (7.4 mg, 77%).

**$^1$H NMR** (400 MHz, MeOD) δ 3.78 (1H, br. s), 3.61-3.56 (2H, m), 3.40 (1H, ddd, J = 14.6, 9.7, 5.2 Hz), 2.95 (2H, app.t., J = 6.9 Hz), 2.24 (1H, ddd, J = 15.0, 10.6, 5.2 Hz), 2.12-1.07 (23H, m), 1.05 (3H, s), 0.97 (3H, d, J = 6.5 Hz), 0.71 (3H, s) ppm.

**$^{19}$F NMR** (376 MHz, MeOD) δ -101.00 (d, J = 251.4 Hz), -105.55 (ddt, J = 250.3, 40.3, 7.8 Hz) ppm.

**LRMS** (ESI$^-$): [M-Na]$^-$ Calcd. 534.2706; found 534.2709.

B. **Sodium *N*-(2,2-difluoro-3β,7β-dihydroxy-5β-cholan-24-amide)-propanoic acid (Compound 11)**

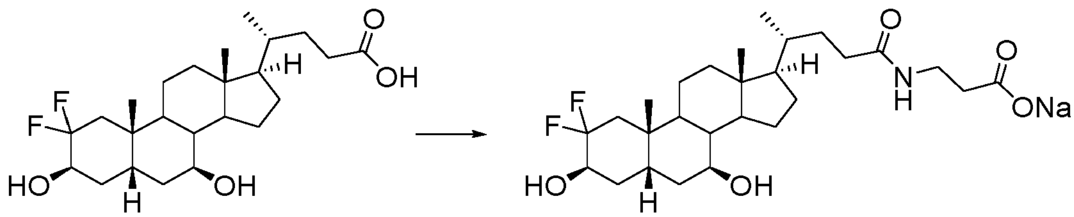

Compound 7

Using general procedure Q, 2,2-difluoro-3a,7a-dihydroxy-5β-cholanic acid (20.0 mg, 0.047 mmol) was conjugated to yield sodium *N*-(2,2-difluoro-3β,7β-dihydroxy-5β-cholan-24-amide)-propanoic acid as a clear residue (17.66 mg, 73%).

**$^1$H NMR** (400 MHz, MeOD) δ 3.78 (1H, br. s), 3.45 -3.35 (3H, m), 2.40-2.30 (2H, m), 2.23 (1H, m), 2.12-1.06 (23H, m), 1.05 (3H, s), 0.96 (3H, d, J = 6.4 Hz), 0.71 (3H, s) ppm.

**$^{19}$F NMR** (376 MHz, MeOD) δ -100.62 (d, J = 250.4 Hz), -105.55 (ddt, J = 250.4, 40.8, 7.6 Hz) ppm.

**HRMS** (ESI$^+$): [M+Na]$^+$ Calcd. 522.3002; found 522.3007.

C. ***N*-(methyl),*N*-(2,2-difluoro-3β,7β-dihydroxy-5β-cholan-24-amide)-acetic acid (Compound 12)**

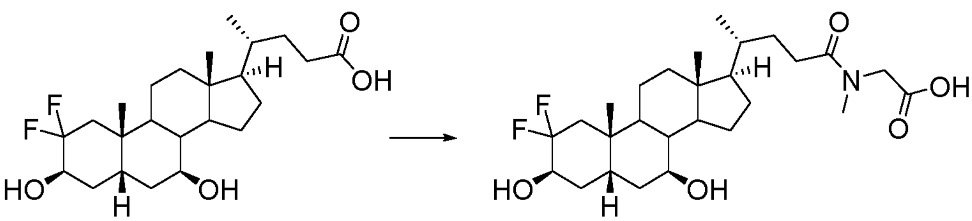

Compound 7

Using general procedure Q, 2,2-difluoro-3a,7a-dihydroxy-5β-cholanic acid (25.36 mg, 0.059 mmol) was conjugated to yield *N*-(methyl),*N*-(2,2-difluoro-3β,7β-dihydroxy-5β-cholan-24-amide)-acetic acid as a clear residue (13.3 mg, 45%).

**$^1$H NMR** (400 MHz, MeOD) δ 3.96 (2H, br. d, J = 18.1 Hz), 3.78 (1H, br. s), 3.40 (1H, m), 3.08 (1.5H, s), 2.94 (1.5H, s), 2.48 (1H, m), 2.41-1.06 (23H, m), 1.05 (1.5H, s), 1.05 (1.5H, s), 1.00 (1.5H, d, J = 6.5 Hz), 0.96 (1.5H, d, J = 6.5 Hz), 0.73

(1.5H, s), 0.71 (1.5H, s) ppm; **$^{19}$F NMR** (376 MHz, MeOD) δ -100.62 (d, J = 250.7 Hz), -105.55 (ddt, J = 250.0, 40.2, 7.2 Hz) ppm.

**HRMS** (ESI$^+$): $[M+Na]^+$ Calcd. 522.3002; found 522.2998.

D. **Sodium** *N*-(2,2-difluoro-3β,7β-dihydroxy-5β-cholan-24-amide)-trans-2-cyclohexane carboxylic acid (Compound 13)

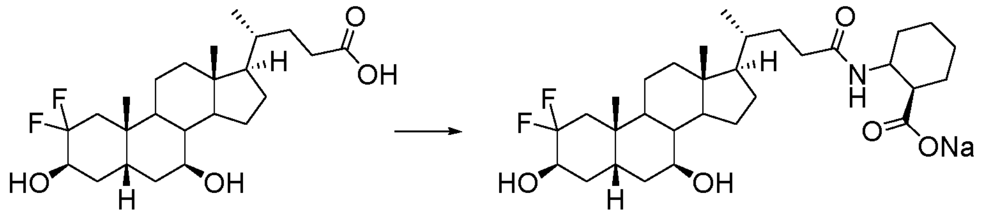

**Compound 7**

Using general procedure Q, 2,2-difluoro-3a,7a-dihydroxy-5β-cholanic acid (20.0 mg, 0.047 mmol) was conjugated to yield Sodium *N*-(2,2-difluoro-3β,7β-dihydroxy-5β-cholan-24-amide)-*trans*-2-cyclohexane carboxylic acid as a clear residue (18.18 mg, 68%).

**$^1$H NMR** (400 MHz, MeOD) δ 3.89-3.72 (2H, m), 3.40 (1H, m), 2.28-1.06 (33H, m), 1.05 (3H, s), 0.96 (3H, d, J = 6.5 Hz), 0.70 (3H, s) ppm.

**$^{19}$F {$^1$H} NMR** (376 MHz, MeOD) δ -100.61 (d, J = 249.4 Hz), -105.55 (d, J = 250.2, 4 Hz) ppm.

**HRMS** (ESI$^+$): $[M+H]^+$ Calcd. 554.3652; found 554.3656.

E. **Sodium 1-(2,2-difluoro-3β,7β-dihydroxy-5β-cholan-24-oyl)-piperidine-3-carboxylate (Compound 14)**

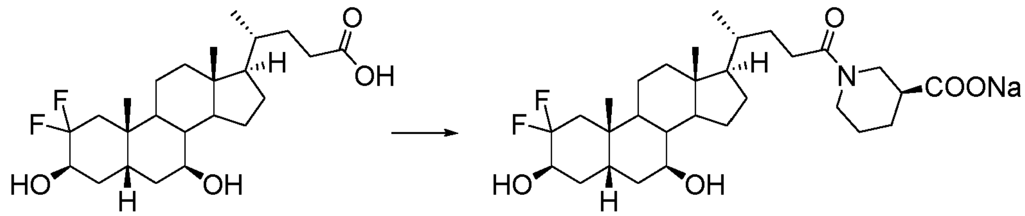

**Compound 7**

Using general procedure Q, 2,2-difluoro-3a,7a-dihydroxy-5β-cholanic acid (20.0 mg, 0.047 mmol) was conjugated to yield sodium 1-(2,2-difluoro-3β,7β-dihydroxy-5β-cholan-24-oyl)-piperidine-3-carboxylate as a residue (21.88 mg, 83%).

**$^1$H NMR** (400 MHz, MeOD) δ 3.78 (1H, br. s), 3.41 (1H, ddd, J = 14.8, 9.9, 5.2 Hz), 2.57-1.07 (33H, m), 1.05 (3H, s), 0.99 (3H, d, J = 6.5 Hz), 0.72 (3H, s) ppm.

**$^{19}$F NMR** (376 MHz, MeOD) δ -100.63 (d, J = 250.3 Hz), -105.55 (ddt, J = 249.8, 40.9, 7.8 Hz) ppm.

**HRMS** (ESI$^+$): $[M+H]^+$ Calcd. 540.3495; found 540.3507.

F. **Sodium 3-(2,2-difluoro-3β,7β-dihydroxy-5β-cholan-24-amide)-4-thiazolidine-carboxylate (Compound 15)**

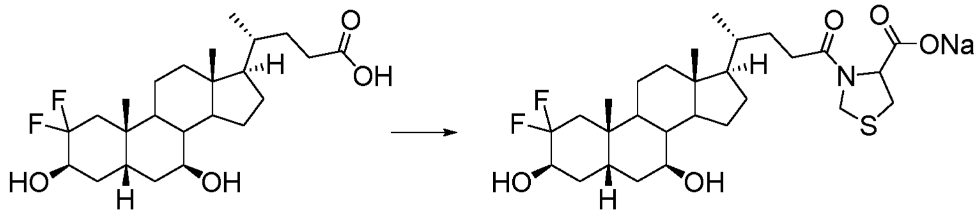

Compound 7

Using general procedure Q, 2,2-difluoro-3a,7a-dihydroxy-5β-cholanic acid (20.0 mg, 0.047 mmol) was conjugated to yield sodium 3-(2,2-difluoro-3β,7β-dihydroxy-5β-cholan-24-amide)-4-thiazolidine-carboxylate as a residue (21.41 mg, 81%).

**$^1$H NMR** (400 MHz, MeOD) δ 4.90-4.78 (0.4H, m), 4.77 (0.6H, d, J = 9.8 Hz), 4.71 (0.4H, d, J = 8.4 Hz), 4.60 (0.4H, d, J = 8.4 Hz), 4.59 (0.6H, m), 4.48 (0.6H, d, J = 9.8 Hz), 3.78 (1H, br. s), 3.46-3.15 (3H, m), 2.58-1.07 (24H, m), 1.04 (3H, br. s), 0.99 (1.2H, d, J = 6.5 Hz), 0.96 (1.8H, d, J = 6.5 Hz), 0.73 (1.2H, s), 0.71 (1.8H, s) ppm.

**$^{19}$F NMR** (376 MHz, MeOD) δ -100.63 (d, J = 249.1 Hz), -105.52 (ddt, J = 250.1, 41.4, 7.8 Hz) ppm.

**HRMS** (ESI$^+$): $[M+H]^+$ Calcd. 544.2903; found 544.2904.

G. ***N*-(2,2-difluoro-3β,7β-dihydroxy-5β-cholan-24-oyl)-morpholine (Compound 16)**

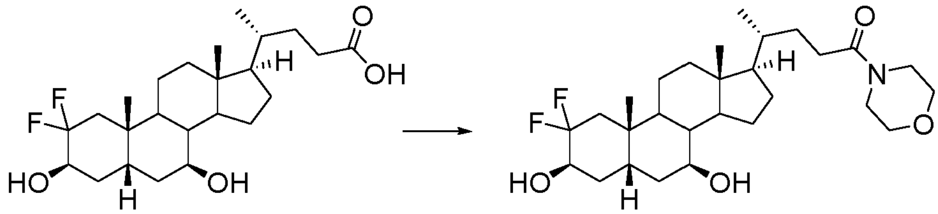

Compound 7

Using general procedure Q, 2,2-difluoro-3a,7a-dihydroxy-5β-cholanic acid (20.0 mg, 0.047 mmol) was conjugated to yield *N*-(2,2-difluoro-3β,7β-dihydroxy-5β-cholan-24-oyl)-morpholine as a clear residue (9.83 mg, 65%).

**$^1$H NMR** (400 MHz, MeOD) δ 3.78 (1H, br. s), 3.72-3.59 (4H, m), 3.59-3.50 (4H, m), 3.40 (1H, ddd, J = 14.8, 9.7, 5.2 Hz), 2.42 (1H, m), 2.30 (1H, m), 2.13-1.06 (22H, m), 1.05 (3H, s), 0.99 (3H, d, J = 6.5 Hz), 0.72 (3H, s) ppm.

**$^{19}$F NMR** (376 MHz, MeOD) δ -101.64 (d, J = 250.3 Hz), -105.55 (ddt, J = 249.6, 40.6, 7.8 Hz) ppm

**HRMS** (ESI$^+$): $[M+H]^+$ Calcd. 498.3389; found 498.3394.

H. **Sodium *N*-(2,2-difluoro-3β,7β-dihydroxy-5β-cholan-24-amide)-methylcarboxylic acid (Compound 17)**

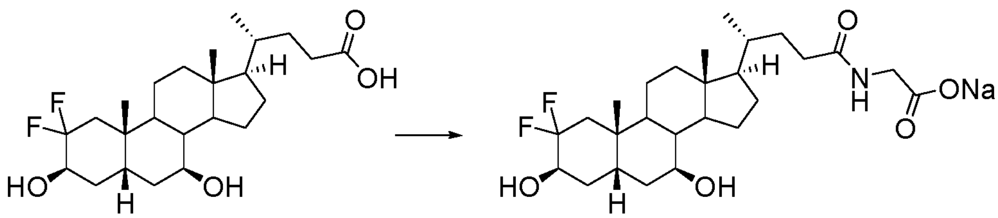

Compound 7

Using general procedure Q, 2,2-difluoro-3a,7a-dihydroxy-5β-cholanic acid (20.0 mg, 0.047 mmol) was conjugated to yield sodium *N*-(2,2-difluoro-3β,7β-dihydroxy-5β-cholan-24-amide)-methylcarboxylic acid as a clear residue (18.12 mg, 77%).

**$^1$H NMR** (400 MHz, MeOD) δ 3.78 (1H, br. s), 3.75 (2H, s), 3.40 (1H, ddd, J = 14.8, 9.7, 5.2 Hz), 2.30 (1H, m), 2.13-1.06 (23H, m), 1.05 (3H, s), 0.98 (3H, d, J = 6.4 Hz), 0.72 (3H, s) ppm.

**$^{19}$F NMR** (376 MHz, MeOD) δ -101.61 (d, *J* = 249.7 Hz), -105.55 (ddt, *J* = 249.6, 40.6, 7.8 Hz) ppm.

**HRMS** (ESI+): [M+H]+ Calcd. 486.3026; found 486.3029.

I. **Disodium *N*-(carboxymethyl)-*N*-(2,2-difluoro-3β,7β-dihydroxy-5β-cholan-24-oyl)-2-amino acetate (Compound 18)**

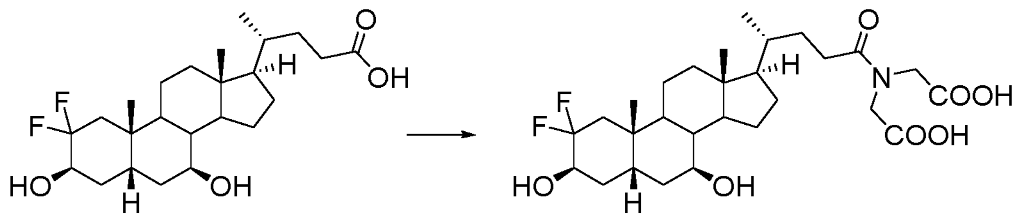

**Compound 7**

Using general procedure Q, 2,2-difluoro-3a,7a-dihydroxy-5β-cholanic acid (20.0 mg, 0.047 mmol) was conjugated to yield disodium *N*-(carboxymethyl)-*N*-(2,2-difluoro-3β,7β-dihydroxy-5β-cholan-24-oyl)-2-amino acetate as a clear residue (14.81 mg, 59%).
**1H NMR** (400 MHz, MeOD) δ 4.00 (4H, d, J = 19.4 Hz), 3.78 (1H, br. s), 3.40 (1H, ddd, J = 14.8, 9.7, 5.2 Hz), 2.42 (1H, m), 2.23 (1H, m), 2.14-1.07 (22H, m), 1.05 (3H, s), 0.96 (3H, d, J = 6.4 Hz), 0.71 (3H, s) ppm.
**19F NMR** (376 MHz, MeOD) δ -100.65 (d, J = 250.7 Hz), -105.55 (ddt, J = 249.6, 40.6, 7.8 Hz) ppm.
**HRMS** (ESI+): [M+H]+ Calcd. 544.3080; found 544.3081.

J. **Sodium *N*-(methyl)-*N*-(2,2-difluoro-3β,7β-dihydroxy-5β-cholan-24-amide) ethylsulfonic acid (Compound 19)**

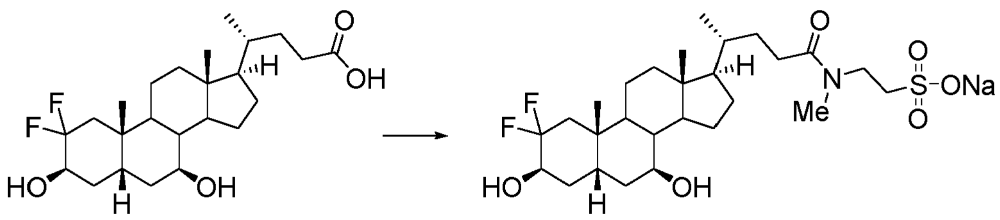

**Compound 7**

Using general procedure Q, 2,2-difluoro-3a,7a-dihydroxy-5β-cholanic acid (20.0 mg, 0.047 mmol) was conjugated to yield sodium *N*-(methyl)-*N*-(2,2-difluoro-3β,7β-dihydroxy-5β-cholan-24-amide)-ethylsulfonic acid as a clear residue (9.49 mg, 36%).
**1H NMR** (400 MHz, MeOD) δ 3.84-3.68 (3H, m), 3.40-3.36 (1H, m), 3.11 (1.5H, s) 3.08-2.99 (2H, m), 2.92 (1.5H, m), 2.57-1.07 (23H, m), 1.05 (3H, s), 0.99 (1.5H, d, J = 6.5 Hz), 0.98 (1.5H, d, J = 6.5 Hz), 0.72 (3H, s) ppm.
**19F NMR** (376 MHz, MeOD) δ -100.64 (d, J = 249.4 Hz), -105.54 (ddt, *J* = 250.3, 40.3, 7.8 Hz) ppm.
**HRMS** (ESI+): [M+Na]+ Calcd. 572.2828; found 572.2830.

K. **Sodium 3-(2,2-difluoro-3β,7β-dihydroxy-5β-cholan-24-oyl) amino-propanesulfonate (Compound 20)**

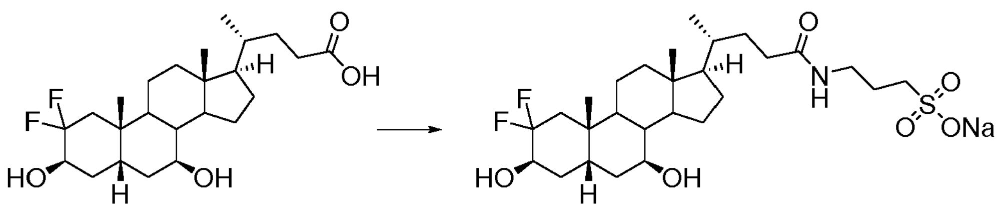

**Compound 7**

Using general procedure Q, 2,2-difluoro-3a,7a-dihydroxy-5β-cholanic acid (25.0 mg, 0.058 mmol) was conjugated to yield sodium 3-(2,2-difluoro-3β,7β-dihydroxy-5β-cholan-24-oyl) amino-propanesulfonate as a clear residue (25.44 mg, 76%).

$^{1}$**H NMR** (400 MHz, MeOD) δ 3.78 (1H, br. s), 3.40 (1H, ddd, J = 14.6, 9.7, 5.2 Hz), 3.33-3.25 (2H, m), 2.86-2.78 (2H, m), 2.24 (1H, m), 2.17-1.07 (25H, m), 1.05 (3H, s), 0.97 (3H, d, J = 6.5 Hz), 0.72 (3H, s) ppm.

$^{19}$**F NMR** (376 MHz, MeOD) δ -100.59 (d, J = 250.1 Hz), -105.55 (ddt, J = 249.4, 40.8, 7.5 Hz) ppm.

**HRMS** (ESI$^{+}$): $[M+Na]^{+}$ Calcd. 594.2647; found 594.2648.

L **Sodium *N*-(2,2-difluoro-3β,7β-dihydroxy-5β-cholan-24-amide) methanesulfonic acid (Compound 21)**

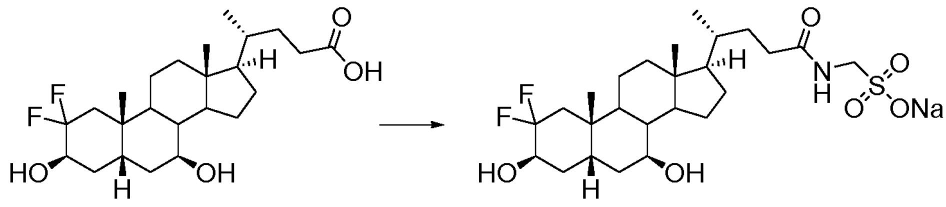

**Compound 7**

Using general procedure Q, 2,2-difluoro-3a,7a-dihydroxy-5β-cholanic acid (25.0 mg, 0.058 mmol) was conjugated to yield sodium *N*-(2,2-difluoro-3β,7β-dihydroxy-5β-cholan-24-amide) methanesulfonic acid as a clear residue (27.63 mg, 87%).

$^{1}$**H NMR** (400 MHz, MeOD) δ 4.32 (1H, d, J = 17.8 Hz), 4.28 (1H, d, J = 17.8 Hz), 3.78 (1H, br. s), 3.41 (1H, ddd, J = 14.6, 9.8, 5.1 Hz), 2.33 (1H, m), 2.25-1.07 (23H, m), 1.05 (3H, s), 0.98 (3H, d, J = 6.4 Hz), 0.72 (3H, s) ppm.

$^{19}$**F NMR** (376 MHz, MeOD) δ -100.61 (d, J = 250.8 Hz), -105.55 (ddt, J = 249.4, 40.8, 7.5 Hz) ppm.

**HRMS** (ESI$^{+}$): $[M-2H+D+2Na]^{+}$ Calcd. 567.2397; found 567.2387.

M. **Sodium *N*-(2,2-difluoro-3β,7β-dihydroxy-5β-cholan-24-oyl)-2-aminoethyl sulfate (Compound 22)**

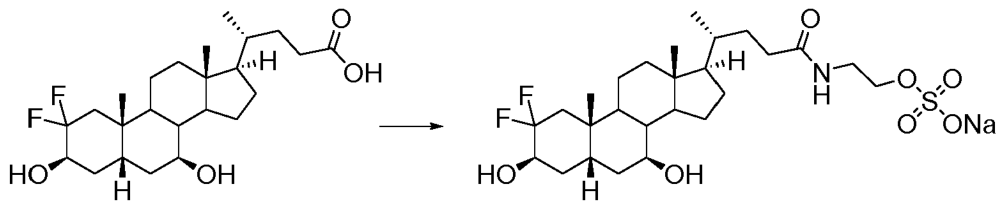

**Compound 7**

Using general procedure Q, 2,2-difluoro-3a,7a-dihydroxy-5β-cholanic acid (30.0 mg, 0.070 mmol) was conjugated to yield sodium *N*-(2,2-difluoro-3β,7β-dihydroxy-5β-cholan-24-oyl)-**H NMR** (400 MHz, MeOD) δ 4.04 (2H, app. t, J = 5.5 Hz), 3.78 (1H, br. s), 3.44 (2H, app. t, J = 5.5 Hz), 3.41 (1H, m), 2.26 (1H, ddd, J = 15.5, 10.5, 5.2 Hz), 2.18-1.06 (23H, m), 1.05 (3H, s), 0.98 (3H, d, J = 6.5 Hz), 0.71 (3H, s) ppm.

$^{19}$**F NMR** (376 MHz, MeOD) δ -100.59 (d, J = 250.0 Hz), -105.48 (ddt, J = 249.4, 40.8, 7.5 Hz) ppm.

**HRMS** (ESI$^{+}$): $[M+Na]^{+}$ Calcd. 574.2621; found 574.2620.

N. **Sodium *O*-(2,2-difluoro-3β,7β-dihydroxy-5β-cholan-24-oyl)-2-hydroxy ethyl sulfonic acid (Compound 23)**

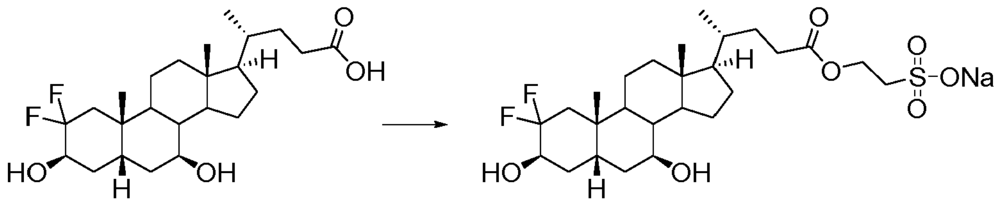

**Compound 7**

Using general procedure Q, 2,2-difluoro-3a,7a-dihydroxy-5β-cholanic acid (20.0 mg, 0.047 mmol) was conjugated to yield sodium *O*-(2,2-difluoro-3β,7β-dihydroxy-5β-cholan-24-oyl)-2-hydroxy ethyl sulfonic acid as a clear residue (15.06 mg, 58%).

**$^1$H NMR** (400 MHz, MeOD) δ 4.42 (2H, app. t, J = 7.0 Hz), 3.78 (1H, br. s), 3.41 (1H, ddd, J = 14.8, 9.8, 5.0 Hz), 3.12 (2H, app. t, J = 7.2 Hz), 2.39 (1H, m), 2.26 (1H, m), 2.14-1.06 (22H, m), 1.05 (3H, s), 0.95 (3H, d, J = 6.7 Hz), 0.71 (3H, s) ppm.

**$^{19}$F NMR** (376 MHz, MeOD) δ -100.65 (d, J = 250.5 Hz), -105.48 (ddt, J = 249.8, 40.9, 7.6 Hz) ppm.

**HRMS** (ESI$^+$): $[M+Na]^+$ Calcd. 581.2331; found 581.2332.

O. **Sodium *N*-(2,2-difluoro-3β,7β-dihydroxy-5β-cholan-24-oyl) aniline-2-sulfonic acid (Compound 24)**

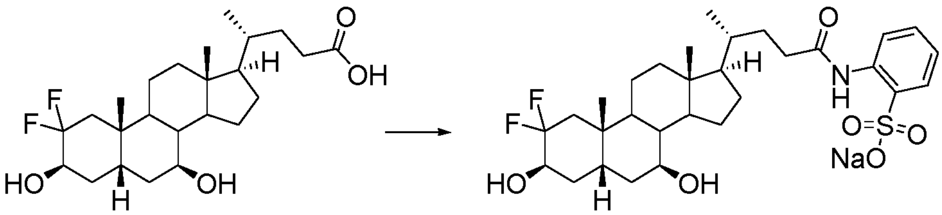

**Compound 7**

Using general procedure Q, 2,2-difluoro-3a,7a-dihydroxy-5β-cholanic acid (20.0 mg, 0.047 mmol) was conjugated to yield sodium *N*-(2,2-difluoro-3β,7β-dihydroxy-5β-cholan-24-oyl) aniline-2-sulfonic acid as a white residue (24.04 mg, 85%).

**$^1$H NMR** (400 MHz, MeOD) δ 8.30 (1H, d, J = 8.2 Hz), 7.86 (1H, dd, J = 7.9, 1.5 Hz), 7.40 (1H, m), 7.12 (1H, m), 3.79 (1H, br. s), 3.41 (1H, ddd, J = 14.8, 9.8, 5.0 Hz), 2.50 (1H, m), 2.34 (1H, m), 2.15-1.08 (22H, m), 1.05 (3H, s), 1.02 (3H, d, J = 6.3 Hz), 0.73 (3H, s) ppm. **$^{19}$F NMR** (376 MHz, MeOD) δ -100.62 (d, J = 251.2 Hz), -105.49 (ddt, J = 249.3, 40.6, 7.2 Hz) ppm.

**HRMS** (ESI$^+$): $[M-H+2Na]^+$ Calcd. 628.2491; found 628.2494.

P. **Sodium *N*-(cyclohexyl)-*N*-(2,2-difluoro-3β,7β-dihydroxy-5β-cholan-24-oyl)-3-amino-propanesulfonate (Compound 25)**

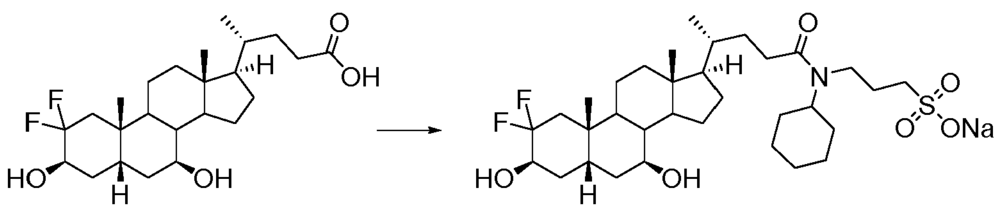

**Compound 7**

Using general procedure Q, 2,2-difluoro-3a,7a-dihydroxy-5β-cholanic acid (20.0 mg, 0.047 mmol) was conjugated to yield sodium *N*-(cyclohexyl)-*N*-(2,2-difluoro-3β,7β-dihydroxy-5β-cholan-24-oyl)-3-amino-propanesulfonate as a clear residue (27.97 mg, 92%).

**$^1$H NMR** (400 MHz, MeOD) δ 4.17 (0.4H, m), 3.78 (1H, br. s), 3.62 (0.6H, m), 3.45-3.33 (3H, m), 2.87-2.75 (2H, m),

2.41 (1H, m), 2.30 (1H, m), 2.14-1.07 (34H, m), 1.05 (3H, br. s), 1.02-0.97 (3H, m), 0.73 (1.8H, s), 0.72 (1.2H, s) ppm.

**$^{19}$F NMR** (376 MHz, MeOD) δ -100.60 (d, J = 249.8 Hz), -105.48 (ddt, J = 250.1, 41.0, 7.6 Hz) ppm.

**HRMS** (ESI$^+$): $[M+Na]^+$ Calcd. 654.3610; found 654.3606.

Q. **Sodium *N*-(cyclohexyl)-*N*-(2,2-difluoro-3β,7β-dihydroxy-5β-cholan-24-oyl)-2-amino-ethanesulfonate (Compound 26)**

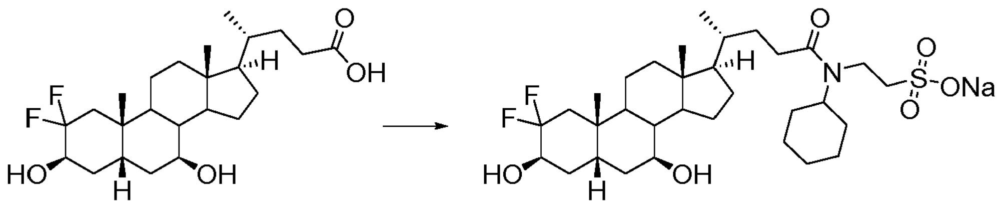

**Compound 7**

Using general procedure Q, 2,2-difluoro-3a,7a-dihydroxy-5β-cholanic acid (20.0 mg, 0.047 mmol) was conjugated to yield sodium *N*-(cyclohexyl)-*N*-(2,2-difluoro-3β,7β-dihydroxy-5β-cholan-24-oyl)-2-amino-ethanesulfonate as a clear residue (28.41 mg, 95%).

**$^1$H NMR** (400 MHz, MeOD) δ 3.78 (1H, br. s), 3.73-3.56 (3H, m), 3.40 (1H, ddd, J = 14.7, 9.7, 5.0 Hz), 3.09-2.96 (2H, m), 2.44 (1H, m), 2.31 (1H, m), 2.15-1.07 (32H, m), 1.05 (3H, br. s), 1.02-0.97 (3H, m), 0.73 (1.8H, s), 0.72 (1.2H, s) ppm.

**$^{19}$F NMR** (376 MHz, MeOD) δ -100.57 (d, J = 251.0 Hz), -105.49 (ddt, J = 250.3, 40.9, 7.0 Hz) ppm.

**HRMS** (ESI$^+$): $[M-H+2Na]^+$ Calcd. 662.3273; found 662.3285.

R. ***N*-(2,2-difluoro-3β,7β-dihydroxy-5β-cholan-24-oyl) 2-aminoethyl methyl sulfone (Compound 27)**

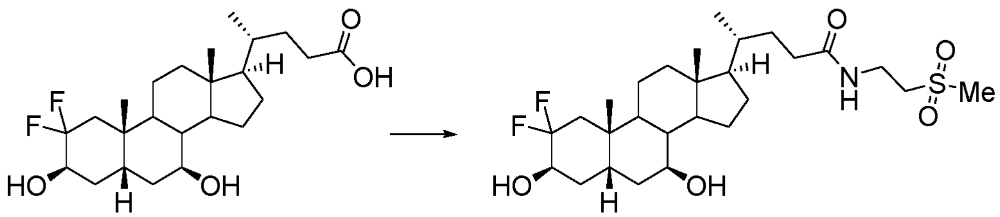

**Compound 7**

Using general procedure Q, 2,2-difluoro-3a,7a-dihydroxy-5β-cholanic acid (20.0 mg, 0.047 mmol) was conjugated to yield *N*-(2,2-difluoro-3β,7β-dihydroxy-5β-cholan-24-oyl) 2-aminoethyl methyl sulfone as a clear residue (22.49 mg, 90%).

**$^1$H NMR** (400 MHz, MeOD) δ 3.78 (1H, br. s), 3.62 (2H, app. t, J = 6.7 Hz), 3.40 (1H, ddd, J = 14.7, 9.6, 4.9 Hz), 3.29 (2H, app. t, J = 6.5 Hz), 2.99 (3H, s), 2.26 (1H, ddd, J = 15.3, 10.4, 5.3 Hz), 2.17-1.06 (23H, m), 1.05 (3H, s), 0.97 (3H, d, J = 6.4 Hz), 0.71 (3H, s) ppm. **$^{19}$F NMR** (376 MHz, MeOD) δ -100.60 (d, J = 249.7 Hz), -105.49 (ddt, J = 249.9, 41.2, 7.1 Hz) ppm.

**HRMS** (ESI$^+$): $[M+Na]^+$ Calcd. 556.2879; found 556.2873.

S. ***N*-(ethyl)-*N*-(2,2-difluoro-3β,7β-dihydroxy-5β-cholan-24-oyl)-3-amino-tetrahydrothiophene dioxide (Compound 28)**

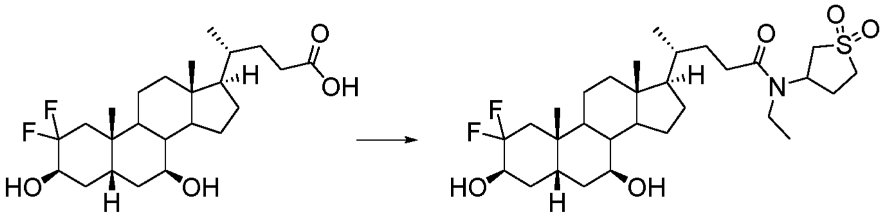

Compound 7

Using general procedure Q, 2,2-difluoro-3a,7a-dihydroxy-5β-cholanic acid (20.0 mg, 0.047 mmol) was conjugated to yield *N*-(ethyl)-*N*-(2,2-difluoro-3β,7β-dihydroxy-5β-cholan-24-oyl)-3-amino-tetrahydrothiophene dioxide as a clear residue (20.71 mg, 77%).

**$^1$H NMR** (400 MHz, MeOD) δ 4.56 (1H, m), 3.78 (1H, br. s), 3.51 (4H, m), 3.33-3.21 (2H, m), 3.08 (1H, m), 2.63-1.06 (29H, m), 1.05 (3H, s), 0.99 (3H, d, J = 6.5 Hz), 0.72 (3H, s) ppm.

**$^{19}$F NMR** (376 MHz, MeOD) δ -100.59 (d, J = 250.4 Hz), -105.48 (ddt, J = 250.4, 41.5, 7.4 Hz) ppm.

**HRMS** (ESI$^+$): $[M+H]^+$ Calcd. 574.3372; found 574.3370.

T. ***N*-(2-(diisopropylamino)ethyl)-*N*-(2,2-difluoro-3β,7β-dihydroxy-5β-cholan-24-oyl)-3-amino-tetrahydrothiophene dioxide (Compound 29)**

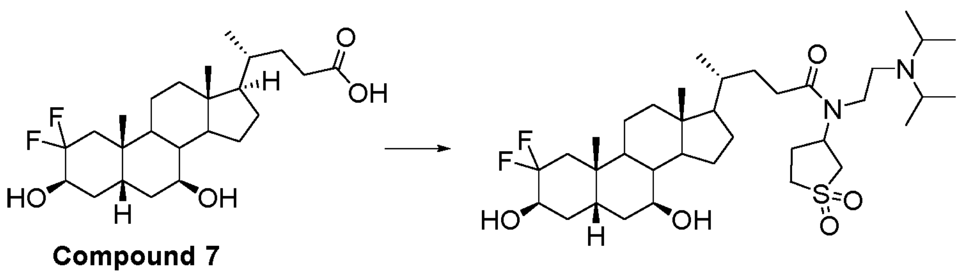

Compound 7

Using general procedure Q, 2,2-difluoro-3a,7a-dihydroxy-5β-cholanic acid (20.0 mg, 0.047 mmol) was conjugated to yield *N*-(2-(diisopropylamino)ethyl)-*N*-(2,2-difluoro-3β,7β-dihydroxy-5p-cholan-24-oyl)-3-amino-tetrahydrothiophene dioxide as a residue (23.90 mg, 76%).

**$^1$H NMR** (400 MHz, MeOD) δ 3.78 (1H, br. s), 3.50-0.88 (m), 0.72 (3H, s) ppm.

**$^{19}$F NMR** (376 MHz, MeOD) δ -100.56 (d, J = 250.5 Hz), -105.49 (m) ppm.

**HRMS** (ESI$^+$): $[M+H]^+$ Calcd. 673.4420; found 673.4430.

U. **N-(2,2-difluoro-3β,7β-dihydroxy-5β-cholan-24-oyl)-thiomorpholine-dioxide (Compound 30)**

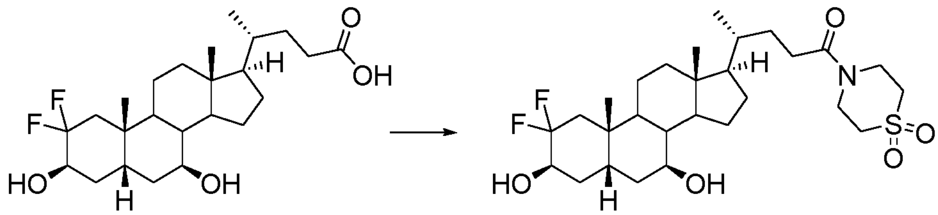

Compound 7

Using general procedure Q, 2,2-difluoro-3a,7a-dihydroxy-5β-cholanic acid (20.0 mg, 0.047 mmol) was conjugated to yield *N*-(2,2-difluoro-3β,7β-dihydroxy-5β-cholan-24-oyl)-thiomorpholine-dioxide as a clear residue (22.2 mg, 87%).

**$^1$H NMR** (400 MHz, MeOD) δ 4.09-3.93 (4H, m), 3.78 (1H, br. s), 3.40 (1H, ddd, J = 14.6, 9.6, 5.1 Hz), 3.23-3.04 (4H, m), 2.52 (1H, m), 2.38 (1H, m), 2.14-1.07 (22H, m), 1.05 (3H, s), 0.99 (3H, d, J = 6.6 Hz), 0.73 (3H, s) ppm.

**$^{19}$F NMR** (376 MHz, MeOD) δ -100.63 (d, J = 249.3 Hz), -105.54 (ddt, J = 250.6, 41.6, 7.5 Hz) ppm.
**HRMS** (ESI$^+$): $[M+H]^+$ Calcd. 546.3059; found 546.3065.

V. ***N*-(2,2-difluoro-3β,7β-dihydroxy-5β-cholan-24-oyl) 1,1-dioxidotetrahydro-2H-thiopyran-3-ylamine (Compound 31)**

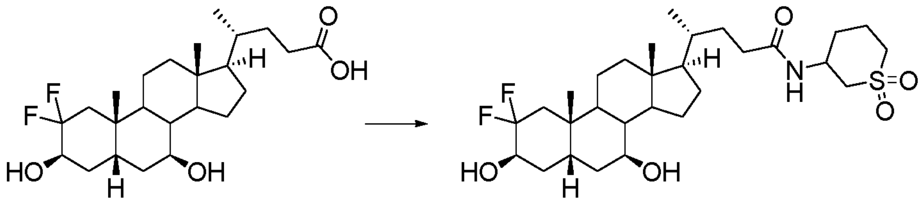

**Compound 7**

Using general procedure Q, 2,2-difluoro-3a,7a-dihydroxy-5β-cholanic acid (20.0 mg, 0.047 mmol) was conjugated to yield *N*-(2,2-difluoro-3β,7β-dihydroxy-5β-cholan-24-oyl) 1,1-dioxidotetrahydro-2H-thiopyran-3-ylamine as a residue (22.44 mg, 88%).
**$^1$H NMR** (400 MHz, MeOD) δ 4.26 (1H, tt, J = 11.1, 3.6 Hz), 3.78 (1H, br. s), 3.40 (1H, ddd, J = 14.5, 10.0, 5.1 Hz), 3.27 (1H, m), 3.08-2.91 (3H, m), 2.30-1.06 (26H, m), 1.05 (3H, s), 0.97 (3H, d, J = 6.5 Hz), 0.71 (3H, s) ppm.
**$^{19}$F NMR** (376 MHz, MeOD) δ -100.63 (d, J = 250.3 Hz), -105.49 (ddt, J = 250.7, 41.5, 7.9 Hz) ppm.
**HRMS** (ESI$^+$): $[M+H]^+$ Calcd. 560.3216; found 560.3217.

**Comparative Example 5** - **Synthesis of 2-fluoroalkene compounds**

**A. Methyl 2-fluoro-3β,7β-dihydroxy-5β-chol-1-enoate (4A.1)**

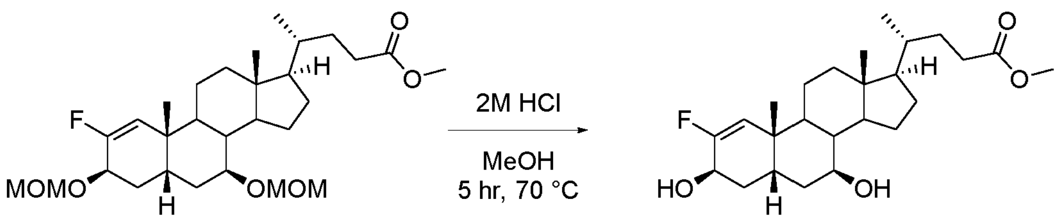


**3J.2**

**4A1** (quantitative)

Using general procedure L, methyl 2-fluoro-3β,7β-dimethoxymethoxyl-5β-chol-1-enoate from Example 4J (900 mg, 1.76 mmol, 1 equiv) was deprotected to yield methyl 2-fluoro-3β,7β-dihydroxy-5β-chol-1-enoate as a white gummy solid (750 mg, quantitative yield).
**$^1$H NMR** (400MHz, $CDCl_3$): δ 5.34 (1H, d, J = 17.6 Hz), 4.20 (1H, ddd, J = 7.7, 4.7, 1.3 Hz), 3.67 (3H, s), 3.50 (1H, ddd, J = 11.2, 9.6, 4.8 Hz), 2.35 (1H, ddd, J = 15.6, 10.5, 5.0 Hz), 2.22 (1H, ddd, J = 15.6, 9.4, 6.5 Hz), 2.12 (1H, td, J = 14.0, 5.3 Hz), 2.04-1.21 (23H, m), 1.12 (3H, d, J = 0.7 Hz), 0.92 (3H, d, J = 6.5 Hz), 0.70 (3H, s) ppm.
**$^{19}$F NMR** (376MHz, $CDCl_3$): δ -117.65 (dt, J = 17.0, 8.5 Hz) ppm.
**LRMS** (ESI$^+$) m/z : 405.5 $[M+H-H_2O]^+$, 387.5 $[M+H-2H_2O]^+$.

B. **Methyl 2-fluoro-3,7-di-oxo-5β-chol-1-enoate (4B.1)**

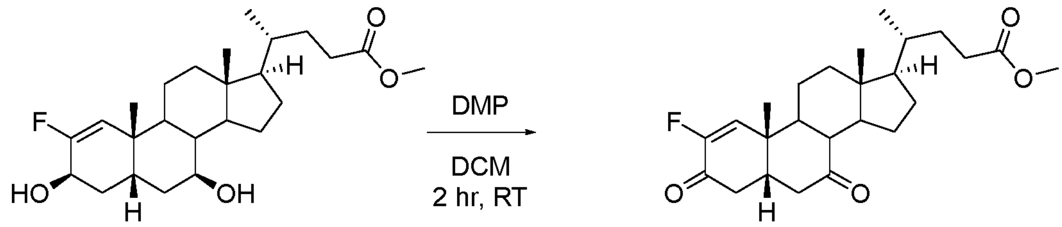


**4A.1** **4B.1** (67%)

Using general procedure M, methyl 2-fluoro-3β,7β-dihydroxy-5β-chol-1-enoate (600 mg, 1.42 mmol, 1 equiv) was oxidised, then purified *via* flash chromatography (Petrol ether/EtOAc : 80:20→70:30→60/40) to yield methyl 2-fluoro-3,7-di-oxo-5β-chol-1-enoate as a gummy solid (400 mg, 0.96 mmol, 67%).

**$^1$H NMR** (400MHz, $CDCl_3$): δ 6.34 (1H, d, J = 14.7 Hz), 3.66 (3H, s), 2.89 (1H, dd, J = 12.8, 5.9 Hz), 2.61 (1H, dtd, J = 13.5, 5.8, 2.0 Hz), 2.55-2.11 (7H, m), 2.07 (1H, dd, J = 13.2, 2.0 Hz), 2.04-1.71 (5H, m), 1.51 (3H, s), 1.49-0.94 (8H, m), 0.92 3H, (d, J = 6.4 Hz), 0.70 (3H, s) ppm.

**$^{19}$F NMR** (376MHz, $CDCl_3$): δ -131.67 (dd, J = 15.6, 3.5 Hz), ppm.

**LRMS** (ESI+) m/z : 419.5 $[M+H]^+$, 460.5 $[M+H+MeCN]^+$.

C. **Methyl 2-fluoro-3α,7α-dihydroxy-5β-chol-1-enoate (4C.1)**

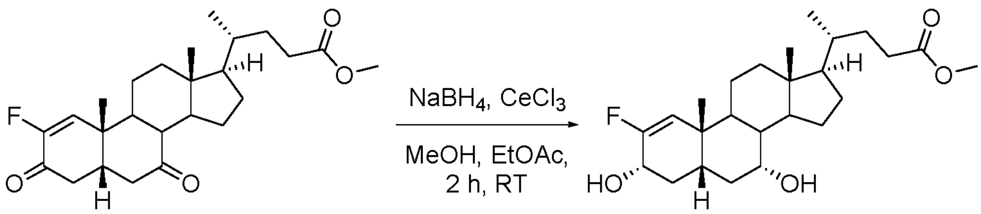


**4B.1** **4C.1** (25%)

Using general procedure B, methyl 2-fluoro-3β,7β-dihydroxy-5β-chol-1-enoate (400 mg, 0.96 mmol, 1 equiv) was reduced, then purified *via* flash chromatography (Petrol ether/EtOAc : 70:30→60:40→50:50) to yield methyl 2-fluoro-3α,7α-dihydroxy-5β-chol-1-enoate as a gummy solid (99 mg, 0.22 mmol, 25%).

**$^1$H NMR** (400MHz, $CDCl_3$): δ 5.25 (1H, d, J = 18.2 Hz), 4.35 (1H, t, J = 7.9 Hz), 3.85 (1H, q, J = 1.7 Hz), 3.67 (3H, s), 3.65 (1H, d, J = 1.8 Hz), 2.53-2.32 (2H, m), 2.29-2.17 (1H, m), 2.04-1.07 (29H, m), 1.04 (3H, d, J = 0.9 Hz), 0.93 (3H, d, J = 6.4 Hz), 0.68 (3H, s) ppm. **$^{19}$F NMR** (376MHz, $CDCl_3$): δ -125.36 (dd, J = 19.1, 6.9 Hz) ppm.

**LRMS** ($ESI^+$) m/z : 405.5 $[M+H-H_2O]^+$, 387.5 $[M+H-2H_2O]^+$.

D **2-fluoro-3β,7β-dihydroxy-5β-chol-1-enic acid (Comparative Compound F)**

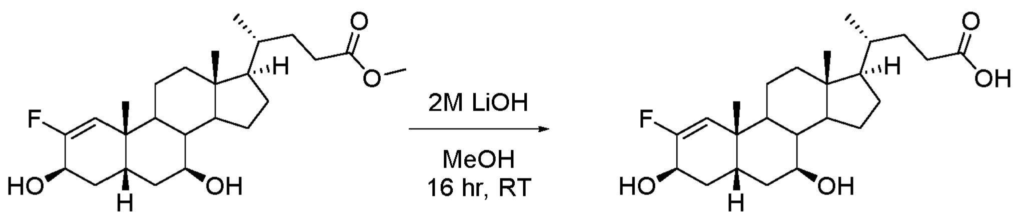


**4A.1** **Compound F** (70%)

Using general procedure C, methyl 2-fluoro-3β,7β-dihydroxy-5β-chol-1-enoate from step A (60 mg, 0.14 mmol, 1 equiv) was hydrolysed to yield 2-fluoro-3β,7β-dihydroxy-5β-chol-1-enic acid (Compound F) as a white solid (40 mg, 0.10 mmol, 70%).

**$^1$H NMR** (400MHz, $CD_3OD$): δ 5.32 (1H, d, J = 17.7 Hz), 4.10 (1H, ddd, J = 8.0, 4.5, 1.0 Hz), 3.38 (1H, td, J = 10.5, 4.8 Hz), 2.40-1.15 (29H, m), 1.13 (3H, s), 0.95 (3H, d, J = 6.5 Hz), 0.73 (3H, s) ppm.

**$^{19}$F NMR** (376MHz, $CD_3OD$): δ -117.71 (dt, J = 15.6, 7.6 Hz) ppm.

**LRMS** ($ESI^+$) m/z : 391.5 $[M+H-H_2O]^+$, 373.5 $[M+H-2H_2O]^+$.

E. **2-fluoro-3,7-dioxo-5β-chol-1-enic acid (Comparative Compound G)**

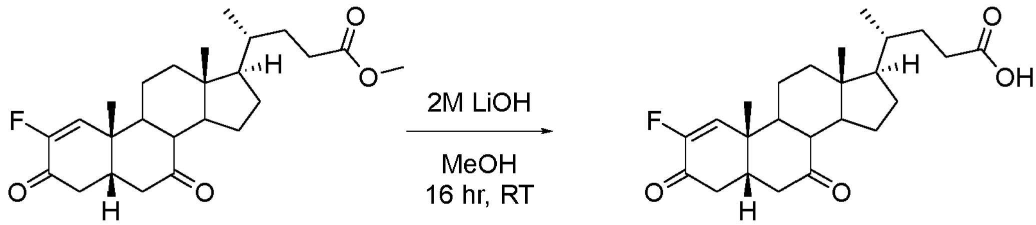


**4B.1** **Compound G** (93%)

Using general procedure C, methyl 2-fluoro-3,7-di-oxo-5β-chol-1-enoate from step B (50 mg, 0.12 mmol, 1 equiv) was hydrolysed to yield 2-fluoro-3,7-dioxo-5β-chol-1-enic acid **(Compound G)** as a white solid (45 mg, 0.11 mmol, 93%).

**$^1$H NMR** (400MHz, $CDCl_3$): δ 6.34 (1H, d, J = 14.7 Hz), 2.89 (1H, dd, J = 13.0, 6.1 Hz), 2.62 (1H, dtd, J = 13.5, 5.8, 2.0 Hz), 2.55-1.73 (13H, m), 1.51 (3H, s), 1.49-0.95 (9H, m), 0.93 (3H, d, J = 6.4 Hz), 0.70 (3H, s) ppm.

**$^{19}$F NMR** (376MHz, $CDCl_3$): δ -131.63 (dd, J = 13.9, 3.5 Hz) ppm.

**LRMS** ($ESI^+$) m/z : 405.4 $[M+H]^+$, 446.5 $[M+H+MeCN]^+$.

F. **2-fluoro-3α,7α-dihydroxy-5β-chol-1-enic acid (Comparative Compound H)**

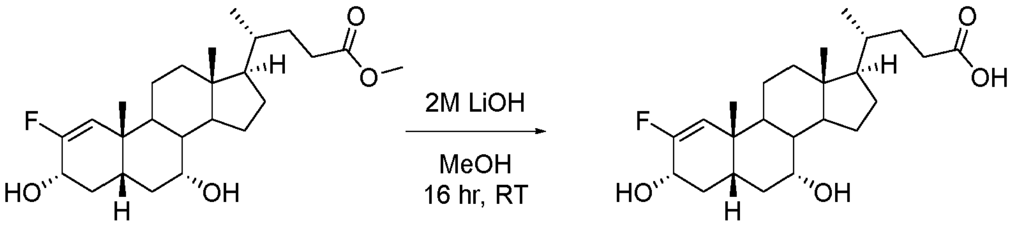


**4C.1** **Compound H** (97%)

Using general procedure C, methyl 2-fluoro-3α,7α-dihydroxy-5β-chol-1-enoate from step C (50 mg, 0.11 mmol, 1 equiv) was hydrolysed to yield 2-fluoro-3α,7α-dihydroxy-5β-chol-1-enic acid **(Compound H)** as a pale solid (45 mg, 0.11 mmol, 97%).

**$^1$H NMR** (400MHz, Acetone-$D_6$): δ 5.16 (1H, d, J = 18.6 Hz), 4.23 (1H, ddd, J = 9.0, 7.0, 2.5 Hz), 4.07 (1H, br. s), 3.82 (1H, q, J =2.7 Hz), 3.32 (1H, br. s), 2.54 (1H, td, J = 13.7, 10.0 Hz), 2.34 (1H, ddd, J = 15.5, 11.0, 5.0 Hz), 2.21 (1H, ddd, J = 15.6, 9.4, 6.5 Hz), 2.02-1.06 (25H, m), 1.05 (3H, d, J = 1.0 Hz), 0.96 (3H, d, J = 6.6 Hz), 0.71 (3H, s) ppm.

**$^{19}$F NMR** (376MHz, Acetone-$D_6$): δ -123.32 (dd, J = 19.1, 6.9 Hz) ppm.

**LRMS** ($ESI^+$) m/z : 373.5 $[M+H-2H_2O]^+$.

**BIOLOGICAL EXAMPLES**

Abbreviations

| | |
|---|---|
| BDNF | Brain-derived neurotrophic factor |
| Cy3 | Cyanine 3 |
| DAPI | 4',6-diamidino-2-phenylindole |
| DAT | Anti-dopamine transporter antibody |
| dcAMP | Dibutyryl cyclic adenosine monophosphate |

(continued)

| | |
|---|---|
| DMEM | Dulbecco Modified Eagle Medium |
| EDTA | Ethylene diamine tetra-acetic acid |
| FGFb | Basic fibroblast growth factor |
| FGF8 | Fibroblast growth factor 8 |
| GDNF | Glial cell line-derived neurotrophic factor |
| HBSS | Hanks' balanced salt solution |
| HEPES | 4-(2-hydroxyethyl)-1-piperazineethanesulfonic acid |
| iNPC | Induced neural progentitor cell |
| MEM | Minimum essential medium |
| MMP | Mitochondrial membrane potential |
| MPTP | 1-methyl-4-phenyl-1,2,3,6-tetrahydropyridine |
| NEEA | Non-essential amino Acid cell culture supplement |
| PBS | Phosphate buffered saline |
| PD | Parkinson's disease (sPD is sporadic Parkinson's disease) |
| TGF-b3 | Transforming growth factor geta-3 |
| TMRM | Tetramethylrhodamine methyl ester perchlorate |
| Tuj | Anti-beta-tubulin III antibody |

**Example 6**

A. **Culture of primary fibroblasts, generation and culture of iNPC's.**

Fibroblasts were cultured in DMEM (Invitrogen) and routinely subcultured every 3-5 days using trypsin to dissociate them. Induced neural progenitor cells (iNPC's) were generated as previously described (Meyer et al, "Direct conversion of patient fibroblasts demonstrates non-cell autonomous toxicity of astrocytes to motor neurons in familial and sporadic ALS" Proc Natl Acad Sci USA 2014). iNPC's were maintained in DMEM/Ham F12 (Invitrogen); N2, B27 supplements (Invitrogen) and FGFb (Peprotech) in fibronectin (Millipore) coated tissue culture dishes and routinely subcultured every 2-3 days using accutase to detach them.

B. **Dopaminergic neuron differentiation of iNPC's**

Briefly, iNPCs are plated in a 6-well plate and cultured for 2 days in DMEM/F-12 medium with Glutamax™ supplemented with 1% NEAA, 2%B27 (Gibco) and 2.5$\mu$M of DAPT. On day 3, DAPT is removed and the medium is supplemented with 1$\mu$M smoothened agonist (SAG) and FGF8 (75ng/ml) for additional 10 days. Neurons are replated at this stage. Subsequently SAG and FGF8 are withdrawn and replaced with BDNF (30 ng/ml), GDNF (30 ng/ml), TGF-b3 (2 mM) and dcAMP (2 mM, Sigma) for 15 days.

C. **Immunofluorescence staining and ELISA**

Cells are plated into 96 well plates and fixed using 4% paraformaldehyde for 30 minutes. After PBS washes cells are permeabilised using 0.1% Triton™ X-100 for 10 minutes and blocked using 5% goat serum for 1 hour. Cells are incubated with primary antibodies tyrosine hydroxylase (St John's Laboratory); DAT (Abcam); Tuj (Millipore); Tom20 (BD Biosciences); activated caspase 3 (Cell Signaling); alpha synuclein (Cell Signaling); phosphorylated alpha synuclein (Millipore) at 4 °C for 16 hours. Cells are washed using PBS-Tween® and incubated with Alexa Fluor™-conjugated secondary antibodies 488 and 568 (Invitrogen) and Hoescht (Sigma) 1$\mu$M prior to imaging. Imaging was performed using the Opera Phenix™ high content imaging system (Perkin Elmer).

Dopamine ELISA is performed using Dopamine research ELISA kit (Labor Diagnostika Nord GmbH&Co. KG) as per manufacturers instructions. Dopamine release is obtained incubating the cells at 37°C using three different conditions at the same time per line. Medium is removed in all wells then the first well is incubated with HBSS with $Ca^{2+}$ and $Mg^{2+}$

(Gibco by Life Technologies) for 30 minutes, the second well is incubated in HBSS with $Ca^{2+}$ and $Mg^{2+}$ for 15 minutes and then 56mM KCl (Fisher chemical) is added for another 15 minutes and the third well is incubated with HBSS without $Ca^{2+}$ and $Mg^{2+}$ (Gibco by Life Technologies) but with 2mM EDTA for 15min and then 56mM KCl is added for another 15 minutes. Straight away media is collected in an eppendorf and cells are harvested using Accutase®, centrifuge at 400g for 4 min and resuspended in 10μl of PBS. EDTA 1mM and Sodium Metabisulfite (Sigma) 4mM are added to both the media and pellet to preserve the dopamine.

MMP protocol

Fibroblasts were cultured and plated into a Griener black 384 μClear® plate at 10000 cells per well in 50μl of media volume. The plates are left overnight in an incubator to allow the fibroblasts to adhere to the plate surface. The following morning the Glucose based medium is replaced with 25μl of Galactose based media. The plates were then dosed with the compounds using an ECHO® 550 liquid handling system. The wells were dosed to provide an 8-point concentration range of 0.06nM-300nM of compound. After dosing the wells are topped up with a further 25μl of Galactose based medium and then left in an incubator for 24 hours. After 24 hours, the medium is removed from the wells and replaced with 25ul phenol free Minimal essential mediuim with 100nM TMRM (Sigma) and 10μM Hoechst Stain (Sigma). The plate is returned to the incubator for another hour after which the stain medium is removed and replaced with 25ul Phenol free MEM. The plate is then imaged using an IN Cell high content microscope (GE Healthcare) with 10 fields of view per well in 2 channels, Cy3 excitation 542nm, emission 604-64nm; and the DAPI excitation 350nm, emission 450-55nm at 37 °C with $CO_2$. After imaging the plate is disposed of and the images are Data mined using the INCell developer Toolkit (GE Healthcare).

ATP Protocol

The ATP protocol is generally as described in Mortiboys et al, "Mitochondrial function and morphology are impaired in parkin-mutant fibroblasts", Ann Neurol. 2008 Nov; 64(5):555-65. Briefly, fibroblasts were cultured as and plated into white 384 well plates with 5000 cells per well in 50 μl of media volume. The plates are left overnight in an incubator to allow the fibroblasts to adhere to the plate surface. The following morning the Glucose based mediuim is replaced with 25μl of Galactose based mediuim. The compounds are added to the plates using a ECHO 550 liquid handling system. The wells were dosed to provide an 8-point concentration range of 0.06nM-300nM of compound. After dosing the wells are topped up with a further 25μl of Galactose based medium and then left in an incubator for 24 hours. Following this incubation the medium is removed from the plate and the wells are washed twice with sterile PBS. The wells are filled with 25μl of Sterile PBS followed by 12.5μl of Lysis solution from the ATPlite™ Luminescence ATP detection assay system (Perkin Elmer), including 16 cell free wells to use as blank controls. The plate is then placed on a rotary shaker for 5 mins at 700 rpm. Following the shaking 12.5μl of ATP substrate solution (Perkin Elmer) is added to each well and a further 5 min of shaking. The plate is then placed in darkness for 10 minutes prior to reading. Using a PHERAStar® plate reader, luminescence intensity is recorded. Following the ATP assay the plates are immediately assayed for DNA content in a CyQUANT® assay.

Immediately following The ATP assay DNA content is assessed with the CyQUANT® NF Cell Proliferation Assay Kit (ThermoFisher). CyQUANT® buffer is prepared immediately before the assay and is comprised of 1ul CyQUANT® dye per ml x1 HBSS solution. 12.5 ul of CyQUANT® buffer is added to each well. Plate left in incubator for 1 hour then read on a PHERAStar® Plate reader with excitation at 497nm and emission at 520nm.

ATP Quantification for each well is determined using the following formula:

$$ATP\ score = \frac{ATPlite\ blank\ corrected\ value}{CyQUANT\ blank\ corrected\ value}$$

Data analysis for primary screen assays.

After the assays had been repeated in triplicate per line and compound the data was then inputted into Graph pad Prism 7 software suite where a dose response curve is generated using the default "[Agonist] vs response(three parameters)" equation.

$$y = Bottom + x \times \frac{Top - Bottom}{EC50 + x}$$

From this $EC_{50}$ values, lowest response and maximal response were taken and used to calculate the Geometric mean between the 5 different lines assessed.

Results derived from compounds that showed an Ambiguous result from the "[Agonist] vs response (three parameters)" equation were excluded from the Geometric mean calculations due to the high skew that was introduced by their inclusion.

Seahorse Assay

Fibroblasts are plated into Seahorse 24 well plates with 50,000 cells per well. Cells are left to attach for 2 days. Media is changed to Seahorse DMEM media with glucose and sodium pyruvate and left to equilibrate at 37 degrees normal air $CO_2$ for 1 hour. The plate is entered into the Seahorse machine and run on a program of mix (2 minutes), wait (3 minutes) and measure (3 minutes). After three measurements of basal respiration and ECAR; 0.5μM oligomycin is injected after which another three measurements are taken; then 0.5μM of FCCP is injected and three measurements taken and finally 1μM rotenone is injected and three measurements taken. After all measurements are complete, cells are stained with 10μM Hoescht and imaged using the InCell to count the number of cells per well for normalisation. This classical mitochondrial stress test experimental protocol allows us to calculate the basal mitochondrial respiration, ATP linked respiration (the amount which is coupled to ATP generation), the maximal and spare respiratory capacity, the non-mitochondrial respiration rate and the extracellular acidification rate which is a proxy measure of glycosolysis.

Complex I assay

*Ex vivo* mice brain was homogenated in a buffer of 250 mM sucrose, 20 mM HEPES, 3 mM EDTA, pH 7.5 at 4°C. Homogenisation was carried out using a Dounce homogenizer, for cortex samples, and by repetitive passage through a 0.5mm syringe for isolated striatum. Samples were then incubated with 30μl of detergent from the AbCam colorimetric Complex I assay kit on ice for 20 minutes. Samples are then centrifuged at 13,000 rpm for 30 mins. Triplicate samples per condition were blocked using the kit blocking buffer on the AbCam colorimetric Complex I assay kit plate for 3 hours. Samples are then washed using the kit wash buffer 3 times before the addition of the kit assay buffer containing NADH and colorimetric dye. The assay plate is read on a plate reader in a kinetic assay programme reading 450nm in a 30 second interval for 50 minutes.

D. **Mitochondrial function and morphology measurements in iNeurons**

Cells are plated in 96 well plates; for live imaging cells are incubated for one hour at 37degree with 80nM tetramethlyrhodamine (TMRM), 1μM LysoTracker® Green (Invitrogen) and Hoechst Stain solution (Sigma) at 1μM before imaging using Opera Phenix™. Cellular ATP measurements are undertaken using ATPlite kit (Perkin Elmer) as per manufacturer's instructions. Mitochondrial reactive oxygen species generation was assessed using mitochondrial NpFR2 (probe; a kind gift from Dr Liz New, University of Sydney, Australia) at 20μM and Hoechst stain solution at 1μM for 30mins at 37 °C, then the dyes are removed and cells images using Opera Phenix™. Images generated from the live imaging experiments were analysed using Harmony® (Perkin Elmer software). We developed protocols in order to segment nucleus, cell boundary and processes, mitochondria, lysosomes, autophagosomes. We only analysed the z projection images collected from the z stacks.

**Results**

**Fibroblasts**

The mitochondrial membrane potential was measured in fibroblasts from 6 (Table 1) or 3

(Table 2) patients with sporadic Parkinson's disease when treated with Compounds of the invention or Comparator compounds. The results are shown in Tables 1 and 2, where "Bottom" = max response with lowest dose of compound (0.06nM) and "top" = max response with highest dose of compound (300nM).

Table 1 - Mitochondrial Membrane Potential data from 6 sporadic PD patient fibroblasts

| Compound | E | F | G | H | 2 | 7 | 9 |
|---|---|---|---|---|---|---|---|
| **Bottom (% of vehicle treated)** | 104.9 | 110 | 103.4 | 99.83 | 107.3 | 100.4 | 109.8 |
| **Top (% of vehicle treated)** | 111.8 | 108.1 | 123.3 | 109.1 | 112 | 114 | 112.4 |
| **$EC_{50}$ (nM)** | 23.29 | 0.6738 | 282.1 | 4.581 | 20.82 | 4.066 | 2.186 |

Table 2 - Mitochondrial Membrane Potential data from 3 sporadic PD patient fibroblasts

| Compound | 14 | 17 | 19 | 20 | 22 | 27 | 30 | 31 |
|---|---|---|---|---|---|---|---|---|
| Bottom (% of vehicle treated) | 94.3 | 88.9 | 95.9 | 99.8 | 99.3 | 102.6 | 98.4 | 99.6 |
| Top (% of vehicle treated) | 166.8 | 152.3 | 143.1 | 147.1 | 145.8 | 158.9 | 155.6 | 151.1 |
| $EC_{50}$ (nM) | 18.9 | 127.6 | 104.4 | 17.7 | 45.4 | 171.1 | 21.01 | 5.5 |
| NB: The sPD patients have a mean reduction in MMP compared to controls of 18%; therefore increase of MMP from the vehicle treated sPD patient level of 118% would restore MMP to control levels. | | | | | | | | |

Cellular ATP levels were measured in fibroblasts from 6 (Table 3) or 3 (Table 4) patients with sporadic Parkinson's disease when treated with Compounds of the invention or Comparator compounds. The results are shown in Tables 3 and 4, where "Bottom" = max response with lowest dose of compound (0.06nM) and "top" = max response with highest dose of compound (300nM).

Table 3 - Cellular ATP levels data from 6 sporadic PD patient fibroblasts

| Compound | E | F | G | H | 2 | 7 | 9 |
|---|---|---|---|---|---|---|---|
| Bottom (% of vehicle treated) | 81.59 | 99.91 | 83.3 | 87.64 | 79.9 | 112 | 99.04 |
| Top (% of vehicle treated) | 135.5 | 111.9 | 92.72 | 120.8 | 112.6 | 171.8 | 102.9 |
| $EC_{50}$ (nM) | 18.79 | 1.912 | 58.35 | 0.1247 | 1.078 | 0.4293 | 21.32 |

Table 4 - Cellular ATP levels data from 3 sporadic PD patient fibroblasts

| Compound | 14 | 17 | 19 | 20 | 22 | 27 | 30 | 31 |
|---|---|---|---|---|---|---|---|---|
| Bottom (% of vehicle treated) | 107 | 100.7 | 98.8 | 102.4 | 95.9 | 101.4 | 96.7 | 108.1 |
| Top (% of vehicle treated) | 126.9 | 132.2 | 122.3 | 121.7 | 115.9 | 113.8 | 115.3 | 133 |
| $EC_{50}$ (nM) | 1.3 | 17.2 | 8.9 | 34.7 | 6.2 | 16.6 | 4.2 | 13.3 |
| NB: sPD fibroblasts have an average reduction of 24% of cellular ATP levels as compared to controls. Therefore a % of vehicle treated sPD fibroblasts of 124% is an increase to control ATP levels. | | | | | | | | |

The MMP and ATP assays described above along with a toxicity measure comprise the primary screen of the Compounds in primary patient fibroblasts. When considering which compound is most active in the primary screens all information is taken into account including EC50 values indicating potency and % maximal responses for both assays; based upon the combined activity expert biologists take decisions for each compound.

Oxygen Consumption data obtained from the seahorse assay for 6 sporadic PD patient fibroblast lines and 6 controls are shown in Figures 1A, 1B and 1C, from which it can be seen that sPD fibroblasts show a significant reduction of basal mitochondrial respiration of 42% (*** $p < 0.005$), maximal respiration of 48% (* $p< 0.05$) and ATP linked respiration of 18% (* $p< 0.05$). This is not improved by treatment with UDCA however treatment with Compound 7 significantly improves all three mitochondrial parameters. Note both compounds are dosed at EC90 concentrations which is 100nM for UDCA, 50nM for Compound 7. Therefore it is clear Compound 7 provides a greater increase in mitochondrial function as measured by oxygen consumption and it provides this affect at a lower concentration.

**Extracellular Acidification Rate in 6 sporadic fibroblasts and 6 controls.**

sPD fibroblasts have a significant reduction in ECAR (a proxy measure of glycolysis) by 44% as compared to controls (*** $p < 0.005$). As shown in Figure 2, this is not altered by treatment with UDCA but is significantly improved by treatment with Compound 7.

The above data shows the mitochondrial protective effects of the compounds in primary fibroblasts from sPD patients however the cell type which is primarily affected in PD is the dopaminergic neuron. The data below shows the results obtained from dopaminergic neurons derived from three sPD patients; these cultures are approximately 96% dopaminergic neurons and currently this methodology is the only protocol to generate such a pure dopaminergic culture from patient cells (method developed by Mortiboys, University of Sheffield); therefore this is the patient derived model which represents most closely the neurons affected in PD.

Table 5 below shows the results for mitochondrial function and neuronal morphology measurements in iNeurons from sPD patients vs controls when untreated or when treated with either UDCA or Compound 7.

Table 5 - iNeurons

| Parameter | % of level in iNeurons from Controls | | |
|---|---|---|---|
| | untreated sPD patients | sPD patients treated with UDCA | sPD patients treated with Compound 7 |
| ATP normalisation (% of controls) | 50 | 82 | 101 |
| ATP $EC_{50}$ (nM) | | 5 | 0.8 |
| MMP normalisation (% of controls) | 52 | 73 | 100 |
| MMP $EC_{50}$ (nM) | | 7.3 | 0.52 |
| ROS (Normalisation) (% controls) | 115 | 110 | 103 |
| Neuronal morphology (elongation; % controls) | 48 | 64 | 78 |
| Activated caspase 3 levels | 162 | 143 | 100 |
| Dopamine level | 0 | No effect | ~5% increase in dopamine release |

The data in Table 5 show very clearly that Compound 7 provides a protective effect on both mitochondrial parameters in sPD derived dopaminergic neurons in addition to improving neuronal morphology and reducing apoptosis levels (as measured by activated caspase 3 levels). Apoptosis is a major mechanism of cell death of the dopaminergic neurons in culture and of dopaminergic neurons in patients with PD.

*In vivo* mouse data with Compound 7.

Figure 3 shows that mitochondrial respiratory chain complex I activity is significantly increased in wild type mouse whole brain homogenate after dosing with Compound 7 4mg/kg and 12mg/kg in a dose dependent fashion. With a 4mg/kg dose, the increase is approximately 380% and with 12mg/kg dose, there is an increase in Complex I activity of approximately 800% compared to untreated controls (* $p < 0.05$).

Figure 4 shows results for Complex 1 activity in left striatum mouse brain homogenate of mice dosed with:

- Vehicle
- Vehicle + 12 mg Compound 7
- MPTP
- MPTP + 1 mg Compound 7
- MPTP + 4 mg Compound 7
- MPTP +12 mg Compound 7

Treatment with Compound 7 alone causes an increase in complex I activity of approximately 30% over untreated controls (* $p < 0.05$). Treatment with MPTP causes a reduction in complex I activity of 50% compared with untreated controls (** $p < 0.01$) but treatment concurrently with 1 mg Compound 7 and MPTP prevents the MPTP induced loss of complex I activity and retains complex I at normal levels (** $p < 0.01$ as compared to MPTP treatment alone), with increased doses of Compound 7 concurrently with MPTP appears to prevent any loss of complex I activity by MPTP.

Activity of Compounds in Alzheimer's Disease patient fibroblasts

Fibroblasts from both sAD and familial AD (PSEN1 mutants) were tested using the same primary screening assay for total cellular ATP levels. sAD and PSEN1 patient fibroblasts have a reduction of 21% as compared to controls. Data shown in the Table 6 below below is the mean increase in ATP levels after 24 hour treatment with compounds at 100nM concentration. As cells have an average decrease in ATP levels of 21%, an increase by 21% restores to control levels, anything over 21% is increasing beyond control levels.

Table 6

| Compound | UDCA | 2 | 7 | 8 |
|---|---|---|---|---|
| **% increase over vehicle treated** | 12% | 65% | 24% | 65% |

The data clearly show that treatment with compounds 2, 7 and 8 have a more beneficial restoration of cellular ATP levels than UDCA treatment. Furthermore compounds 2 and 8 are particularly effective increasing ATP levels dramatically.

## Claims

**1.** A compound of general formula (I):

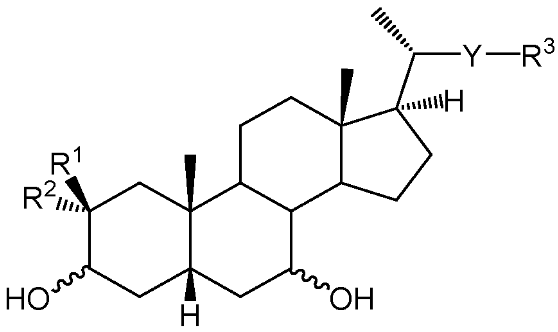

(I)

wherein

one of $R^1$ and $R^2$ is F and the other of $R^1$ and $R^2$ is H or F;
Y is a bond, or a $C_{1-20}$ alkylene, $C_{2-20}$ alkenylene or $C_{2-20}$ alkynylene linker group;
$R^3$ is $C(O)OR^{12}$, $C(O)NR^{12}R^{13}$, $S(O)_2R^{12}$, $OS(O)_2R^{12}$, $S(O)_2OR^{12}$, $OS(O)_2OR^{12}$, $S(O)_2NR^{12}R^{13}$, $C(O)NR^{12}S(O)_2R^{13}$, $NHC(O)NR^{12}S(O)_2R^{13}$, $OP(O)(OR^{12})_2$, $C(O)NR^{12}[CH(R^{15})]_nR^{16}$ or $C(O)NR^{12}C(O)CH_2NR^{12}[CH(R^{15})]_nR^{16}$;

each $R^{12}$ is independently H or $C_{1-6}$ alkyl optionally substituted by one or more substituents selected from halo, $OR^{10}$, $NR^{10}R^{11}$, $R^{16}$ and aryl;
each $R^{10}$ and $R^{11}$ is independently H or $C_{1-6}$ alkyl;
$R^{13}$ is H, $C_{1-6}$ alkyl optionally substituted by one or more substituents selected from halo and aryl; or a 3- to 8-membered carbocyclic ring or heterocyclic ring, wherein said carbocyclic or heterocyclic ring is optionally substituted with one or more substituents selected from =O and $R^{16}$; or a phenyl or 5- or 6-membered heteroaryl ring, wherein said phenyl or heteroaryl ring is optionally substituted with a substituent $R^{16}$; or when $R^3$ is $C(O)NR^{12}R^{13}$ or $S(O)_2NR^{12}R^{13}$, $R^{12}$ and $R^{13}$ together with the nitrogen atom to which they are attached form a 3- to 8-membered heterocyclic ring which optionally contains one or more further hetero atoms selected from N, O and S; and is optionally substituted with one or more substituents selected from $CH_2C(O)OH$, $C(O)OH$, $C_{1-6}$ alkyl, $C(O)OC_{1-6}$ alkyl, $S(O)_2OH$, =O and =N-OH; and is optionally fused to a phenyl group which is unsubstituted or substituted with one or more substituents selected from halo and nitro;
n is 1, 2 or 3;
each $R^{15}$ is independently H or $C_{1-6}$ alkyl optionally substituted by one or more substituents selected from halo, phenyl and 5- or 6-membered heteroaryl; a 3- to 8-membered cycloalkyl group; or a group $R^{14}$, where $R^{14}$ is a side chain of an amino acid; or
when n is 2 or 3, two $R^{15}$ groups together with the carbon atoms to which they are attached, and optionally an intervening carbon atom where present, can combine to form $-(CH_2)_p-$ such that the group $[CH(R^{15})]_n$ is a 3- to 8 membered carbocyclic ring;
p is 1, 2, 3, 4, 5 or 6;
$R^{16}$ is selected from $C(O)OH$, $S(O)_2OH$, $S(O)_2(C_{1-6}$ alkyl), $OS(O)_2OH$ and $P(O)(OH)_2$;

or a pharmaceutically acceptable salt or isotopic variant thereof.

**2.** A compound according to claim 1 which is a compound of general formula (IA), (IB), (IC) or (ID):

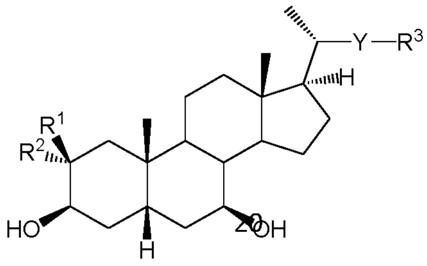

(IA)

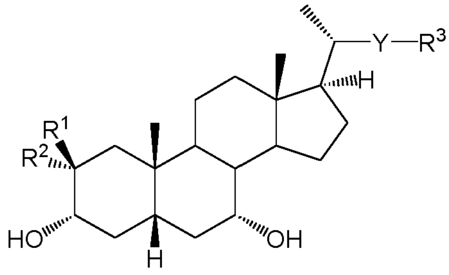

(IB)

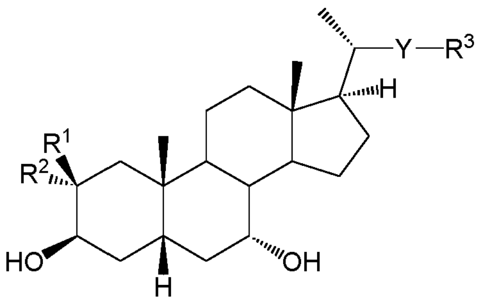

(IC)

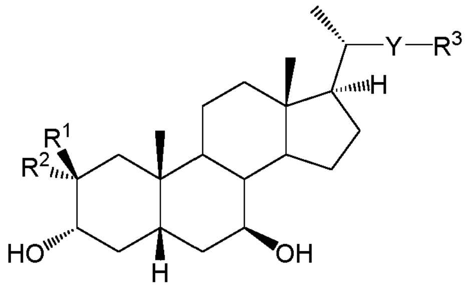

(ID)

wherein $R^1$, $R^2$, Y and $R^3$ are as defined above for general formula (I).

**3.** A compound according to claim 2 which is a compound of general formula (IA) or a compound of general formula (IB).

**4.** A compound according to any one of claims 1 to 3 wherein both $R^1$ and $R^2$ are F.

**5.** A compound according to any one of claims 1 to 4 wherein, independently or in any combination:

Y is a bond, or a $C_{1-3}$ alkylene or $C_{2-3}$ alkenylene linker group;

$R^3$ is $C(O)OR^{12}$, $C(O)NR^{12}CH(R^{14})C(O)OH$ or $C(O)NR^{12}CH(R^{15})CH(R^{15})S(O)_2OH$, wherein $R^{12}$ and $R^{14}$ are as defined in claim 1 and $R^{15}$ is H or $C_{1-6}$ alkyl optionally substituted by one or more halo or aryl groups; or $R^3$ is $C(O)OR^{12}$, $(C(O)N(R^{12})(R^{13})$ or $C(O)NR^{12}[CH(R^{15})]_nR^{16}$;

wherein $R^{12}$, $R^{13}$, $R^{15}$, $R^{16}$ and n are as defined in claim 1.

**6.** A compound according to any one of claims 1 to 5 wherein

Y is $-CH_2CH_2-$; and/or
$R^{12}$ is H or methyl or ethyl optionally substituted with $R^{16}$ or $N(R^{10})(R^{11})$; or
$R^3$ is $C(O)NR^{12}S(O)_2R^{13}$, $NHC(O)NR^{12}S(O)_2R^{13}$, $C(O)NR^{12}[CH(R^{15})]_nR^{16}$ or $C(O)NR^{12}C(O)CH_2NR^{12}[CH(R^{15})]_nR^{16}$; and $R^{12}$ is H or methyl; and/or
$R^{13}$ is a 5- or 6-membered carbocyclic ring or heterocyclic ring optionally substituted with $R^{16}$ or =O; or phenyl optionally substituted with $R^{16}$; or
$R^3$ is $C(O)NR^{12}R^{13}$ or $S(O)_2NR^{12}R^{13}$ and $R^{12}$ and $R^{13}$ together with the nitrogen atom to which they are attached form a 5- or 6-membered heterocyclic ring optionally substituted with one or more substituents selected from $R^{16}$ and =O and optionally comprising one or more further heteroatoms selected from O, N and S; and/or
$R^{16}$ is $C(O)OH$, $S(O)_2OH$, $S(O)_2(C_{1-6}$ alkyl) or $OS(O)_2OH$; or
$R^{16}$ is $C(O)OH$, $S(O)_2OH$, $OS(O)_2OH$ or $P(O)(OH)_2$ and the compound is in the form of a pharmaceutically acceptable salt.

**7.** A compound according to any one of claims 1 to 6 wherein

$R^3$ is $C(O)OR^{12}$, $C(O)NR^{12}[CH(R^{15})]_nR^{16}$ or $C(O)NR^{12}R^{13}$;
or a pharmaceutically acceptable salt thereof.

**8.** A compound according to claim 7 wherein:

$R^3$ is $C(O)OR^{12}$ and $R^{12}$ is H, $CH_2R^{16}$ or $-CH_2CH_2R^{16}$ where $R^{16}$ is as defined above; or
$R^3$ is $C(O)NR^{12}[CH(R^{15})]_nR^{16}$ wherein $R^{12}$ is H, methyl or methyl substituted with $R^{16}$; or
$R^3$ is $C(O)NR^{12}R^{13}$, wherein $R^{12}$ is H or $C_{1-3}$ alkyl substituted with a single $R^{16}$ substituent, wherein $R^{16}$ is as defined in claim 1; and/or $R^{13}$ is phenyl, or a 5- to 7-membered cycloalkyl or heterocyclyl group, any of which is optionally substituted with a single $R^{15}$ substituent and where cycloalkyl and heterocyclyl groups are substituted with one or more =O substituents.

**9.** A compound according to claim 7 or claim 8 wherein:

$R^3$ is $C(O)NR^{12}CH(R^{14})C(O)OH$ or $C(O)NR^{12}CH(R^{15})CH(R^{15})S(O)_2OH$, wherein $R^{14}$ is a side chain of an amino acid and $R^{15}$ is H or $C_{1-6}$ alkyl optionally substituted by one or more halo or aryl groups; or
$R^3$ is $C(O)NR^{12}[CH(R^{15})]_nR^{16}$ and each $R^{15}$ is independently H or $C_{1-4}$ alkyl optionally substituted as set out in claim 1; or
n is 2 or 3 and two $R^{15}$ groups combine with the carbon atoms to which they are attached, and optionally with an intervening carbon atom where present, to form a 5- to 7 membered carbocyclic ring.

**10.** A compound according to claim 9 wherein:

$R^3$ is $C(O)NR^{12}CH(R^{14})C(O)OH$, wherein $R^{12}$ is H or methyl and $R^{14}$ is H; or
$R^3$ is $C(O)NR^{12}CH(R^{15})CH(R^{15})S(O)_2OH$, wherein $R^{12}$ is H or methyl and both $R^{15}$ moieties are H.

**11.** A compound according to claim 1 selected from:

2β-fluorochenodeoxycholic acid (Compound 1);
2β-fluoro-3β,7α-dihydroxy-5β-cholanic acid (Compound 2);
2α-fluoro-3β,7α-dihydroxy-5β-cholanic acid (Compound 3);
2α-fluoro-3β,7β-dihydroxy-5β-cholanic acid (Compound 4);
2α-fluoro-3α,7α-dihydroxy-5β-cholanic acid (Compound 5);
2α-fluoro-3α,7β-dihydroxy-5β-cholanic acid (Compound 6);
2,2-difluoro-3β,7β-dihydroxy-5β-cholanic acid (Compound 7);
2,2-difluoro-3α,7α-dihydroxy-5β-cholanic acid (Compound 8);
2,2-difluoro-3α,7β-dihydroxy-5β-cholanic acid (Compound 9);
*N*-(2,2-difluoro-3β,7β-dihydroxy-5β-cholan-24-amide)-ethylsulfonic acid (Compound 10);
*N*-(2,2-difluoro-3β,7β-dihydroxy-5β-cholan-24-amide)-propanoic acid (Compound 11);
*N*-(methyl),N-(2,2-difluoro-3β,7β-dihydroxy-5β-cholan-24-amide)-acetic acid (Compound 12);
*N*-(2,2-difluoro-3β,7β-dihydroxy-5β-cholan-24-amide)-trans-2-cyclohexane carboxylic acid (Compound 13);
1-(2,2-difluoro-3β,7β-dihydroxy-5β-cholan-24-oyl)-piperidine-3-carboxylic acid (Compound 14);
3-(2,2-difluoro-3β,7β-dihydroxy-5β-cholan-24-amide)-4-thiazolidine-carboxylic acid (Compound 15);
*N*-(2,2-difluoro-3β,7β-dihydroxy-5β-cholan-24-oyl)-morpholine (Compound 16);
*N*-(2,2-difluoro-3β,7β-dihydroxy-5β-cholan-24-amide)-methylcarboxylic acid (Compound 17)
*N*-(carboxymethyl)-N-(2,2-difluoro-3β,7β-dihydroxy-5β-cholan-24-oyl)-2-amino acetic acid (Compound 18);
*N*-(methyl)-N-(2,2-difluoro-3β,7β-dihydroxy-5β-cholan-24-amide) ethylsulfonic acid (Compound 19);
3-(2,2-difluoro-3β,7β-dihydroxy-5β-cholan-24-oyl) amino-propanesulfonic acid (Compound 20);
*N*-(2,2-difluoro-3β,7β-dihydroxy-5β-cholan-24-amide) methanesulfonic acid (Compound 21);
*N*-(2,2-difluoro-3β,7β-dihydroxy-5β-cholan-24-oyl)-2-aminoethyl sulfuric acid (Compound 22);
*O*-(2,2-difluoro-3β,7β-dihydroxy-5β-cholan-24-oyl)-2-hydroxy ethyl sulfonic acid (Compound 23);
*N*-(2,2-difluoro-3β,7β-dihydroxy-5β-cholan-24-oyl) aniline-2-sulfonic acid (Compound 24);
*N*-(cyclohexyl)-N-(2,2-difluoro-3β,7β-dihydroxy-5β-cholan-24-oyl)-3-amino-propanesulfonic acid (Compound 25);
*N*-(cyclohexyl)-*N*-(2,2-difluoro-3β,7β-dihydroxy-5β-cholan-24-oyl)-2-aminoethanesulfonic acid (Compound 26);

*N*-(2,2-difluoro-3β,7β-dihydroxy-5β-cholan-24-oyl) 2-aminoethyl methyl sulfone (Compound 27);
*N*-(ethyl)-*N*-(2,2-difluoro-3β,7β-dihydroxy-5β-cholan-24-oyl)-3-amino-tetrahydrothiophene dioxide (Compound 28);
*N*-(2-(diisopropylamino)ethyl)-N-(2,2-difluoro-3β,7β-dihydroxy-5β-cholan-24-oyl)-3-amino-tetrahydrothiophene dioxide (Compound 29);
*N*-(2,2-difluoro-3β,7β-dihydroxy-5β-cholan-24-oyl)-thiomorpholine-dioxide (Compound 30);
*N*-(2,2-difluoro-3β,7β-dihydroxy-5β-cholan-24-oyl) 1,1-dioxidotetrahydro-2H-thiopyran-3-ylamine (Compound 31); and
pharmaceutically acceptable salts thereof, where appropriate, especially metal salts, such as sodium or potassium salts, particularly sodium salts or, for Compound 18, the disodium salt.

**12.** A compound according to claim 1 which is selected from:

2,2-difluoro-3β,7β-dihydroxy-5β-cholanic acid (Compound 7);
2,2-difluoro-3α,7α-dihydroxy-5β-cholanic acid (Compound 8);
2,2-difluoro-3α,7β-dihydroxy-5β-cholanic acid (Compound 9); and
pharmaceutically acceptable salts of any one thereof.

**13.** A compound according to any one of claims 1 to 12 for use in medicine, for example for use in the treatment or prevention of a neurodegenerative disorder such as Parkinson's disease, mild cognitive impairment, dementia; including Alzheimer's disease, vascular dementia and dementia with Lewy bodies; Huntington's disease, amyotrophic lateral sclerosis (motor neurone disease), progressive supranuclear palsy or Wilson's disease.

**14.** A compound for use according to claim 13, wherein the neurodegenerative disorder is Parkinson's disease and the compound is selected from 2,2-difluoro-3β,7β-dihydroxy-5β-cholanic acid (Compound 7) and 2,2-difluoro-3α,7β-dihydroxy-5β-cholanic acid (Compound 9); or
wherein the neurodegenerative disorder is Alzheimer's disease and the compound is 2,2-difluoro-3α,7α-dihydroxy-5β-cholanic acid (Compound 8).

**15.** A pharmaceutical composition comprising a compound according to any one of claims 1 to 12 and a pharmaceutically acceptable excipient or carrier.

**16.** A process for the preparation of a compound according to any one of claims 1 to 12 comprising:

A. for a compound of general formula (IB) or (IC) as defined in claim 2 in which $R^1$ is F and $R^3$ is $C(O)OR^{12a}$: wherein $R^{12a}$ is $C_{1-6}$ alkyl optionally substituted by one or more halo or aryl groups:

treating with an acid a compound of general formula (II):

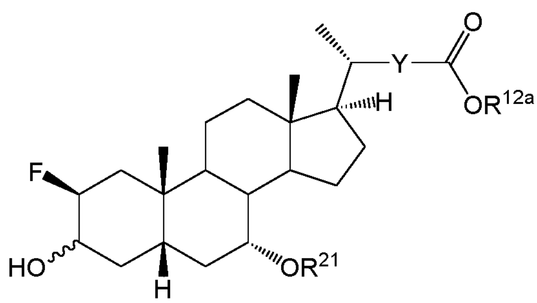

(II)

wherein Y and $R^3$ are as defined in claim 1; $R^{12a}$ is $C_{1-6}$ alkyl optionally substituted by one or more halo or aryl groups; and $R^{21}$ is an OH protecting group which is acid labile;

B. for a compound of general formula (IA) or (IC) as defined in claim 2 in which $R^2$ is F and $R^3$ is $C(O)OR^{12a}$, wherein $R^{12a}$ is as defined for general formula (II):

reducing a compound of general formula (XIIa):

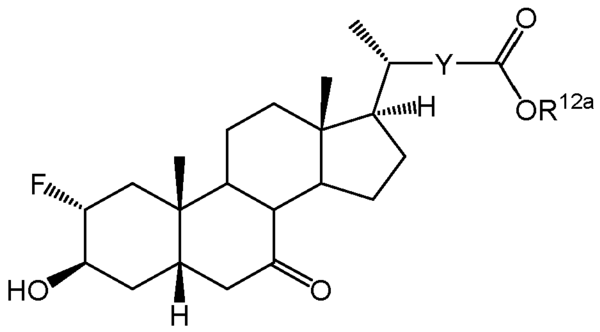

(XIIa)

wherein Y is as defined in claim 1 and $R^{12a}$ is as defined for general formula (II);

C. for a compound of general formula (IB) or (ID) as defined in claim 2 in which $R^2$ is F and $R^3$ is $C(O)OR^{12a}$, wherein $R^{12a}$ is as defined for general formula (II):

reducing a compound of general formula (XIIb):

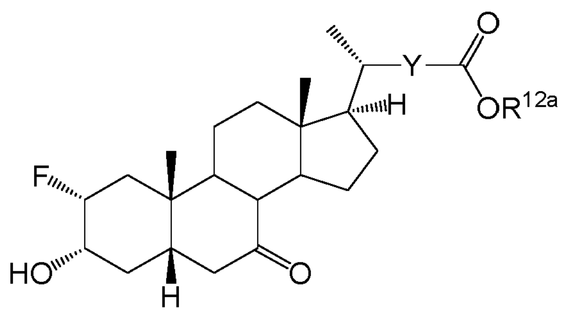

(XIIb)

wherein Y is as defined in claim 1 and $R^{12a}$ is as defined for general formula (II);

D. for a compound of general formula (IA) as defined in claim 2 in which both $R^1$ and $R^2$ are F and $R^3$ is $C(O)OR^{12a}$, wherein $R^{12a}$ is as defined for general formula (II):

reacting with an acid a compound of general formula (XXI):

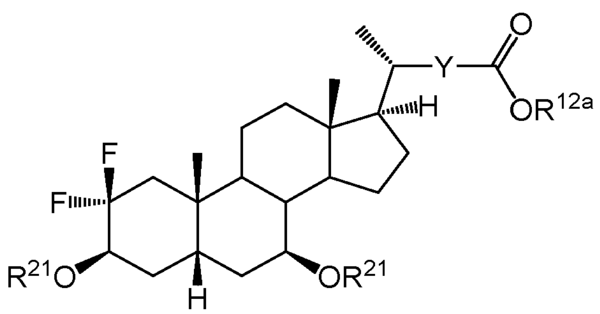

(XXI)

wherein Y is as defined in claim 1 and $R^{12a}$ and $R^{21}$ are as defined for general formula (II);

E. for a compound of general formula (IB) or (ID) as defined in claim 2 in which both $R^1$ and $R^2$ are F and $R^3$ is $C(O)OR^{12a}$, wherein $R^{12a}$ is as defined for general formula (II):

reducing a compound of general formula (XXII):

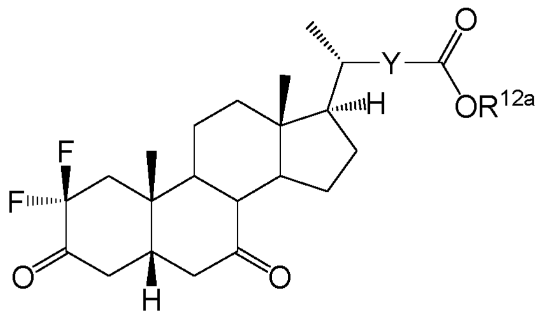

(XXII)

wherein Y is as in claim 1 and $R^{12a}$ is as defined for general formula (II);

F. for a compound of general formula (I) in which $R^3$ is C(O)OH:
hydrolysing a compound of general formula (I) in which $R^3$ is $C(O)R^{12a}$, wherein $R^{12a}$ is as defined above for general formula (II);
G. for a compound of general formula (I) in which $R^3$ is $C(O)NR^{12}R^{13}$:

reacting a compound of general formula (I) in which $R^3$ is C(O)OH with an amine of general formula:

$H\text{-}NR^{12}R^{13}$

wherein $R^{12}$ and $R^{13}$ are as defined in claim 1;
in the presence of a coupling reagent and an amine;

H. for a compound of general formula (I) in which $R^3$ is $C(O)NR^{12}[CH(R^{15})_{]n}R^{16}$:

reacting a compound of general formula (I) in which $R^3$ is C(O)OH with a compound of general formula (XL):

$HNR^{12}[CH(R^{15})]_nR^{16}$ (XL)

wherein $R^{12}$, $R^{15}$, n and $R^{16}$ are as defined in claim 1;
in the presence of a coupling agent and an amine;

I. for a compound of general formula (I) in which $R^3$ is $C(O)NR^{12}CH(R^{14})C(O)OH$: reacting a compound of general formula (I) in which $R^3$ is C(O)OH by reaction with an amino acid of general formula (XLI):

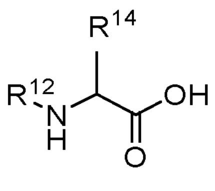

(XLI)

wherein $R^{12}$ and $R^{14}$ are as defined in claim 1;
in the presence of a coupling agent and an amine;

J. for a compound of general formula (I) in which $R^3$ is $C(O)NR^{12}CH(R^{15})CH(R^{15})S(O)_2OH$:
reacting a compound of general formula (I) in which $R^3$ is C(O)OH by reaction with a compound of general formula (XLII):

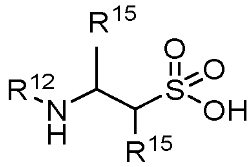

(XLII)

wherein $R^{12}$ and $R^{15}$ are as defined in claim 1;
in the presence of a coupling agent and an amine;

K. for a compound of general formula (I) in which $R^3$ is $C(O)NR^{12}S(O)_2R^{13}$:

reacting a compound of general formula (I) in which $R^3$ is C(O)OH with a compound of formula:

$NHR^{12}S(O)_2R^{13}$

wherein $R^{12}$ and $R^{13}$ are as defined in claim 1, in the presence of a coupling reagent and an amine;

L. for a compound of general formula (I) in which $R^3$ is $NHC(O)NR^{12}S(O)_2R^{13}$: reacting a compound of general formula (I) in which $R^3$ is C(O)OH as follows:

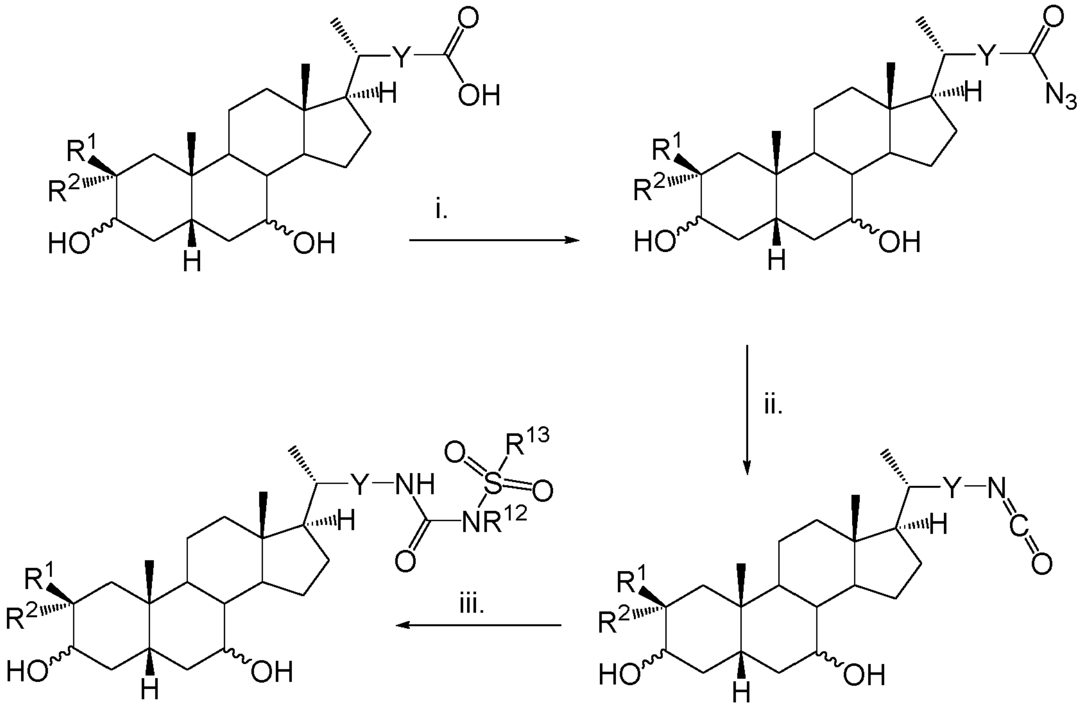


i diphenyl phosphoryl azide / triethylamine
ii heat
iii $R^{13}SO_2NHR^{12}$

wherein $R^1$, $R^2$, $R^{12}$ and $R^{13}$ are as defined in claim 1;
M. for a compound of general formula (I) in which $R^3$ is $S(O)_2OR^{12}$:

reacting a compound of general formula (I) in which $R^3$ is C(O)OH with a $C_{1-6}$ alkanoyl or benzoyl chloride or with a $C_{1-6}$ alkanoic anhydride to give an intermediate in which OH groups are protected; and
converting the carboxylic acid group, $R^3$, of the protected intermediate to OH by reduction with a hydride reducing agent to give a reduced intermediate; and
halogenating the reduced intermediate to give a halogenated intermediate in which the OH group is replaced

with a halogen; and
reacting the halogenated intermediate with sodium sulphite in an alcoholic solvent;

N. for a compound of general formula (I) in which $R^3$ is $OS(O)_2R^{12}$:

reacting a compound of general formula (I) in which $R^3$ is $C(O)OR^{12}$ with a $C_{1-6}$ alkanoyl or benzoyl chloride or with a $C_{1-6}$ alkanoic anhydride to protect any OH groups; and
converting the $C(O)OR^{12}$ of the protected intermediate to OH by reduction with a hydride reducing agent to give a reduced intermediate; and
reacting the reduced intermediate with chlorosulfonic acid in the presence of a base to give a protected product; and
base hydrolysis of the protected product to remove the protecting groups;

O. for a compound of general formula (I) in which $R^3$ is $S(O)_2R^{12}$:
Reacting the reduced intermediate of (M) or (N) above with Lawesson's reagent followed by oxidation of the resultant product.

## Patentansprüche

**1.** Verbindung der allgemeinen Formel (I):

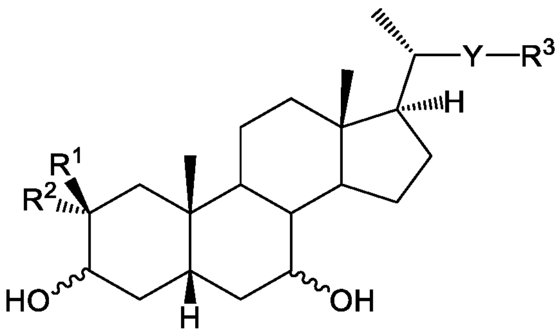

(I)

wobei
eines von $R^1$ und $R^2$ F ist und das andere von $R^1$ und $R^2$ H oder F ist;
Y eine Bindung oder eine $C_{1-20}$-Alkylen-, $C_{2-20}$-Alkenylen- oder $C_{2-20}$-Alkinylen-Linkergruppe ist;
$R^3$ $C(O)OR^{12}$, $C(O)NR^{12}R^{13}$, $S(O)_2R^{12}$, $OS(O)_2R^{12}$, $S(O)_2OR^{12}$, $OS(O)_2OR^{12}$, $S(O)_2NR^{12}R^{13}$, $C(O)NR^{12}S(O)_2R^{13}$, $NHC(O)NR^{12}S(O)_2R^{13}$, $OP(O)(OR^{12})_2$, $C(O)NR^{12}[CH(R^{15})]_nR^{16}$ oder $C(O)NR^{12}C(O)CH_2NR^{12}[CH(R^{15})]_nR^{16}$ ist;
jedes $R^{12}$ unabhängig H oder $C_{1-6}$-Alkyl ist, gegebenenfalls substituiert mit einem oder mehreren Substituenten, ausgewählt aus Halogen, $OR^{10}$, $NR^{10}R^{11}$, $R^{16}$ und Aryl;
$R^{10}$ und $R^{11}$ jeweils unabhängig voneinander H oder $C_{1-6}$-Alkyl sind; $R^{13}$ H, $C_{1-6}$-Alkyl, gegebenenfalls substituiert mit einem oder mehreren Substituenten, ausgewählt aus Halogen und Aryl; oder ein 3- bis 8-gliedriger carbocyclischer Ring oder heterocyclischer Ring, wobei der carbocyclische oder heterocyclische Ring gegebenenfalls mit einem oder mehreren Substituenten substituiert ist, ausgewählt aus =O und $R^{16}$; oder ein Phenyl- oder 5- oder 6-gliedriger Heteroarylring ist, wobei der Phenyl- oder Heteroarylring gegebenenfalls mit einem Substituenten $R^{16}$ substituiert ist; oder
wenn $R^3$ $C(O)NR^{12}R^{13}$ oder $S(O)_2NR^{12}R^{13}$ ist, $R^{12}$ und $R^{13}$ zusammen mit dem Stickstoffatom, an das sie gebunden sind, einen 3- bis 8-gliedrigen heterocyclischen Ring bilden, der gegebenenfalls ein oder mehrere weitere Heteroatome enthält, ausgewählt aus N, O und S; und gegebenenfalls mit einem oder mehreren Substituenten substituiert ist, ausgewählt aus $CH_2C(O)OH$, $C(O)OH$, $C_{1-6}$-Alkyl, $C(O)OC_{1-6}$-Alkyl, $S(O)_2OH$, =O und =N-OH; und gegebenenfalls an eine Phenylgruppe kondensiert ist, die unsubstituiert oder mit einem oder mehreren Substituenten substituiert ist, ausgewählt aus Halogen und Nitro;
n 1, 2 oder 3 ist;
jedes $R^{15}$ unabhängig H oder $C_{1-6}$-Alkyl, gegebenenfalls substituiert mit einem oder mehreren Substituenten,

ausgewählt aus Halogen, Phenyl und 5- oder 6-gliedrigem Heteroaryl; eine 3-bis 8-gliedrige Cycloalkylgruppe; oder eine Gruppe $R^{14}$ ist, wobei $R^{14}$ eine Seitenkette einer Aminosäure ist; oder

wenn n 2 oder 3 ist, zwei $R^{15}$-Gruppen zusammen mit den Kohlenstoffatomen, an die sie gebunden sind, und gegebenenfalls einem dazwischenliegenden Kohlenstoffatom, sofern vorhanden, unter Bilden von $-(CH_2)_p-$ derart vereinigt werden können, dass die Gruppe $[CH(R^{15})]_n$ ein 3- bis 8-gliedriger carbocyclischer Ring ist;

p 1, 2, 3, 4, 5 oder 6 ist;

$R^{16}$ ausgewählt ist aus C(O)OH, $S(O)_2OH$, $S(O)_2(C_{1-6}$-Alkyl), $OS(O)_2OH$ und $P(O)(OH)_2$;

oder ein pharmazeutisch verträgliches Salz oder eine pharmazeutisch verträgliche Isotopenvariante davon.

**2.** Verbindung nach Anspruch 1, die eine Verbindung der allgemeinen Formel (IA), (IB), (IC) oder (ID) ist:

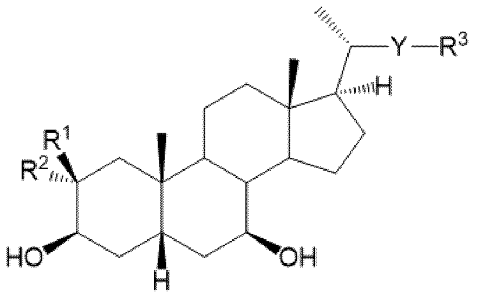

(IA)

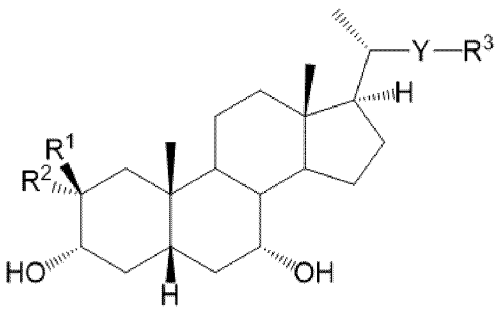

(IB)

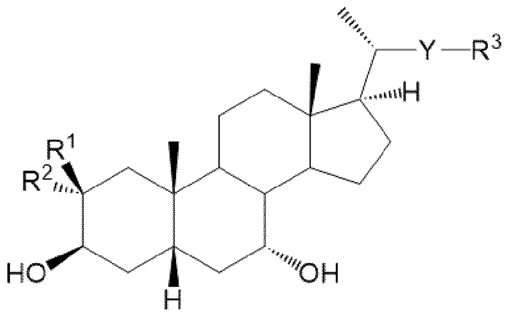

(IC)

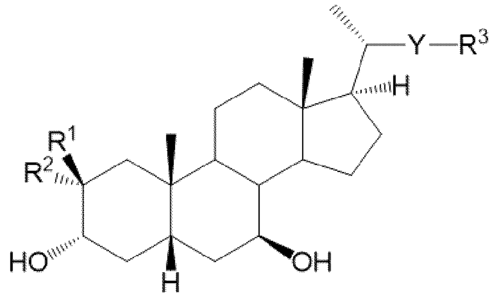

(ID)

wobei $R^1$, $R^2$, Y und $R^3$ wie vorstehend für die allgemeine Formel (I) definiert sind.

**3.** Verbindung nach Anspruch 2, die eine Verbindung der allgemeinen Formel (IA) oder eine Verbindung der allgemeinen Formel (IB) ist.

**4.** Verbindung nach einem der Ansprüche 1 bis 3, wobei sowohl $R^1$ als auch $R^2$ F sind.

**5.** Verbindung nach einem der Ansprüche 1 bis 4, wobei unabhängig oder in beliebiger Kombination:

Y eine Bindung oder eine $C_{1-3}$-Alkylen- oder $C_{2-3}$-Alkenylen-Linkergruppe ist;

$R^3$ $C(O)OR^{12}$, $C(O)NR^{12}CH(R^{14})C(O)OH$ oder $C(O)NR^{12}CH(R^{15})CH(R^{15})S(O)_2OH$ ist, wobei $R^{12}$ und $R^{14}$ wie in Anspruch 1 definiert sind und $R^{15}$ H oder $C_{1-6}$-Alkyl ist, gegebenenfalls substituiert mit einem/r oder mehreren Halogenen oder Arylgruppen; oder

$R^3$ $C(O)OR^{12}$, $C(O)N(R^{12})(R^{13})$ oder $C(O)NR^{12}[CH(R^{15})]_nR^{16}$ ist;

wobei $R^{12}$, $R^{13}$, $R^{15}$, $R^{16}$ und n wie in Anspruch 1 definiert sind.

**6.** Verbindung nach einem der Ansprüche 1 bis 5, wobei Y $-CH_2CH_2-$ ist; und/oder

$R^{12}$ H oder Methyl oder Ethyl ist, gegebenenfalls substituiert mit $R^{16}$ oder $N(R^{10})(R^{11})$; oder

$R^3$ C (O) $NR^{12}S(O)$ $_2R^{13}$, $NHC(O)NR^{12}S(O)_2R^{13}$, $C(O)NR^{12}[CH(R^{15})]R^6$ oder $C(O)NR^{12}C(O)CH_2NR^{12}$

$[CH(R^{15})]R^6$ ist; und $R^{12}$ H oder Methyl ist; und/oder
$R^{13}$ ein 5- oder 6-gliedriger carbocyclischer Ring oder heterocyclischer Ring ist, gegebenenfalls substituiert mit $R^{16}$ oder =O; oder Phenyl, gegebenenfalls substituiert mit $R^{16}$; oder $R^3C(O)NR^{12}R^{13}$ oder $S(O)_2NR^{12}R^{13}$ ist und $R^{12}$ und $R^{13}$ zusammen mit dem Stickstoffatom, an das sie gebunden sind, einen 5- oder 6-gliedrigen heterocyclischen Ring bilden, gegebenenfalls substituiert mit einem oder mehreren Substituenten, ausgewählt aus $R^{16}$ und =O und gegebenenfalls umfassend ein oder mehrere weitere Heteroatome, ausgewählt aus O, N und S; und/oder
$R^{16}$ C(O)OH, $S(O)_2OH$, $S(O)_2(C_{1-6}$-Alkyl) oder $OS(O)_2OH$ ist; oder
$R^{16}$ C(O)OH, $S(O)_2OH$, $OS(O)_2OH$ oder $P(O)(OH)_2$ ist und die Verbindung in Form eines pharmazeutisch verträglichen Salzes vorliegt.

**7.** Verbindung nach einem der Ansprüche 1 bis 6, wobei

$R^3$ $C(O)OR^{12}$, $C(O)NR^{12}[CH(R^{15})]_nR^{16}$ oder C(O) $NR^{12}R^{13}$ ist;
oder ein pharmazeutisch verträgliches Salz davon.

**8.** Verbindung nach Anspruch 7, wobei:

$R^3$ $C(O)OR^{12}$ ist und $R^{12}$ H, $CH_2R^{16}$ oder $-CH_2CH_2R^{16}$ ist, wobei $R^{16}$ wie vorstehend definiert ist; oder
$R^3$ $C(O)NR^{12}[CH(R^{15})]_nR^{16}$ ist, wobei $R^{12}$ H, Methyl oder mit $R^{16}$ substituiertes Methyl ist; oder
$R^3$ $C(O)NR^{12}R^{13}$ ist, wobei $R^{12}$ H oder mit einem einzigen $R^{16}$-Substituenten substituiertes $C_{1-3}$-Alkyl ist, wobei $R^{16}$ wie in Anspruch 1 definiert ist; und/oder $R^{13}$ Phenyl oder eine 5- bis 7-gliedrige Cycloalkyl- oder Heterocyclylgruppe ist, von denen jede gegebenenfalls mit einem einzigen $R^{16}$-Substituenten substituiert ist und wobei die Cycloalkyl- und
Heterocyclylgruppe mit einem oder mehreren =O-Substituenten substituiert sind.

**9.** Verbindung nach Anspruch 7 oder Anspruch 8, wobei:

$R^3$ $C(O)NR^{12}CH(R^{14})C(O)OH$ oder $C(O)NR^{12}CH(R^{15})CH(R^{15})S(O)_2OH$ ist, wobei $R^{14}$ eine Seitenkette einer Aminosäure ist und $R^{15}$ H oder $C_{1-6}$-Alkyl ist, gegebenenfalls substituiert mit einem/r oder mehreren Halogenen oder Arylgruppen; oder
$R^3$ $C(O)NR^{12}[CH(R^{15})]_nR^{16}$ ist und jedes $R^{15}$ unabhängig H oder $C_{1-4}$-Alkyl ist, gegebenenfalls substituiert wie in Anspruch 1 ausgeführt; oder
n 2 oder 3 ist und zwei $R^{15}$-Gruppen mit den Kohlenstoffatomen, an die sie gebunden sind, und gegebenenfalls mit einem dazwischenliegenden Kohlenstoffatom, sofern vorhanden, unter Bilden eines 5- bis 7-gliedrigen carbocyclischen Rings vereinigt werden.

**10.** Verbindung nach Anspruch 9, wobei:

$R^3$ $C(O)NR^{12}CH(R^{14})C(O)OH$ ist, wobei $R^{12}$ H oder Methyl ist und $R^{14}$ H ist; oder
$R^3$ $C(O)NR^{12}CH(R^{15})CH(R^{15})S(O)_2OH$ ist, wobei $R^{12}$ H oder Methyl ist und beide $R^{15}$-Gruppierungen H sind.

**11.** Verbindung nach Anspruch 1, ausgewählt aus:

2β-Fluorchenodesoxycholsäure (Verbindung 1);
2β-Fluor-3β,7α-dihydroxy-5β-cholansäure (Verbindung 2);
2α-Fluor-3β,7α-dihydroxy-5β-cholansäure (Verbindung 3);
2α-Fluor-3β,7β-dihydroxy-5β-cholansäure (Verbindung 4);
2α-Fluor-3α,7α-dihydroxy-5β-cholansäure (Verbindung 5);
2α-Fluor-3α,7β-dihydroxy-5β-cholansäure (Verbindung 6);
2,2-Difluor-3β,7β-dihydroxy-5β-cholansäure (Verbindung 7);
2,2-Difluor-3α,7α-dihydroxy-5β-cholansäure (Verbindung 8);
2,2-Difluor-3α,7β-dihydroxy-5β-cholansäure (Verbindung 9);
*N*-(2,2-Difluor-3β,7β-dihydroxy-5β-cholan-24-amid)ethylsulfonsäure (Verbindung 10);
*N*-(2,2-Difluor-3β,7β-dihydroxy-5β-cholan-24-amid)propansäure (Verbindung 11);
*N*-(Methyl),*N*-(2,2-Difluor-3β,7β-dihydroxy-5β-cholan-24-amid)essigsäure (Verbindung 12);
*N*-(2,2-Difluor-3β,7β-dihydroxy-5β-cholan-24-amid)-trans-2-cyclohexancarbonsäure (Verbindung 13);
1-(2,2-Difluor-3β,7β-dihydroxy-5β-cholan-24-oyl)piperidin-3-carbonsäure (Verbindung 14);

3-(2,2-Difluor-3β,7β-dihydroxy-5β-cholan-24-amid)-4-thiazolidincarbonsäure (Verbindung 15);
*N*-(2,2-Difluor-3β,7β-dihydroxy-5β-cholan-24-oyl)morpholin (Verbindung 16);
*N*-(2,2-Difluor-3β,7β-dihydroxy-5β-cholan-24-amid)methylcarbonsäure (Verbindung 17)
*N*-(Carboxymethyl)-*N*-(2,2-difluor-3β,7β-dihydroxy-5β-cholan-24-oyl)-2-aminoessigsäure (Verbindung 18);
*N*-(Methyl)-*N*-(2,2-difluor-3β,7β-dihydroxy-5β-cholan-24-amid)ethylsulfonsäure (Verbindung 19);
3-(2,2-Difluor-3β,7β-dihydroxy-5β-cholan-24-oyl)aminopropansulfonsäure (Verbindung 20);
*N*-(2,2-Difluor-3β,7β-dihydroxy-5β-cholan-24-amid)methansulfonsäure (Verbindung 21);
*N*-(2,2-Difluor-3β,7β-dihydroxy-5β-cholan-24-oyl)-2-aminoethylschwefelsäure (Verbindung 22);
*O*-(2,2-Difluor-3β,7β-dihydroxy-5β-cholan-24-oyl)-2-hydroxyethylsulfonsäure (Verbindung 23);
*N*-(2,2-Difluor-3β,7β-dihydroxy-5β-cholan-24-oyl) anilin-2-sulfonsäure (Verbindung 24);
*N*-(Cyclohexyl)-*N*-(2,2-difluor-3β,7β-dihydroxy-5β-cholan-24-oyl)-3-aminopropansulfonsäure (Verbindung 25);
*N*-(Cyclohexyl)-*N*-(2,2-difluor-3β,7β-dihydroxy-5β-cholan-24-oyl)-2-aminoethansulfonsäure (Verbindung 26);
*N*-(2,2-Difluor-3β,7β-dihydroxy-5β-cholan-24-oyl)-2-aminoethylmethylsulfon (Verbindung 27);
*N*-(Ethyl)-*N*-(2,2-difluor-3β,7β-dihydroxy-5β-cholan-24-oyl)-3-aminotetrahydrothiophen-Dioxid (Verbindung 28);
*N*-(2-(Diisopropylamino)ethyl)-*N*-(2,2-difluor-3β,7β-dihydroxy-5β-cholan-24-oyl)-3-aminotetrahydrothiophen-Dioxid (Verbindung 29);
*N*-(2,2-Difluor-3β,7β-dihydroxy-5β-cholan-24-oyl)thiomorpholin-Dioxid (Verbindung 30);
*N*-(2,2-Difluor-3β,7β-dihydroxy-5β-cholan-24-oyl)-1,1-dioxidotetrahydro-2H-thiopyran-3-ylamin (Verbindung 31); und
pharmazeutisch verträgliche Salze davon, sofern angemessen, insbesondere Metallsalze, wie Natrium- oder Kaliumsalze, insbesondere Natriumsalze oder, bei Verbindung 18, das Dinatriumsalz.

**12.** Verbindung nach Anspruch 1, ausgewählt aus:

2,2-Difluor-3β,7β-dihydroxy-5β-cholansäure (Verbindung 7);
2,2-Difluor-3α,7α-dihydroxy-5β-cholansäure (Verbindung 8);
2,2-Difluor-3α,7β-dihydroxy-5β-cholansäure (Verbindung 9); und
pharmazeutisch verträgliche Salze einer beliebigen davon.

**13.** Verbindung nach einem der Ansprüche 1 bis 12 zur Verwendung in der Medizin, zum Beispiel zur Verwendung bei der Behandlung oder Prävention einer neurodegenerativen Störung, wie Morbus Parkinson, leichter kognitiver Beeinträchtigung, Demenz, einschließlich Morbus Alzheimer, vaskulärer Demenz und Lewy-Körperchen-Demenz, Chorea Huntington, amyotropher Lateralsklerose (Motoneuron-Erkrankung), progressiver supranukleärer Lähmung oder Morbus Wilson.

**14.** Verbindung zur Verwendung nach Anspruch 13, wobei die neurodegenerative Störung Morbus Parkinson ist und die Verbindung ausgewählt ist aus 2,2-Difluor-3β,7β-dihydroxy-5β-cholansäure (Verbindung 7) und 2,2-Difluor-3α,7β-dihydroxy-5β-cholansäure (Verbindung 9); oder
wobei die neurodegenerative Störung Morbus Alzheimer ist und die Verbindung 2,2-Difluor-3α,7α-dihydroxy-5β-cholansäure (Verbindung 8) ist.

**15.** Pharmazeutische Zusammensetzung, umfassend eine Verbindung nach einem der Ansprüche 1 bis 12 und einen pharmazeutisch verträglichen Hilfsstoff oder Träger.

**16.** Verfahren zur Herstellung einer Verbindung nach einem der Ansprüche 1 bis 12, umfassend:

A. für eine Verbindung der allgemeinen Formel (IB) oder (IC) wie in Anspruch 2 definiert, worin $R^1$ F ist und $R^3$ $C(O)OR^{12a}$ ist:
wobei $R^{12a}$ $C_{1-6}$-Alkyl ist, gegebenenfalls substituiert mit einem/r oder mehreren Halogenen oder Arylgruppen:

Behandeln einer Verbindung der allgemeinen Formel (II) mit einer Säure:

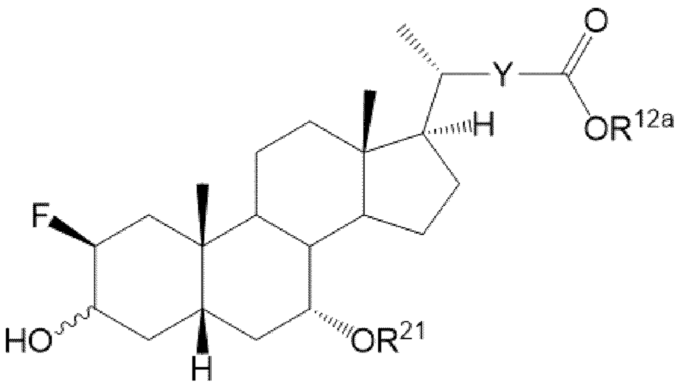

(II)

wobei Y und $R^3$ wie in Anspruch 1 definiert sind; $R^{12a}$ $C_{1-6}$-Alkyl ist, gegebenenfalls substituiert mit einem/r oder mehreren Halogenen oder Arylgruppen; und $R^{21}$ eine OH-Schutzgruppe ist, die säurelabil ist;

B. für eine Verbindung der allgemeinen Formel (IA) oder (IC) wie in Anspruch 2 definiert, worin $R^2$ F ist und $R^3$ C(O)O$R^{12a}$ ist, wobei $R^{12a}$ wie für die allgemeine Formel (II) definiert ist:

Reduzieren einer Verbindung der allgemeinen Formel (XIIa):

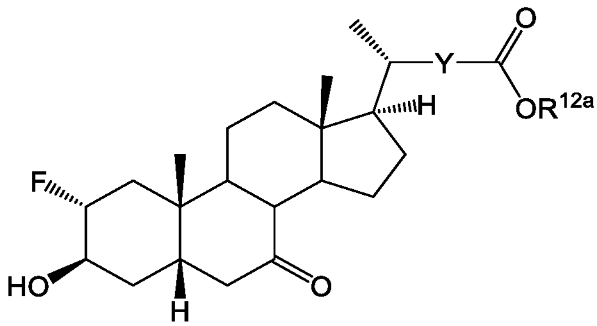

(XIIa)

wobei Y wie in Anspruch 1 definiert ist und $R^{12a}$ wie für die allgemeine Formel (II) definiert ist;

C. für eine Verbindung der allgemeinen Formel (IB) oder (ID) wie in Anspruch 2 definiert, worin $R^2$ F ist und $R^3$ C(O)O$R^{12a}$ ist, wobei $R^{12a}$ wie für die allgemeine Formel (II) definiert ist: Reduzieren einer Verbindung der allgemeinen Formel (XIIb):

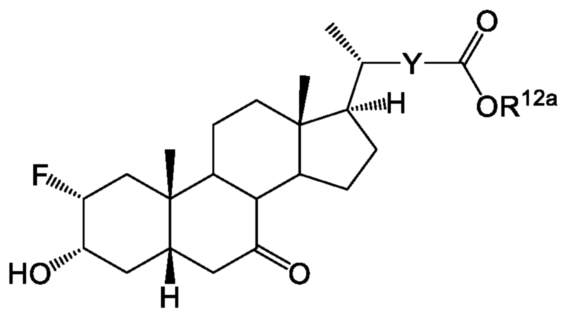

(XIIb)

wobei Y wie in Anspruch 1 definiert ist und $R^{12a}$ wie für die allgemeine Formel (II) definiert ist;

D. für eine Verbindung der allgemeinen Formel (IA) wie in Anspruch 2 definiert, worin sowohl $R^1$ und $R^2$ F sind und

$R^3$ C(O)OR$^{12a}$ ist, wobei $R^{12a}$ wie für die allgemeine Formel (II) definiert ist:

Umsetzen einer Verbindung der allgemeinen Formel (XXI) mit einer Säure:

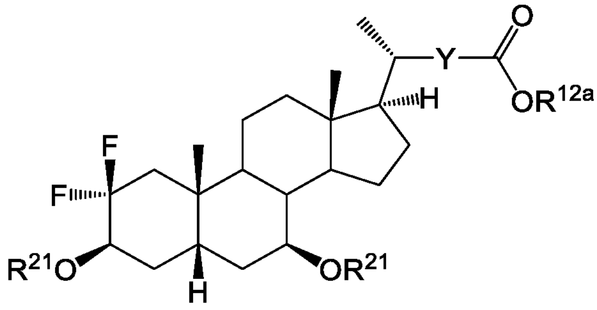

(XXI)

wobei Y wie in Anspruch 1 definiert ist und $R^{12a}$ und $R^{21}$ wie für die allgemeine Formel (II) definiert sind;

E. für eine Verbindung der allgemeinen Formel (IB) oder (ID) wie in Anspruch 2 definiert, worin sowohl $R^1$ und $R^2$ F sind und $R^3$ C(O)OR$^{12a}$ ist, wobei $R^{12a}$ wie für die allgemeine Formel (II) definiert ist:

Reduzieren einer Verbindung der allgemeinen Formel (XXII):

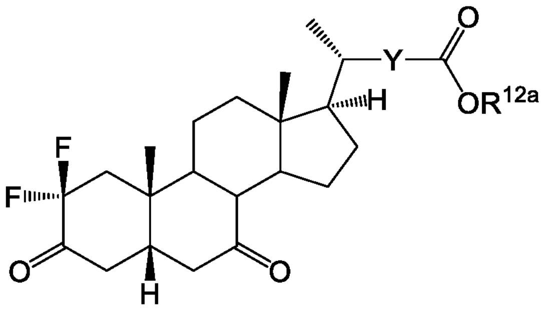

(XXII)

wobei Y wie in Anspruch 1 ist und $R^{12a}$ wie für die allgemeine Formel (II) definiert ist;

F. für eine Verbindung der allgemeinen Formel (I), worin $R^3$ C(O)OH ist:
Hydrolysieren einer Verbindung der allgemeinen Formel (I), worin $R^3$ C(O)R$^{12a}$ ist, wobei $R^{12a}$ wie vorstehend für die allgemeine Formel (II) definiert ist;
G. für eine Verbindung der allgemeinen Formel (I), worin $R^3$ C(O)NR$^{12}$R$^{13}$ ist:

Umsetzen einer Verbindung der allgemeinen Formel (I), worin $R^3$ C(O)OH ist, mit einem Amin der allgemeinen Formel:

H-NR$^{12}$R$^{13}$

wobei $R^{12}$ und $R^{13}$ wie in Anspruch 1 definiert sind;
in Gegenwart eines Kopplungsreagenzes und eines Amins;

H. für eine Verbindung der allgemeinen Formel (I), worin $R^3$ C(O)NR$^{12}$[CH(R$^{15}$)]$_n$R$^{16}$ ist:

Umsetzen einer Verbindung der allgemeinen Formel (I), worin $R^3$ C(O)OH ist, mit einer Verbindung der allgemeinen Formel (XL):

HNR$^{12}$[CH(R$^{15}$)]$_n$R$^{16}$ (XL)

wobei $R^{12}$, $R^{15}$, n und $R^{16}$ wie in Anspruch 1 definiert sind;
in Gegenwart eines Kopplungsmittels oder eines Amins;

I. für eine Verbindung der allgemeinen Formel (I), worin $R^3$ $C(O)NR^{12}CH(R^{14})C(O)OH$ ist: Umsetzen einer Verbindung der allgemeinen Formel (I), worin $R^3$ C(O)OH ist, durch Umsetzung mit einer Aminosäure der allgemeinen Formel (XLI):

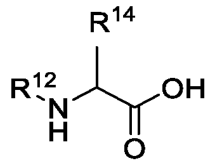

(XLI)

wobei $R^{12}$ und $R^{14}$ wie in Anspruch 1 definiert sind;
in Gegenwart eines Kopplungsmittels oder eines Amins;

J. für eine Verbindung der allgemeinen Formel (I), worin $R^3$ $C(O)NR^{12}CH(R^{15})CH(R^{15})S(O)_2OH$ ist:

Umsetzen einer Verbindung der allgemeinen Formel (I), worin $R^3$ C(O)OH ist, durch Umsetzung mit einer Verbindung der allgemeinen Formel (XLII):

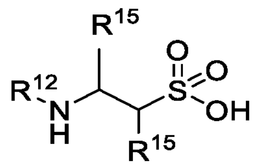

(XLII)

wobei $R^{12}$ und $R^{15}$ wie in Anspruch 1 definiert sind;
in Gegenwart eines Kopplungsmittels oder eines Amins;

K. für eine Verbindung der allgemeinen Formel (I), worin $R^3$ $C(O)NR^{12}S(O)_2R^{13}$ ist:

Umsetzen einer Verbindung der allgemeinen Formel (I), worin $R^3$ C(O)OH ist, mit einer Verbindung der Formel:

$NHR^{12}S(O)_2R^{13}$

wobei $R^{12}$ und $R^{13}$ wie in Anspruch 1 definiert sind, in Gegenwart eines Kopplungsreagenzes und eines Amins;

L. für eine Verbindung der allgemeinen Formel (I), worin $R^3$ $NHC(O)NR^{12}S(O)_2R^{13}$ist:

Umsetzen einer Verbindung der allgemeinen Formel (I), worin $R^3$ C(O)OH ist, wie folgt:

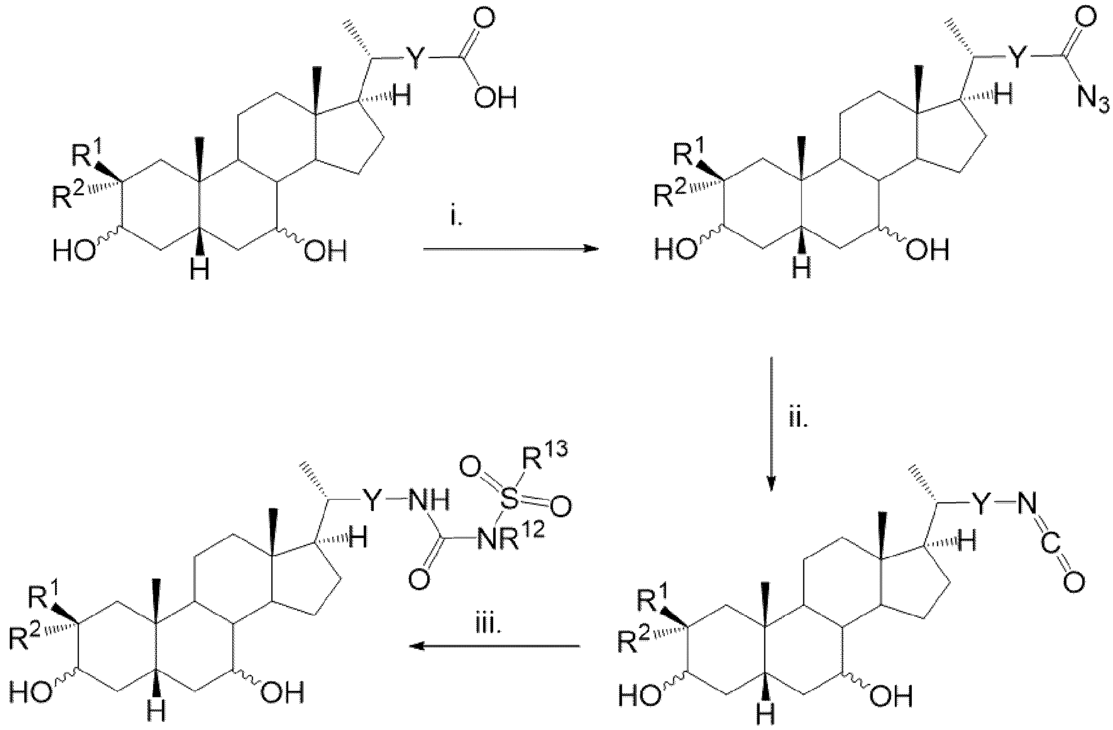


i Diphenylphosphorylazid/Triethylamin
ii Wärme
iii $R^{13}SO_2NHR^{12}$

wobei $R^1$, $R^2$, $R^{12}$ und $R^{13}$ wie in Anspruch 1 definiert sind;

M. für eine Verbindung der allgemeinen Formel (I), worin $R^3$ S(O)2OR$^{12}$ ist:

Umsetzen einer Verbindung der allgemeinen Formel (I), worin $R^3$ C(O)OH ist, mit einem $C_{1-6}$-Alkanoyl- oder Benzoylchlorid oder mit einem $C_{1-6}$-Alkansäureanhydrid, um ein Zwischenprodukt zu erhalten, worin die OH-Gruppen geschützt sind; und
Umwandeln der Carbonsäuregruppe $R^3$ des geschützten Zwischenprodukts durch Reduktion mit einem Hydridreduktionsmittel in OH, um ein reduziertes Zwischenprodukt zu erhalten; und
Halogenieren des reduzierten Zwischenprodukts, um ein halogeniertes Zwischenprodukt zu erhalten, worin die OH-Gruppe durch ein Halogen ersetzt ist; und
Umsetzen des halogenierten Zwischenprodukts mit Natriumsulfit in einem alkoholischen Lösungsmittel;

N. für eine Verbindung der allgemeinen Formel (I), worin $R^3$ $OS(O)_2R^{12}$ ist:

Umsetzen einer Verbindung der allgemeinen Formel (I), worin $R^3$ $C(O)OR^{12}$ ist, mit einem $C_{1-6}$-Alkanoyl- oder Benzoylchlorid oder mit einem $C_{1-6}$-Alkansäureanhydrid zum Schützen von OH-Gruppen; und
Umwandeln von $C(O)OR^{12}$ des geschützten Zwischenprodukts durch Reduktion mit einem Hydridreduktionsmittel in OH, um ein reduziertes Zwischenprodukt zu erhalten; und
Umsetzen des reduzierten Zwischenprodukts mit Chlorsulfonsäure in Gegenwart einer Base, um ein geschütztes Produkt zu erhalten; und
basische Hydrolyse des geschützten Produkts zum Entfernen der Schutzgruppen;

O. für eine Verbindung der allgemeinen Formel (I), worin $R^3$ $S(O)_2R^{12}$ ist:
Umsetzen des reduzierten Zwischenprodukts von (M) oder (N) vorstehend mit dem Lawesson-Reagenz, gefolgt von Oxidation des resultierenden Produkts.

## Revendications

1. Composé de formule générale (I) :

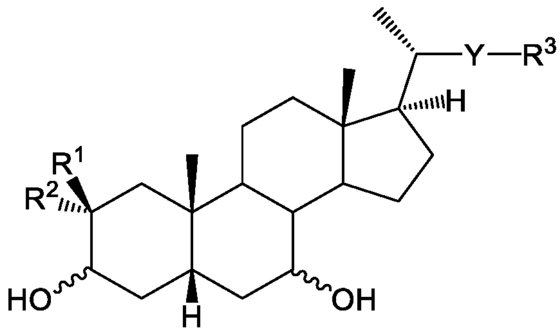

(I)

dans lequel
l'un de $R^1$ et $R^2$ est F et l'autre de $R^1$ et $R^2$ est H ou F ;
Y est une liaison ou un groupe de liaison alkylène en $C_{1-20}$, alcénylène en $C_{2-20}$ ou alcynylène en $C_{2-20}$ ;
$R^3$ est $C(O)OR^{12}$, $C(O)NR^{12}R^{13}$, $S(O)_2R^{12}$, $OS(O)_2R^{12}$, $S(O)_2OR^{12}$, $OS(O)_2OR^{12}$, $S(O)_2NR^{12}R^{13}$, $C(O)NR^{12}S(O)_2R^{13}$, $NHC(O)NR^{12}S(O)_2R^{13}$, $OP(O)(OR^{12})_2$, $C(O)NR^{12}[CH(R^{15})]_nR^{16}$ ou $C(O)NR^{12}C(O)CH_2NR^{12}[CH(R^{15})]_nR^{16}$ ;
chaque $R^{12}$ est indépendamment H ou alkyle en $C_{1-6}$ éventuellement substitué par un ou plusieurs substituants choisis parmi halogéno, $OR^{10}$, $NR^{10}R^{11}$, $R^{16}$ et aryle ;
chaque $R^{10}$ et $R^{11}$ est indépendamment H ou alkyle en $C_{1-6}$ ;
$R^{13}$ est H, alkyle en $C_{1-6}$ éventuellement substitué par un ou plusieurs substituants choisis parmi halogéno et aryle ; ou un cycle carbocyclique ou un cycle hétérocyclique de 3 à 8 chaînons, dans lequel ledit cycle carbocyclique ou hétérocyclique est éventuellement substitué par un ou plusieurs substituants choisis parmi =O et $R^{16}$ ; ou un cycle phényle ou hétéroaryle de 5 ou 6 chaînons, dans lequel ledit cycle phényle ou hétéroaryle est éventuellement substitué par un substituant $R^{16}$ ; ou
lorsque $R^3$ est $C(O)NR^{12}R^{13}$ ou $S(O)_2NR^{12}R^{13}$, $R^{12}$ et $R^{13}$ forment avec l'atome d'azote auquel ils sont attachés un cycle hétérocyclique de 3 à 8 chaînons qui contient éventuellement un ou plusieurs hétéroatomes supplémentaires choisis parmi N, O et S ; et est éventuellement substitué par un ou plusieurs substituants choisis parmi $CH_2C(O)OH$, $C(O)OH$, alkyle en $C_{1-6}$, C(O)O-alkyle en $C_{1-6}$, $S(O)_2OH$, =O et =N-OH ; et est éventuellement fusionné à un groupe phényle qui est non substitué ou substitué par un ou plusieurs substituants choisis parmi halogéno et nitro ;
n vaut 1, 2 ou 3 ;
chaque $R^{15}$ est indépendamment H ou alkyle en $C_{1-6}$ éventuellement substitué par un ou plusieurs substituants choisis parmi halogéno, phényle et hétéroaryle à 5 ou 6 chaînons ; un groupe cycloalkyle à 3 à 8 chaînons ; ou un groupe $R^{14}$, où $R^{14}$ est une chaîne latérale d'un acide aminé ; ou
lorsque n est 2 ou 3, deux groupes $R^{15}$ avec les atomes de carbone auxquels ils sont attachés, et éventuellement un atome de carbone intermédiaire lorsqu'il est présent, peuvent se combiner pour former $-(CH_2)_p-$ de telle sorte que le groupe $[CH(R^{15})]_n$ soit un cycle carbocyclique de 3 à 8 chaînons ;
p est 1, 2, 3, 4, 5 ou 6 ;
$R^{16}$ est choisi parmi $C(O)OH$, $S(O)_{2O}H$, $S(O)_2$(alkyle en $C_{1-6}$), $OS(O)_2OH$ et $P(O)(OH)_2$ ;
ou un sel ou un variant isotopique pharmaceutiquement acceptable de celui-ci.

2. Composé selon la revendication 1 qui est un composé de formule générale (IA), (IB), (IC) ou (ID) :

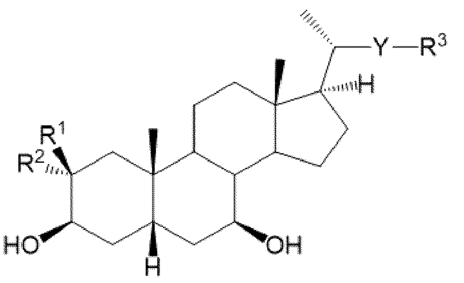

(IA)

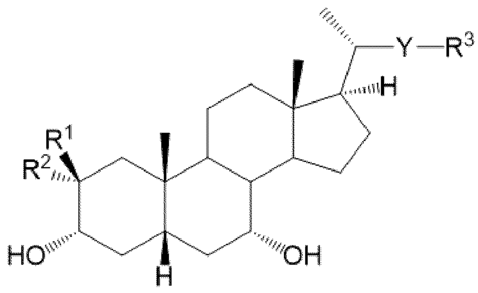

(IB)

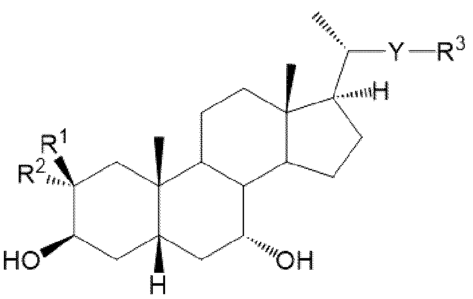

(IC)

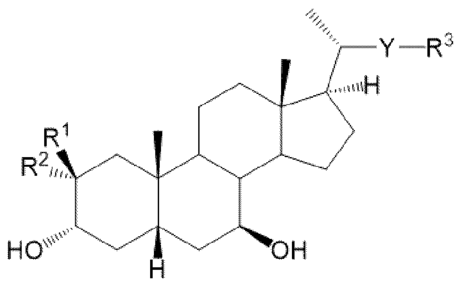

(ID)

dans lequel $R^1$, $R^2$, Y et $R^3$ sont tels que définis ci-dessus pour la formule générale (I).

**3.** Composé selon la revendication 2 qui est un composé de formule générale (IA) ou un composé de formule générale (IB).

**4.** Composé selon l'une quelconque des revendications 1 à 3, dans lequel $R^1$ et $R^2$ sont tous deux F.

**5.** Composé selon l'une quelconque des revendications 1 à 4, dans lequel, indépendamment ou en combinaison quelconque :

Y est une liaison ou un groupe de liaison alkylène en $C_{1-3}$ ou alcénylène en $C_{2-3}$ ;
$R^3$ est $C(O)OR^{12}$, C(O) $NR^{12}CH$ $(R^{14})C(O)$ OH ou $C(O)NR^{12}CH(R^{15})CH(R^{15})S(O)_2OH$, dans lequel $R^{12}$ et $R^{14}$ sont tels que définis dans la revendication 1 et $R^{15}$ est H ou alkyle en $C_{1-6}$ éventuellement substitué par un ou plusieurs groupes halogéno
ou aryle ; ou
$R^3$ est $C(O)OR^{12}$, $(C(O)N(R^{12})(R^{13})$ ou $C(O)NR^{12}[CH(R^{15})]_nR^{16}$ ;
dans lequel $R^{12}$, $R^{13}$, $R^{15}$, $R^{16}$ et n sont tels que définis dans la revendication 1.

**6.** Composé selon l'une quelconque des revendications 1 à 5, dans lequel

Y est $-CH_2CH_2-$ ; et/ou
$R^{12}$ est H ou méthyle ou éthyle éventuellement substitué par $R^{16}$ ou $N(R^{10})(R^{11})$ ; ou
$R^3$ est C (O) $NR^{12}S(O)_2R^{13}$, $NHC(O)NR^{12}S(O)_2R^{13}$, $C(O)NR^{12}[CH(R^{15})]_nR^{16}$ ou $C(O)NR^{12}C(O)CH_2NR^{12}[CH(R^{15})]_nR^{16}$ ; et $R^{12}$ est H ou méthyle ; et/ou $R^{13}$ est un cycle carbocyclique ou un cycle hétérocyclique à 5 ou 6 chaînons éventuellement substitué par $R^{16}$ ou =O ; ou phényle éventuellement substitué par $R^{16}$ ; ou
$R^3$ est $C(O)NR^{12}R^{13}$ ou $S(O)_2NR^{12}R^{13}$ et $R^{12}$ et $R^{13}$ forment avec l'atome d'azote auquel ils sont liés un cycle hétérocyclique à 5 ou 6 chaînons éventuellement substitué par un ou plusieurs substituants choisis parmi $R^{16}$ et =O et comprenant éventuellement un ou plusieurs hétéroatomes supplémentaires choisis parmi O, N et S ; et/ou
$R^{16}$ est C(O)OH, $S(O)_2OH$, $S(O)_2$(alkyle en $C_{1-6}$) ou $OS(O)_2OH$ ; ou $R^{16}$ est C(O)OH, $S(O)_2OH$, $OS(O)_2OH$ ou $P(O)(OH)_2$ et le composé est sous la forme d'un sel pharmaceutiquement acceptable.

**7.** Composé selon l'une quelconque des revendications 1 à 6, dans lequel

$R^3$ est $C(O)OR^{12}$, $C(O)NR^{12}[CH(R^{15})]_nR^{16}$ ou $C(O)NR^{12}R^{13}$ ;
ou un sel pharmaceutiquement acceptable de celui-ci.

**8.** Composé selon la revendication 7, dans lequel :

$R^3$ est $C(O)OR^{12}$ et $R^{12}$ est H, $CH_2R^{16}$ ou $-CH_2CH_2R^{16}$ où $R^{16}$ est tel que défini ci-dessus ; ou
$R^3$ est C (O) $NR^{12}[CH\ (R^{15})]_nR^{16}$ dans lequel $R^{12}$ est H, méthyle ou méthyle substitué par $R^{16}$ ; ou
$R^3$ est $C(O)NR^{12}R^{13}$, dans lequel $R^{12}$ est H ou alkyle en $C_{1-3}$ substitué par un seul substituant $R^{16}$, dans lequel $R^{16}$ est tel que défini dans la revendication 1 ; et/ou $R^{13}$ est phényle, ou un groupe cycloalkyle ou hétérocyclyle à 5 à 7 chaînons, dont l'un quelconque est éventuellement substitué par un seul substituant $R^{16}$ et où les groupes cycloalkyle et hétérocyclyle sont substitués par un ou plusieurs substituants =O.

**9.** Composé selon la revendication 7 ou la revendication 8, dans lequel :

$R^3$ est $C(O)NR^{12}CH(R^{14})C(O)OH$ ou $C(O)NR^{12}CH(R^{15})CH\ (R^{15})S(O)_2OH$, dans lequel $R^{14}$ est une chaîne latérale d'un acide aminé et $R^{15}$ est H ou alkyle en $C_{1-6}$ éventuellement substitué par un ou plusieurs groupes halogéno ou aryle ; ou
$R^3$ est C (O) $NR^{12}[CH\ (R^{15})]_nR^{16}$ et chaque $R^{15}$ est indépendamment H ou alkyle en $C_{1-4}$ éventuellement substitué comme indiqué dans la revendication 1 ; ou
n est 2 ou 3 et deux groupes $R^{15}$ se combinent avec les atomes de carbone auxquels ils sont attachés, et éventuellement avec un atome de carbone intermédiaire lorsqu'il est présent, pour former un cycle carbocyclique de 5 à 7 chaînons.

**10.** Composé selon la revendication 9, dans lequel :

$R^3$ est $C(O)NR^{12}CH(R^{14})C(O)OH$, dans lequel $R^{12}$ est H ou méthyle et $R^{14}$ est H ; ou
$R^3$ est $C(O)NR^{12}CH(R^{15})CH(R^{15})S(O)_2OH$, dans lequel $R^{12}$ est H ou méthyle et les deux fractions $R^{15}$ sont H.

**11.** Composé selon la revendication 1, choisi parmi :

l'acide 2β-fluorochénodésoxycholique (composé 1) ;
l'acide 2β-fluoro-3β,7α-dihydroxy-5β-cholanique (composé 2) ;
l'acide 2α-fluoro-3β,7α-dihydroxy-5β-cholanique (composé 3) ;
l'acide 2α-fluoro-3β,7β-dihydroxy-5β-cholanique (composé 4) ;
l'acide 2α-fluoro-3α,7α-dihydroxy-5β-cholanique (composé 5) ;
l'acide 2α-fluoro-3α,7β-dihydroxy-5β-cholanique (composé 6) ;
l'acide 2,2-difluoro-3β,7β-dihydroxy-5β-cholanique (composé 7) ;
l'acide 2,2-difluoro-3α,7α-dihydroxy-5β-cholanique (composé 8) ;
l'acide 2,2-difluoro-3α,7β-dihydroxy-5β-cholanique (composé 9) ;
l'acide N-(2,2-difluoro-3β,7β-dihydroxy-5β-cholan-24-amide)-éthylsulfonique (composé 10) ;
l'acide N-(2,2-difluoro-3β,7β-dihydroxy-5β-cholan-24-amide)-propanoïque (composé 11) ;
*l'acide* N-(méthyl),N-(2,2-difluoro-3β,7β-dihydroxy-5β-cholan-24-amide)-acétique (composé 12) ;
l'acide N-(2,2-difluoro-3β,7β-dihydroxy-5β-cholan-24-amide)-trans-2-cyclohexane carboxylique (composé 13) ;
l'acide 1-(2,2-difluoro-3β,7β-dihydroxy-5β-cholan-24-oyl)-pipéridine-3-carboxylique (composé 14) ;
l'acide 3-(2,2-difluoro-3β,7β-dihydroxy-5β-cholan-24-amide)-4-thiazolidine-carboxylique (composé 15) ;
*la* N-(2,2-difluoro-3β,7β-dihydroxy-5β-cholan-24-oyl)-morpholine (Composé 16) ;
l'acide N-(2,2-difluoro-3β,7β-dihydroxy-5β-cholan-24-amide)-méthylcarboxylique (Composé 17) ;
l'acide N-(carboxyméthyl)-N-(2,2-difluoro-3β,7β-dihydroxy-5β-cholan-24-oyl)-2-amino acétique (composé 18) ;
l'acide N-(méthyl)-N-(2,2-difluoro-3β,7β-dihydroxy-5β-cholan-24-amide)-éthylsulfonique (composé 19) ;
l'acide 3-(2,2-difluoro-3β,7β-dihydroxy-5β-cholan-24-oyl) amino-propanesulfonique (composé 20) ;
l'acide *N*-(2,2-difluoro-3β,7β-dihydroxy-5β-cholan-24-amide)-méthanesulfonique (composé 21) ;
*l'acide N*-(2,2-difluoro-3β,7β-dihydroxy-5β-cholan-24-oyl)-2-aminoéthyl sulfurique (composé 22) ;
l'acide O-(2,2-difluoro-3β,7β-dihydroxy-5β-cholan-24-oyl)-2-hydroxy éthyl sulfonique (composé 23) ;
l'acide N-(2,2-difluoro-3β,7β-dihydroxy-5β-cholan-24-oyl)aniline-2-sulfonique (composé 24) ;
l'acide *N*-(cyclohexyl)-N-(2,2-difluoro-3β,7β-dihydroxy-5β-cholan-24-oyl)-3-amino-propanesulfonique (composé 25) ;
l'acide *N*-(cyclohexyl)-N-(2,2-difluoro-3β,7β-dihydroxy-5β-cholan-24-oyl)-2-amino-éthanesulfonique (composé 26) ;

la *N*-(2,2-difluoro-3β,7β-dihydroxy-5β-cholan-24-oyl) 2-aminoéthyl méthyl sulfone (Composé 27) ;
le *dioxyde* de *N*-(éthyl)-N-(2,2-difluoro-3β,7β-dihydroxy-5β-cholan-24-oyl)-3-amino-tétrahydrothiophène (composé 28) ;
*le dioxyde* de N-(2-(diisopropylamino)éthyl)-N-(2,2-difluoro-3β,7β-dihydroxy-5β-cholan-24-oyl)-3-amino-tétrahydrothiophène (composé 29) ;
le *dioxyde* de *N*-(2,2-difluoro-3β,7β-dihydroxy-5β-cholan-24-oyl)-thiomorpholine (Composé 30) ;
la *N*-(2,2-difluoro-3β,7β-dihydroxy-5β-cholan-24-oyl) 1,1-dioxidotétrahydro-2H-thiopyran-3-ylamine (Composé 31) ; et
des sels pharmaceutiquement acceptables de ceux-ci, le cas échéant, notamment les sels métalliques, tels que les sels de sodium ou de potassium, en particulier les sels de sodium ou, pour le composé 18, le sel disodique.

**12.** Composé selon la revendication 1 qui est choisi parmi :

l'acide 2,2-difluoro-3β,7β-dihydroxy-5β-cholanique (composé 7) ;
l'acide 2,2-difluoro-3α,7α-dihydroxy-5β-cholanique (composé 8) ;
l'acide 2,2-difluoro-3α,7β-dihydroxy-5β-cholanique (composé 9) ; et
ou des sels pharmaceutiquement acceptables de l'un quelconque des ceux-ci.

**13.** Composé selon l'une quelconque des revendications 1 à 12 pour une utilisation en médecine, par exemple pour une utilisation dans le traitement ou la prévention d'un trouble neurodégénératif tel que la maladie de Parkinson, un trouble cognitif léger, la démence ; y compris la maladie d'Alzheimer, la démence vasculaire et la démence à corps de Lewy ; la maladie de Huntington, la sclérose latérale amyotrophique (maladie du motoneurone), la paralysie supranucléaire progressive ou la maladie de Wilson.

**14.** Composé pour une utilisation selon la revendication 13, dans lequel le trouble neurodégénératif est la maladie de Parkinson et le composé est choisi parmi l'acide 2,2-difluoro-3β,7β-dihydroxy-5β-cholanique (composé 7) et l'acide 2,2-difluoro-3α,7β-dihydroxy-5β-cholanique (composé 9) ; ou
dans lequel le trouble neurodégénératif est la maladie d'Alzheimer et le composé est l'acide 2,2-difluoro-3α,7α-dihydroxy-5β-cholanique (composé 8).

**15.** Composition pharmaceutique comprenant un composé selon l'une quelconque des revendications 1 à 12 et un excipient ou un support pharmaceutiquement acceptable.

**16.** Procédé de préparation d'un composé selon l'une quelconque des revendications 1 à 12 comprenant :

A. pour un composé de formule générale (IB) ou (IC) tel que défini dans la revendication 2 dans lequel $R^1$ est F et $R^3$ est $C(O)OR^{12a}$ : dans lequel $R^{12a}$ est alkyle en $C_{1-6}$ éventuellement substitué par un ou plusieurs groupes halogéno ou aryle :

le traitement avec un acide d'un composé de formule générale (II) :

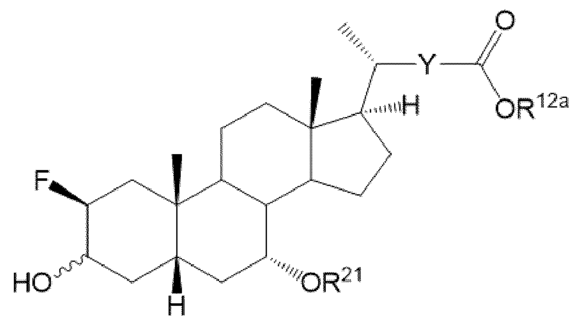

(II)

dans lequel Y et $R^3$ sont tels que définis dans la revendication 1 ; $R^{12a}$ est alkyle en $C_{1-6}$ éventuellement substitué par un ou plusieurs groupes halogéno ou aryle ; et $R^{21}$ est un groupe protecteur d'OH qui est labile par un acide ;

B. pour un composé de formule générale (IA) ou (IC) tel que défini dans la revendication 2 dans lequel $R^2$ est F et $R^3$ est $C(O)OR^{12a}$, dans lequel $R^{12a}$ est tel que défini pour la formule générale (II) :

la réduction d'un composé de formule générale (XIIa) :

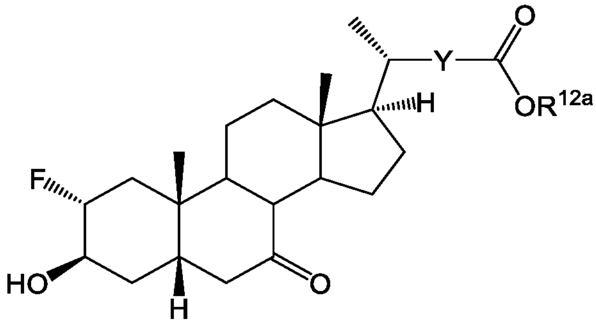

(XIIa)

dans lequel Y est tel que défini dans la revendication 1 et $R^{12a}$ est tel que défini pour la formule générale (II) ;

C. pour un composé de formule générale (IB) ou (ID) tel que défini dans la revendication 2 dans lequel $R^2$ est F et $R^3$ est $C(O)OR^{12a}$, dans lequel $R^{12a}$ est tel que défini pour la formule générale (II) :

la réduction d'un composé de formule générale (XIIb) :

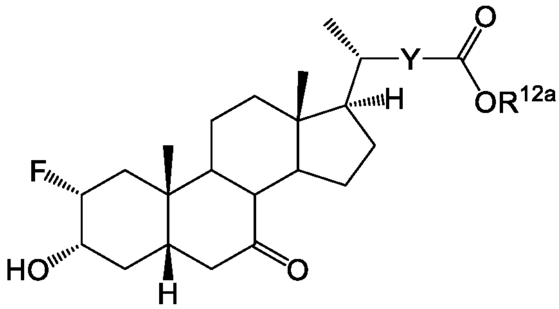

(XIIb)

dans lequel Y est tel que défini dans la revendication 1 et $R^{12a}$ est tel que défini pour la formule générale (II) ;

D. pour un composé de formule générale (IA) tel que défini dans la revendication 2 dans lequel $R^1$ et $R^2$ sont tous deux F et $R^3$ est $C(O)OR^{12a}$, dans lequel $R^{12a}$ est tel que défini pour la formule générale (II) :

la réaction avec un acide d'un composé de formule générale (XXI) :

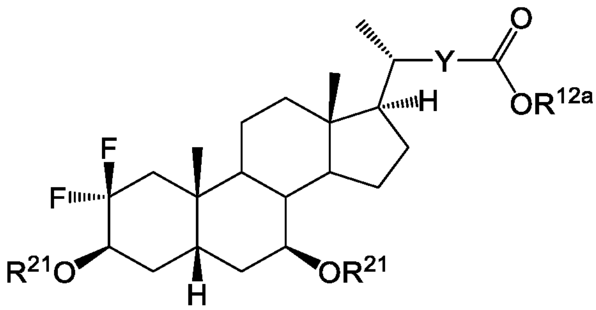

(XXI)

dans lequel Y est tel que défini dans la revendication 1 et $R^{12a}$ et $R^{21}$sont tels que définis pour la formule

générale (II) ;

E. pour un composé de formule générale (IB) ou (ID) tel que défini dans la revendication 2 dans lequel $R^1$ et $R^2$ sont tous deux F et $R^3$ est $C(O)OR^{12a}$, dans lequel $R^{12a}$ est tel que défini pour la formule générale (II) :

la réduction d'un composé de formule générale (XXII) :

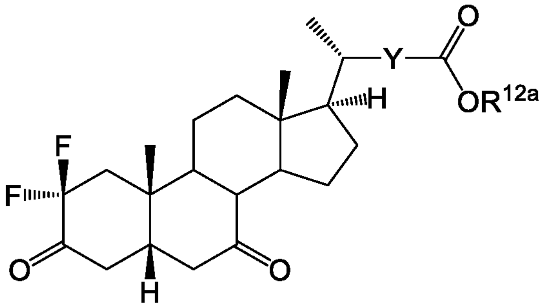

(XXII)

dans lequel Y est tel que dans la revendication 1 et $R^{12a}$ est tel que défini pour la formule générale (II) ;

F. pour un composé de formule générale (I) dans lequel $R^3$ est C(O)OH :
l'hydrolyse d'un composé de formule générale (I) dans lequel $R^3$ est $C(O)R^{12a}$, dans lequel $R^{12a}$ est tel que défini ci-dessus pour la formule générale (II) ;
G. pour un composé de formule générale (I) dans lequel $R^3$ est $C(O)NR^{12}R^{13}$ :

la réaction d'un composé de formule générale (I) dans lequel $R^3$ est C(O)OH avec une amine de formule générale :

$$H\text{-}NR^{12}R^{13}$$

dans lequel $R^{12}$ et $R^{13}$ sont tels que définis dans la revendication 1 ;
en présence d'un réactif de couplage et d'une amine ;

H. pour un composé de formule générale (I) dans lequel $R^3$ est $C(O)NR^{12}[CH\ (R^{15})]_nR^{16}$ :

la réaction d'un composé de formule générale (I) dans lequel $R^3$ est C(O)OH avec un composé de formule générale (XL) :

$$HNR^{12}[CH\ (R^{15})]_nR^{16} \qquad (XL)$$

dans lequel $R^{12}$, $R^{15}$, n et $R^{16}$ sont tels que définis dans la revendication 1 ;
en présence d'un agent de couplage et d'une amine ;

I. pour un composé de formule générale (I) dans lequel $R^3$ est $C(O)NR^{12}CH(R^{14})C(O)OH$ :

la réaction d'un composé de formule générale (I) dans lequel $R^3$ est C(O)OH par réaction avec un acide aminé de formule générale (XLI) :

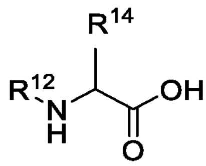

(XLI)

dans lequel $R^{12}$ et $R^{14}$ sont tels que définis dans la revendication 1 ;
en présence d'un agent de couplage et d'une amine ;

J. pour un composé de formule générale (I) dans lequel $R^3$ est $C(O)NR^{12}CH(R^{15})CH\ (R^{15})S(O)_2OH$ :

la réaction d'un composé de formule générale (I) dans lequel $R^3$ est C(O)OH par réaction avec un composé de formule générale (XLII) :

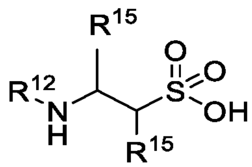

(XLII)

dans lequel $R^{12}$ et $R^{15}$ sont tels que définis dans la revendication 1 ;
en présence d'un agent de couplage et d'une amine ;

K. pour un composé de formule générale (I) dans lequel $R^3$ est $C(O)NR^{12})S(O)_2R^{13}$ :

la réaction d'un composé de formule générale (I) dans lequel $R^3$ est C(O)OH avec un composé de formule :

$NHR^{12}S(O)_2R^{13}$

dans lequel $R^{12}$ et $R^{13}$ sont tels que définis dans la revendication 1, en présence d'un réactif de couplage et d'une amine ;

L. pour un composé de formule générale (I) dans lequel $R^3$ est $NHC(O)NR^{12})S(O)_2R^{13}$ :

la réaction d'un composé de formule générale (I) dans lequel $R^3$ est C(O)OH de la manière suivante :

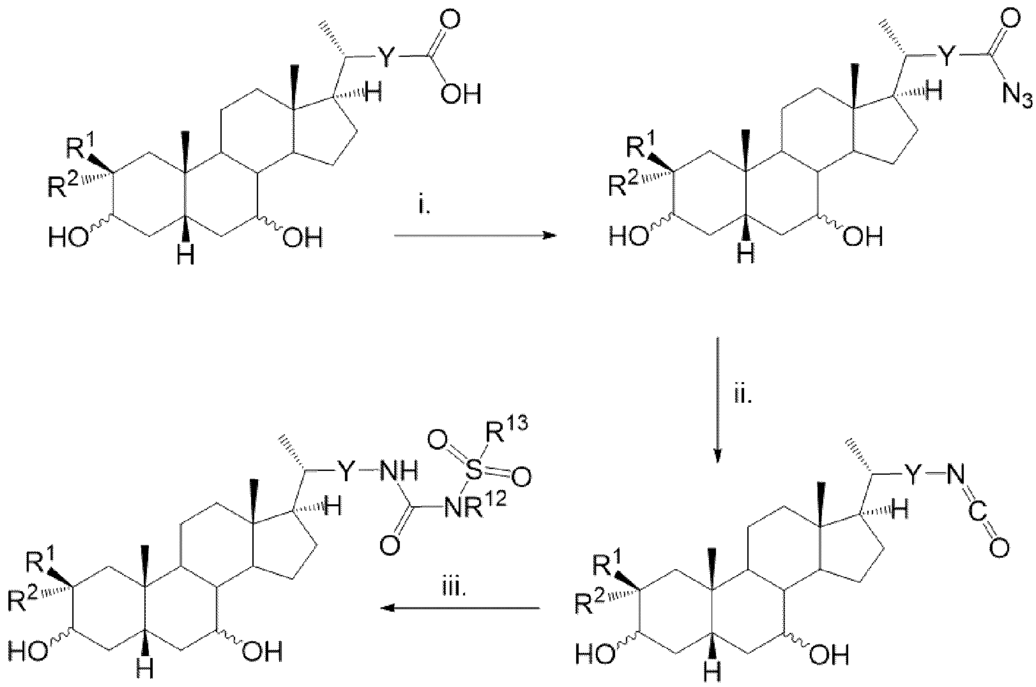


i azoture de diphénylphosphoryle/triéthylamine
ii chaleur

iii $R^{13}SO_2NHR^{12}$

dans lequel $R^1$, $R^2$, $R^{12}$ et $R^{13}$ sont tels que définis dans la revendication 1 ;

M. pour un composé de formule générale (I) dans lequel $R^3$ est $S(O)_2OR^{12}$ :

la réaction d'un composé de formule générale (I) dans lequel $R^3$ est C(O)OH avec un chlorure d'alcanoyle en $C_{1-6}$ ou de benzoyle ou avec un anhydride alcanoïque en $C_{1-6}$ pour donner un intermédiaire dans lequel les groupes OH sont protégés ; et
la conversion du groupe acide carboxylique, $R^3$, de l'intermédiaire protégé en OH par réduction avec un agent réducteur hydrure pour donner un intermédiaire réduit ; et
l'halogénation de l'intermédiaire réduit pour donner un intermédiaire halogéné dans lequel le groupe OH est remplacé par un halogène ; et
la réaction de l'intermédiaire halogéné avec du sulfite de sodium dans un solvant alcoolique ;

N. pour un composé de formule générale (I) dans lequel $R^3$ est $OS(O)_2R^{12}$ :

la réaction d'un composé de formule générale (I) dans lequel $R^3$ est $C(O)OR^{12}$ avec un chlorure d'alcanoyle en $C_{1-6}$ ou de benzoyle ou avec un anhydride alcanoïque en $C_{1-6}$ pour protéger tous groupes OH ; et
la conversion du $C(O)OR^{12}$ de l'intermédiaire protégé en OH par réduction avec un agent réducteur hydrure pour donner un intermédiaire réduit ; et
la réaction de l'intermédiaire réduit avec de l'acide chlorosulfonique en présence d'une base pour donner un produit protégé ; et
l'hydrolyse basique du produit protégé pour éliminer les groupes protecteurs ;

O. pour un composé de formule générale (I) dans lequel $R^3$ est $S(O)_2R^{12}$ :
la réaction de l'intermédiaire réduit de (M) ou (N) ci-dessus avec le réactif de Lawesson suivie de l'oxydation du produit résultant.

Figure 1A

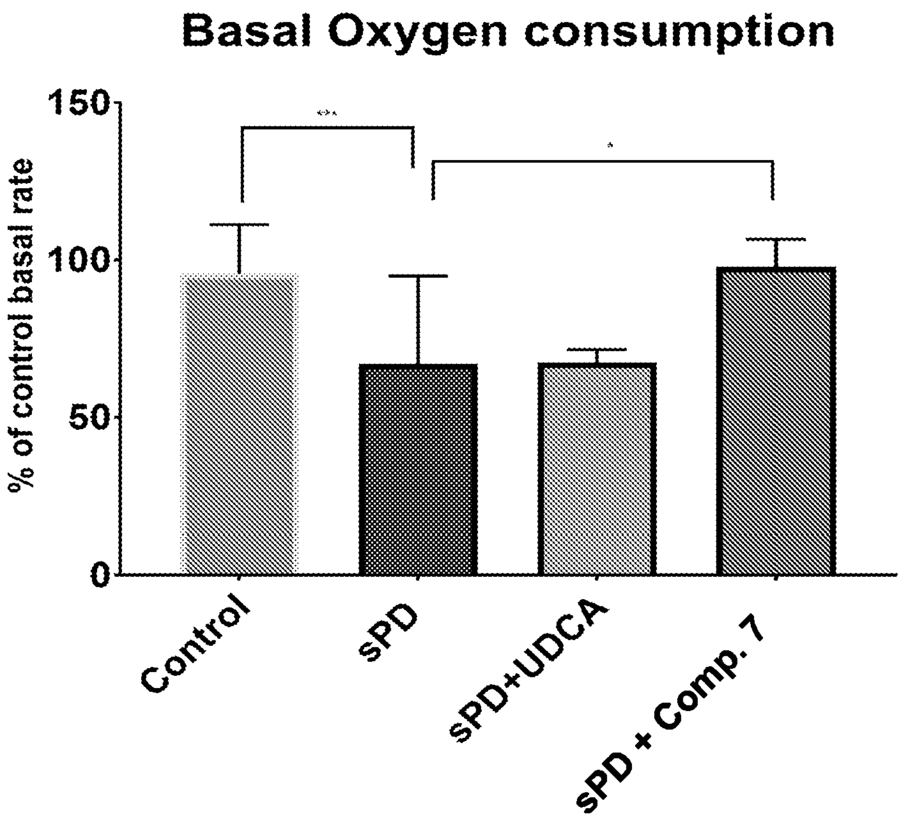
Basal Oxygen consumption
% of control basal rate
150
100
50
0
Control
sPD
sPD+UDCA
sPD + Comp. 7

Figure 1B

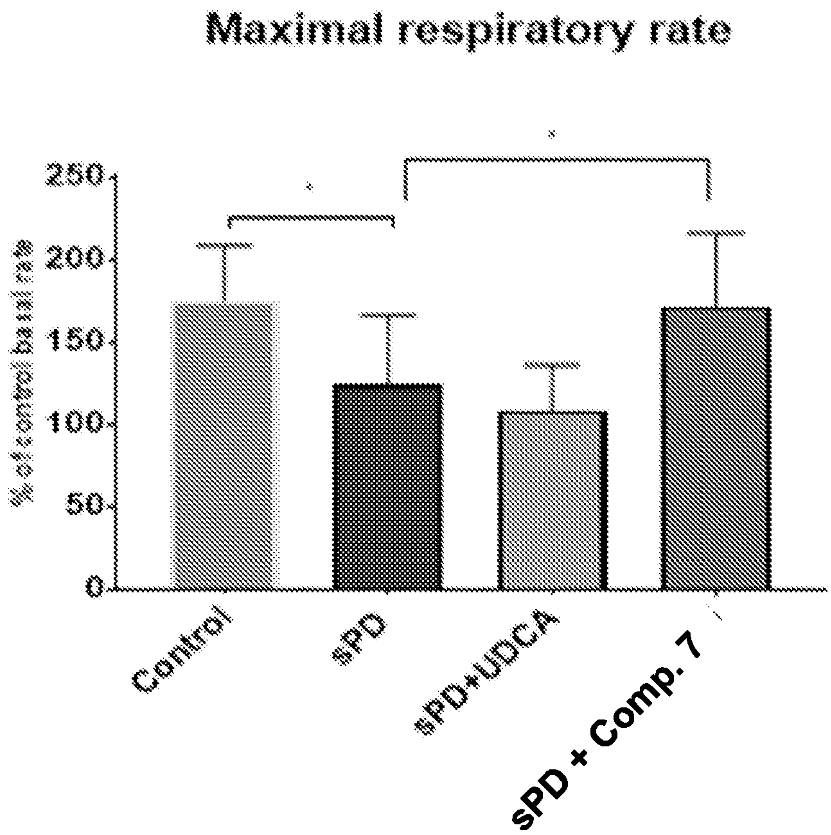
Maximal respiratory rate
% of control basal rate
250
200
150
100
50
0
Control
sPD
sPD+UDCA
sPD + Comp. 7

Figure 1C

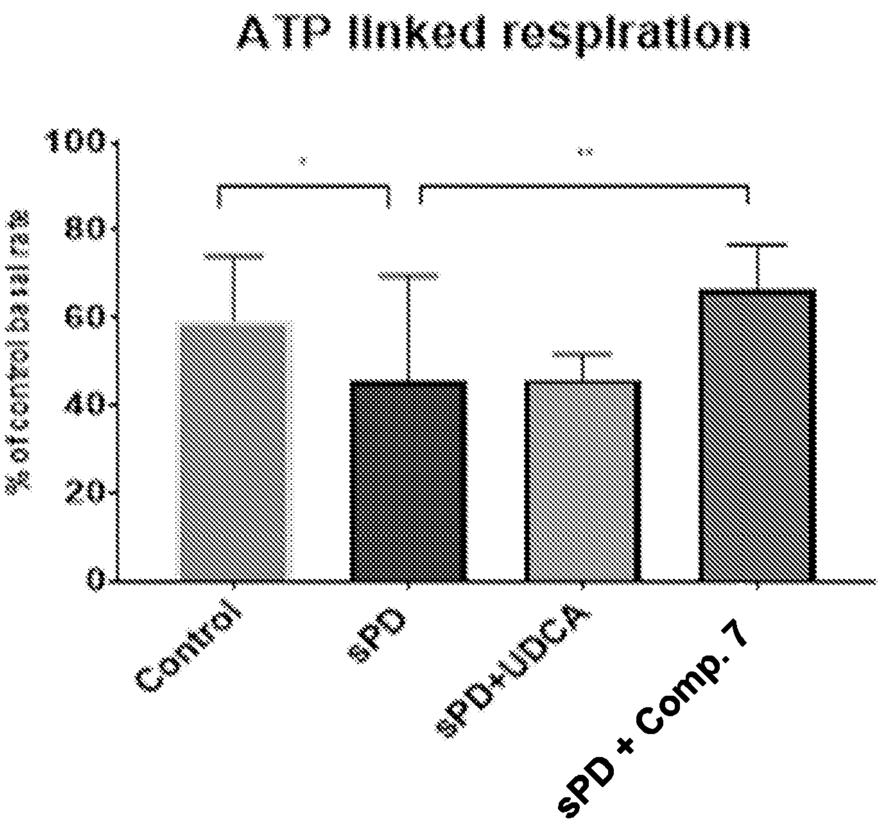
ATP linked respiration
100
80
60
40
20
0
% of control basal rate
*
**
Control
sPD
sPD+UDCA
sPD + Comp. 7

Figure 2

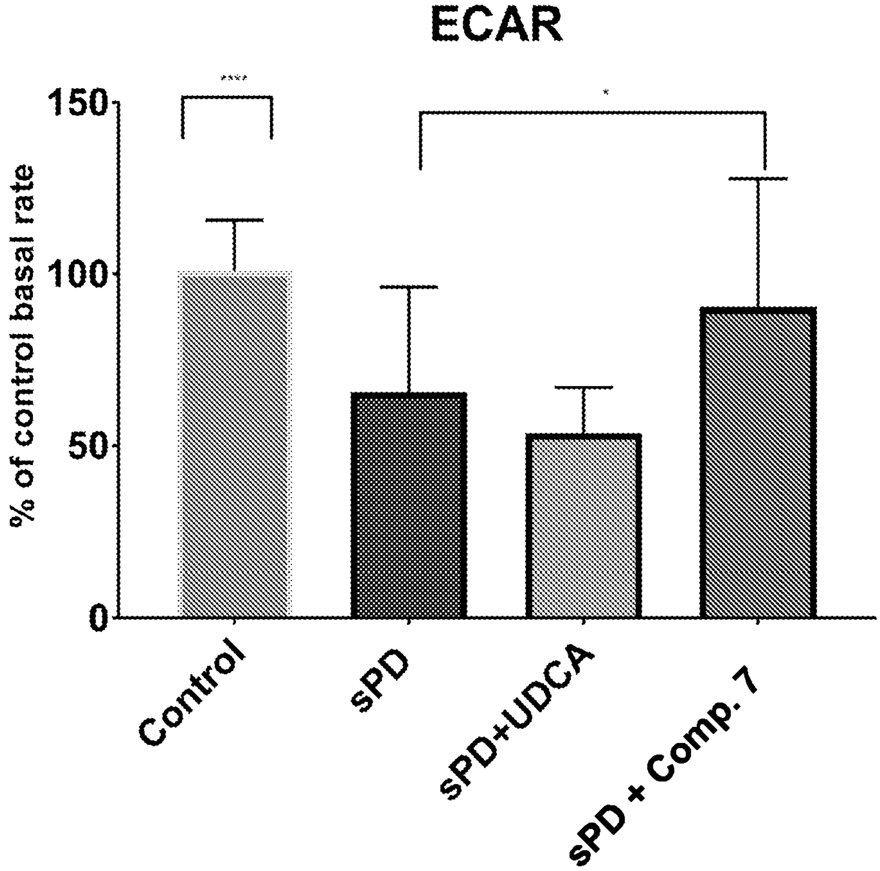
ECAR
****
*
150
100
50
0
% of control basal rate
Control
sPD
sPD+UDCA
sPD + Comp. 7

Figure 3

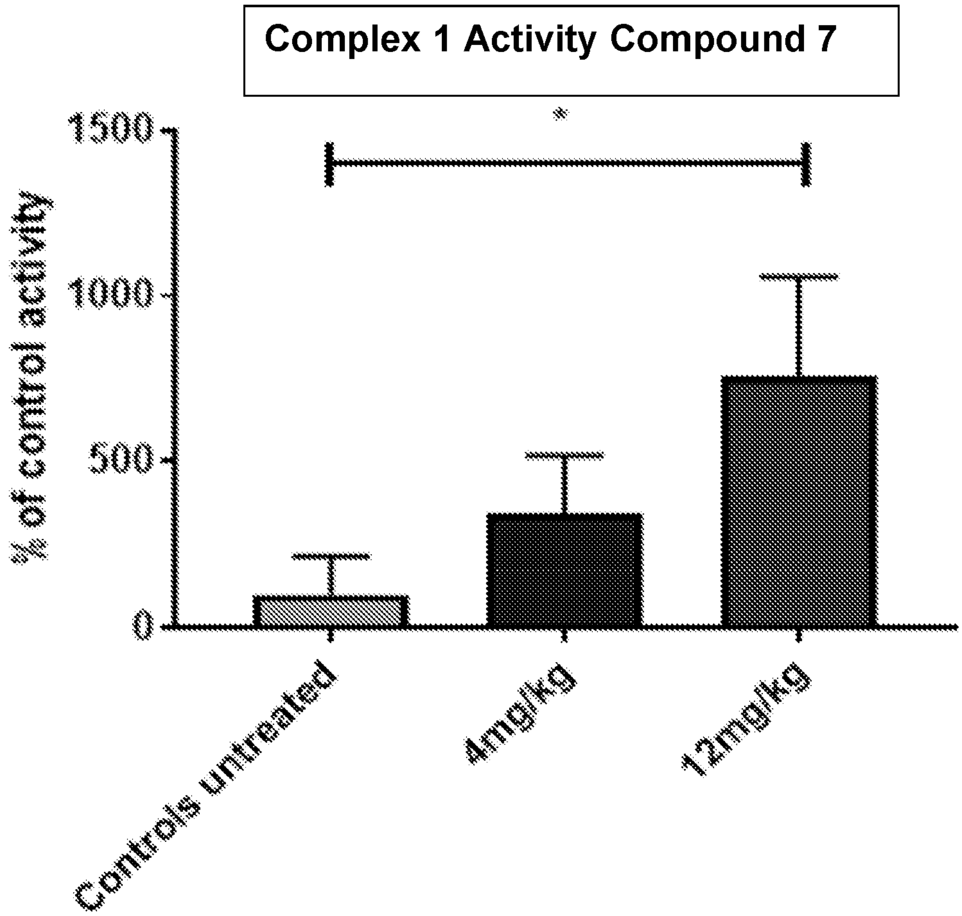


| | |
|---|---|
| Unpaired t test between controls and 12mg/kg dose | |
| P value | 0.0239 |
| P value summary | * |

Figure 4

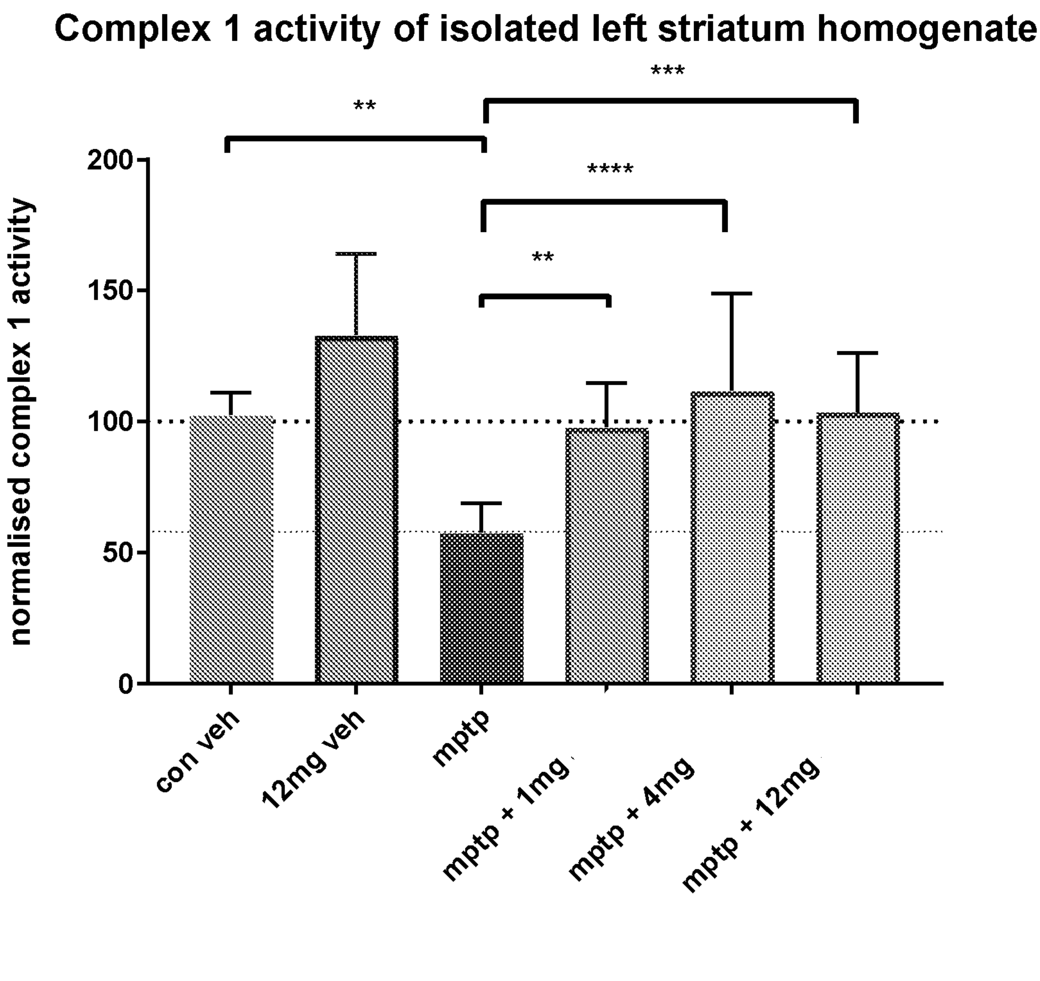
Complex 1 activity of isolated left striatum homogenate
***
**
****
**
200
150
100
50
0
normalised complex 1 activity
con veh
12mg veh
mptp
mptp + 1mg
mptp + 4mg
mptp + 12mg

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014036379 A
- WO 2015061421 A
- WO 2016145216 A


### Non-patent literature cited in the description


- **MORTIBOYS et al.** Ursocholanic acid rescues mitochondrial function in common forms of familial Parkinson's disease. *Brain*, 2013, vol. 136 (10), 3038-3050
- **MORTIBOYS et al.** *Neurology*, 2015, vol. 85, 846-852
- **BELL et al.** Ursodeoxycholic Acid Improves Mitochondrial Function and Redistributes Drp1 in Fibroblasts from Patients with either Sporadic or Familial Alzheimer's Disease. *Journal of Molecular Biology*, 2018, vol. 18, 30987-2
- **PAULEKUHN et al.** *J. Med. Chem.*, 2007, vol. 50, 6665-6672
- **FESTA et al.** *J. Med. Chem.*, 2014, vol. 57, 8477-8495
- **CLASSON et al.** *J. Org. Chem.*, 1988, vol. 53, 6126-6130
- *ACS Med. Chem. Lett.*, 2012, vol. 3, 273-277
- *Steroids*, 2012, vol. 77, 1233-1241
- **BARLOW et al.** *Eur. J. Med. Chem.*, 2011, vol. 46, 1545-1554
- **FUJIMOTO et al.** *Bioorg. Med. Chem. Lett.*, 2011, vol. 21, 6409-6413
- **ZHAO et al.** *Eur. J. Org. Chem.*, 2005, vol. 2005, 4414-4427
- **MEYER et al.** Direct conversion of patient fibroblasts demonstrates non-cell autonomous toxicity of astrocytes to motor neurons in familial and sporadic ALS. *Proc Natl Acad Sci USA*, 2014
- **MORTIBOYS et al.** Mitochondrial function and morphology are impaired in parkin-mutant fibroblasts. *Ann Neurol.*, November 2008, vol. 64 (5), 555-65